# EUROPEAN PATENT SPECIFICATION

(11) **EP 0 189 427 B1**
(45) Date of publication and mention of the grant of the patent: **08.02.1989**
(21) Application number: 85902404.4
(22) Date of filing: 08.05.1985
(51) Int. Cl.: C08B 37/10, C07H 13/04, C07C 101/28, C07D 213/79, C07D 213/55, C08B 37/16, A61K 31/725

(54) **ORALLY ACTIVE HEPARIN MULTIPLETS**
ORAL AKTIVER POLYELEKTROLYTISCHER KOMPLEX AUF DER BASIS VON HEPARIN
MULTIPLETS D'HEPARINE ORALEMENT ACTIFS

(30) Priority: 21.05.1984 US 612593
(43) Date of publication of application: 06.08.1986
(73) Proprietor: BODOR, Nicholas S., Gainsville, FL 32608 (US)
(72) Inventor: BODOR, Nicholas S., Gainsville, FL 32608 (US)
(74) Representative: Pendlebury, Anthony
(86) International application number: US8500846
(87) International publication number: WO8505362

(56) References cited:
- AT-A- 326 127
- GB-A- 923 052
- GB-A- 2 005 663
- US-A- 3 557 130
- Chemical Abstracts, volume 98, 7 March 1983, page 341 abstract number 76066u Bodor Nicholas: "Improved delivery through biological membranes"
- Die Makromoleculare Chemie, volume 183, nr. 4, April 1982, (Heidelberg, DE) pages 849-861 Robert B. Cundall: "Polyelectrolyte Complexes", 3a. The interaction between Heparin and Protamine"

## Description

### Field of the Invention

The present invention provides novel orally active ionic multiplets of polyanionic heparinic acid with selected polycationic materials. The multiplets are stable lipoidal "ion-pairs" or salts which can be absorbed through the gastrointestinal wall and which slowly release heparinic acid to achieve long-lasting anticoagulant activity.

### Background of the Invention

Since the initial reports about heparin [Howell et al, Am. J. Physiol. 47, 328 (1918)] and bishydroxy- coumarin or dicumarol [Link, Harvey Lect., 39,162-216 (1944)], anticoagulants have become the object of extensive biological investigation. Heparin is still considered by many as the drug of choice, despite the fact that it is not well absorbed orally and must be administered by a parenteral route. Toxic side effects are uncommon, but parenteral administration, whether as intermittent injections or continuous infusion, precludes its long term use. Therapy employing the clinically used oral anticoagulants, on the other hand, is difficult to control between desired limits because of the considerable variability in their rates of metabolism under differing conditions. In addition to undesirable delay in onset of activity after administration, drug interaction problems and side effects make the oral anticoagulants which are now available poor substitutes for heparin itself.

The possibility of administering heparin by routes other than injection while obtaining results comparable to those obtained by injection has aroused the interest of many investigators. See, for example, Windsor et al, Nature, London, 190, 263 (1961); Teow Yan Koh, United States Patents Nos. 3,482,014, 3,510,561 and 3,548,052; and Teow Yan Koh et al, United States Patents Nos. 3,506,642 and 3,577,534. Notably, orally active heparin salts and complexes have been described in the patent literature, e.g. amines, amides (the aforementioned Teow Yan Koh patents) and acid salts such as Na, K, Li etc. (the aforementioned Teow Yan Koh et al patents). Attempts have also been made to enhance oral absorption using sulfoxides and sulfones.

Nevertheless, serious need exists in this art for improved oral delivery of heparin.

### Summary and Objects of the Invention

Accordingly, a major object of the present invention is to provide improved delivery of heparin by the oral route. Another object of this invention is provide a form of heparin which will be readily absorbable from the intestine. Another object of this invention is to provide an orally active form of heparin which wil be highly stable and which will have a long duration of anticoagulant activity as compared to heparin sodium. These and other objects are achieved by the use of novel orally active ionic multiplets of polyanionic heparinic acid with selected polycationic materials. These multiplets are stable lipoidal "ion-pairs" or salts which can be absorbed through the gastrointestinal wall and which slowly release heparinic acid to achieve long-lasting anticoagulant activity. These novel ionic multiplets or salts result from combination of multivalent cationic units with the multivalent anionic heparin entity.

### Brief Description of the Drawings

FIGURE 1 is a graph plotting the amount of time required for clotting, in minutes, against the time elapsed since oral administration to rats, in hours, for Compounds 4 [heparin inositol hexa-(1-methyl-3-pyridiniumcarboxylate) multiplet], Compound 5 [heparin N,N-dimethyl-N-dodecyl-N-(I3-hydroxy)ethylammonium multiplet] and heparin sodium;
FIGURE 2 is a graph plotting the amount of time required for clotting, in hours, against the time elapsed since jejunal administration to rats, in hours, for Compound 4 [heparin inositol hexa-(1-methyl-3-pyridiniumcarboxylate) multiplet] and heparin sodium; and
FIGURE 3 is a graph plotting the amount of time required for clotting, in minutes, against the time elapsed since jejunal administration to rabbits, in hours, for Compound 4 [heparin inositol hexa-(1-methyl-3-pyridiniumcarboxylate) multiplet] and heparin sodium.

### Detailed Description of the Invention

"Heparin", or heparinic acid, as used herein refers to a mucopolysaccharide present in mammalian tissue which has strong anticoagulant activity. The polysaccharide is a dextrorotatory, highly sulfated, negatively charged, strongly acidic polymer mixture of disaccharides or the corresponding tetrasaccharides. The precise chemical formula, structure and molecular weight are not yet fully elucidated and appear to vary with biological source. Goodman and Gilman's The Pharmacological Basis of Therapeutics, sixth edition, Macmillian Publishing Co., Inc., New York, New York, Chapter 58, pp. 1348-1351, indicates that heparin has an average molecular weight of 15,000 daltons and that commercial heparin is composed of polymers of two repeating disaccharide units, namely, a D-glucosamine-L-iduronic acid unit and a D-glucosamine-D-glucuronic acid unit. According to Goodman and Gilman, most samples of heparin sodium contain from 8 to 15 repeats of each unit, although they may not be in equal proportions. A structure for heparin sodium as depicted by Goodman and Gilman is shown below. See also Cutting's Handbook of Pharmacology, seventh edition, ed. T. Z. Csaky, M.D. and Byron A. barnes, Ph.D., Appleton-Century-Crofts, Norwalk, Connecticut, Chapter 27, pp. 318-319; The Merck Index, tenth edition, Merck & Co., Inc., Rahway, New Jersey, 1983, pages 672-673; and The Condensed Chemical Dictionary, eighth edition, revised by Gessner G. Hawley, Van Nostrand Reinhold Company, New York, page 436.

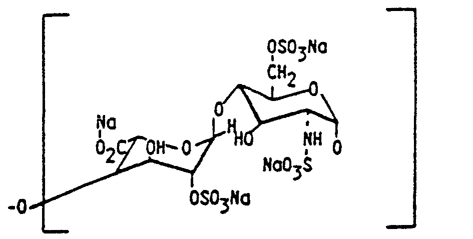

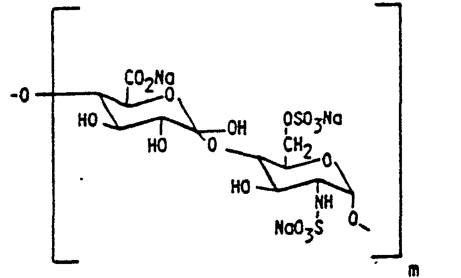

### Heparln Sodium Unit

The chain lengths and potency of heparin samples vary widely. Consequently heparin is prescribed on a unit basis. The U.S.P. unit of heparin is the amount required to prevent 1.0 ml of citrated sheep plasma from clotting for one hour after addition of 0.2 ml of a 1:100 calcium chloride solution. Heparin Sodium, U.S.P., must contain at least 120 U.S.P. units/mg.

The heparin multiolets or complexes of the present invention can be represented bv the structural formula 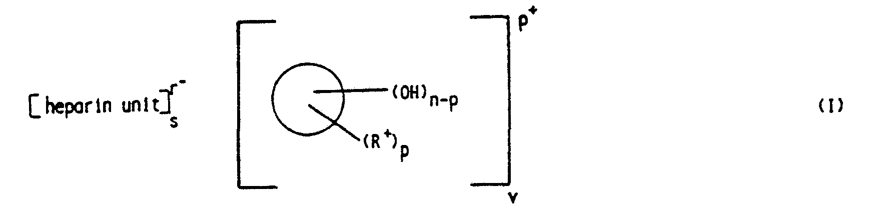 wherein ○ is the skeleton of a polyol, said skeleton being the portion of said polyol which would remain after removal of all hydroxy substituents therefrom; n is a number from 3 to 24 which represents the total number of hydroxy groups in said polyol; p is a number ≥ 3 and ≤ n; r is the available valence of the heparin unit and is ≥ 3 and ≤ 7; s is the number which when multiplied by r is equal to pv; v is the number which when multiplied by p is equal to rs; R⁺ is 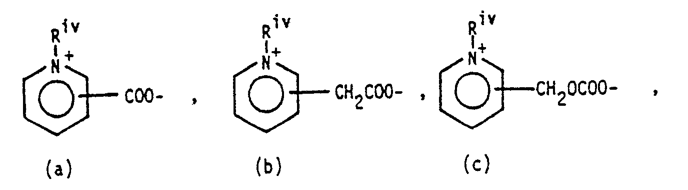 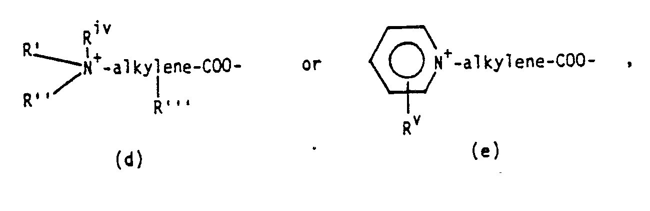 wherein the -COO-, -CH₂COO-, -CH₂0COO- and R" ring substituents can each be in the 2-, 3- or 4- position of the pyridinium ring; R^{iv} is C₁―C₃ alkyl; R' and R", which can be the same or different are each C₁-C₇ alkyl, or R' and R" are combined with the adjacent nitrogen atom such that 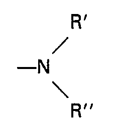 represents the resdidue of a saturated monocyclic secondary amine; R'" is 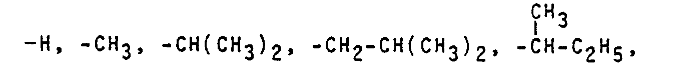 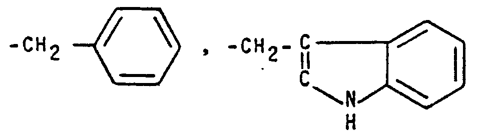 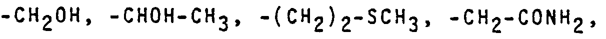 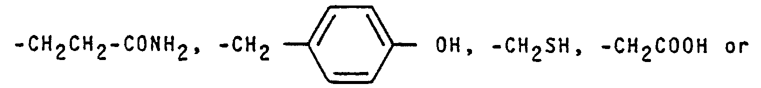 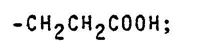 the alkylene groups can be straight or branched and contain 1 to 3 carbon atoms; and R^{v} is H, -CONH₂ or ―COO(C₁―C₇ alkyl).

The expression "[heparin unit]" as used herein is intended to indicate the basic tetrasaccharide unit which makes up heparin sodium itself except that from 3 to 7 of the sodium ions are removed, leaving a polyanion having an available or effective valence of from 3 to 7 which can then be "ion-paired" to the desired polycations to form the multiplets of formula (1). While a typical heparin sodium tetrasaccharide unit would contain one each of the D-glucosamine-L-iduronic acid disaccharide unit and the D-glucosamine-D-glucuronic acid disacchariae unit, the exact identity of the tetrasaccharide unit will vary with source as already explained hereinabove, as will the number of repeats of each disaccharide unit (typically 8 to 15 of each, but not necessarily in equal proportion) in a given sample. Thus, for example a given heparin sodium tetrasaccharide unit may well be composed of two D-glucosamine-L-iduronic acid disaccharide units or two D-glucosamine-D- glucuronic acid disaccharide units rather than one of each disaccharide, and a particular heparin sample may well contain tetrasaccharide units of each of these three types. The expression "[heparin unit]" as used herein is intended to encompass any such units, save of course for the absence of some or all of the sodium cations, as explained above; in any given multiplet of this invention, the heparin units will be identical to those in the heparin sodium sample from which the multiplet is derived except for the difference in sodium content.

The expression "polyol" as used herein is intended' to indicate a monosaccharide, oligosaccharide, C₅―C₁₈ alicyclic polyhydroxy compound or C₃―C₁₅ aliphatic polyhydroxy compound, i.e. a compound of the formula 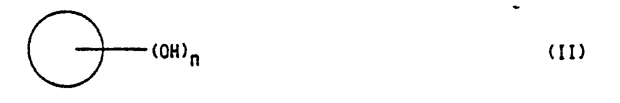 wherein 0 and n are defined as before. The hydroxy groups are usually situated on the carbon atoms in the polyol backbone or skeleton, but in some cases are located on pendant methyl radicals, particularly in the case of the monosaccharides and oligosaccharides.

When the polyol is an aliphatic polyol, it is preferably a C₃―C₈ alkyl polyol such as glycerol, erythritol (tetrahydroxybutane) or 1,2,6-trihydroxyhexane.

When the polyol is an alicyclic polyol, it is preferably a C₅―C₁₀ cycloalkyl or fused fully hydrogenated aromatic polyol (e.g. a cyclohexane or decahydronaphthalene polyol). A particularly preferred cycloalkyl polyol is inositol, a non-toxic substance which is widely distributed in plants and microorganisms. Inositol, which has the empirical formula C₆H₁₂O₆, has a number of possible stereoisomers. Shown below is the structure for cis-1,2,3,5-trans-4,6-cyclohexanehexol, the prevalent natural form:

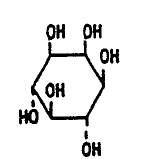

When the polyol is a monosaccharide, it is preferably a pentose, hexose or heptose. Suitable such simple sugars include aldopentoses such as ribose, arabinose, xylose and lyxose; ketopentoses such as ribulose and xylulose; aldohexoses such as glucose, galactose and mannose; ketohexoses such as fructose, sorbose and tagatose; and ketoheptoses such as mannoheptulose and sedoheptulose. Structures for some typical monosaccharides contemplated by the present invention are as follows:

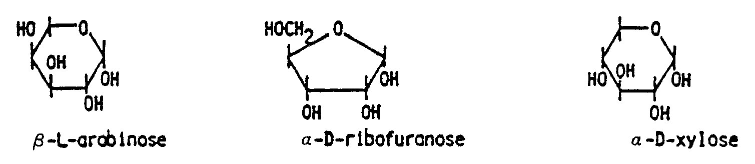

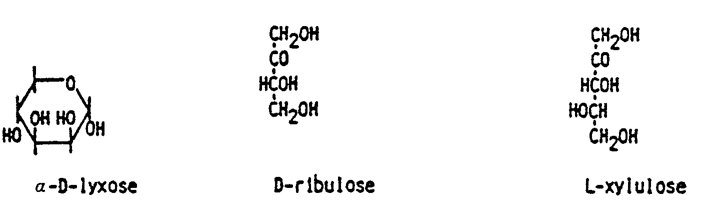

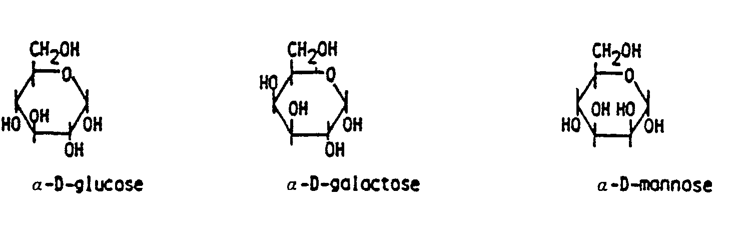

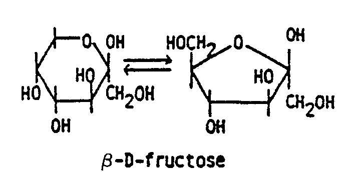

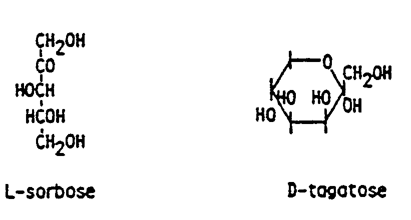

When the polyol is an oligosaccharide, i.e. a carbohydrate which yields 2 to 8 monosaccharide units upon acid hydrolysis, it is preferably a disaccharide, a trisaccharide or a cyclodextrin. Representative disaccharides are sucrose, lactose and maltose. A representative trisaccharide is raffinose. The cyclo- dextrins, or cycloamyloses, are homogeneous cyclic a-(1→4) linked D-glucopyranose units. a-Cyclodextrin contains 6 units, β-cyclodextrin contains 7 and y-cyclodextrin contains 8. Structures for representative oligosaccharides contemplated by the present invention are as follows:

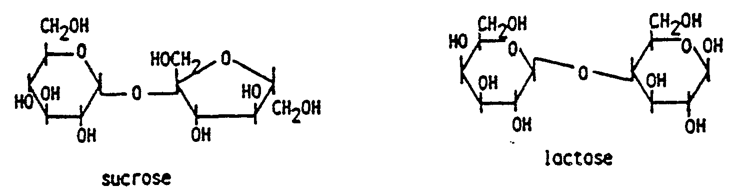

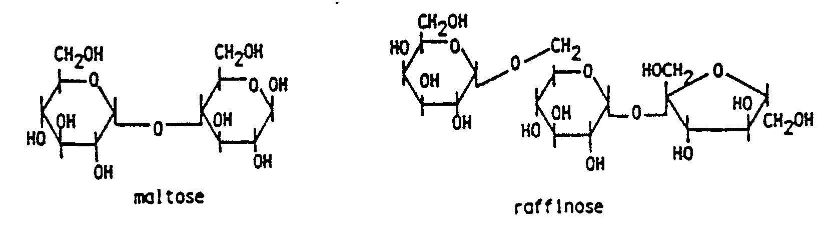

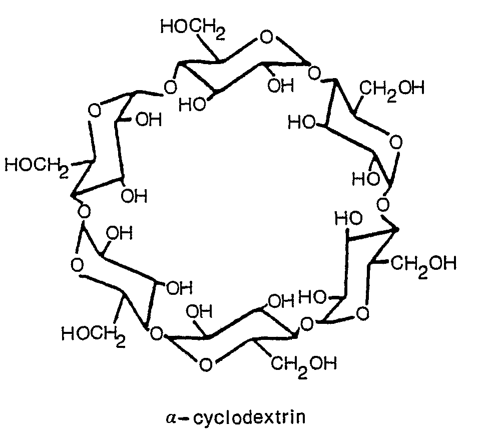

The expression "C₁-C₃ alkyl" and "C₁―C₇ alkyl" as used herein indicate straight or branched-chain groups containing the indicated number of carbon atoms, e.g. methyl, ethyl, propyl, isopropyl, as well as the higher homologues when the alkyl radical contains more than 3 carbon atoms, e.g. butyl, pentyl, hexyl and heptyl and the corresponding branched-chain isomers, e.g. isobutyl and tert-butyl.

The group "R"' can be hydrogen, as in glycine; methyl, as in alanine; -CH(CH₃)₂, as in valine; ―CH₂―CH(CH₃)₂, as in leucine; 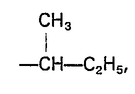 as in isoleucine; 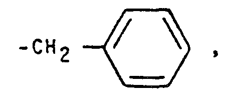 as in phenylalanine; 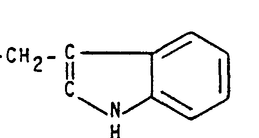 as in tryptophan; -CH₂0H, as in serine; -CHOH-CH₃, as in threonine; -(CH₂)₂-SCH₃, as in methionine; -CH₂-CONH₂, as in asparagine; ―CH₂CH₂―CONH₂, as in glutamine; 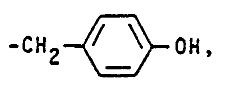 as in tyrosine; -CH₂SH, as in cysteine; -CH₂COOH, as in aspartic acid; and ―CH₂CH₂COOH, as in glutamic acid.

When R' and R" are combined with the adjacent nitrogen atom such that 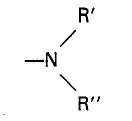 represents the residue of a saturated monocyclic secondary amine, such monocycles preferably have 5 to 7 ring atoms, optionally contain another hetero ring atom (-0-, ―S― or-N-) in addition to the indicated nitrogen atom, and optionally bear one or more substituents such as methyl. Illustrative of residues of saturated monocyclic secondary amines which are encompassed by the 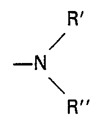 term are morpholino, 1-pyrrolidinyl, perhydro-1,2,4-oxathiazin-4-yl, 1- or 4-piperazinyl, 4-methyl-1-piperazinyl, piperidino, hexamethyleneimino, 2-methyl-1-pyrazolidinyl, 1- or 2-pyrazolidinyl, 3-methyl-1-imidazolidinyl and 1- or 3-imidazolidinyl.

When R⁺ in formula (I) is structure (a), R^{iv} is preferably methyl and the -COO- group is preferably located in the 3-position. When R⁺ in formula (I) is structure (b), R^{iv} is preferably methyl and the -CH₂COO- group is preferably located in the 3-position. When R⁺ in formula (I) is structure (c), R^{iv} is preferably methyl and the ―CH₂OCOO― group is preferably located in the 3-position. When R⁺ in formula (I) is structure (d), preferably R' and R" are both methyl or both ethyl, or R'R"N- represents morpholino, piperidino, 1-pyrrolidinyl or 1-piperazinyl; R^{iv} is preferably methyl; alkylene is preferably-CH-; and R'" is preferably 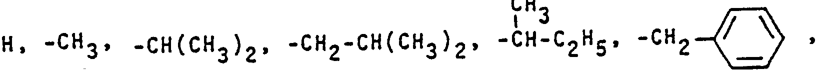 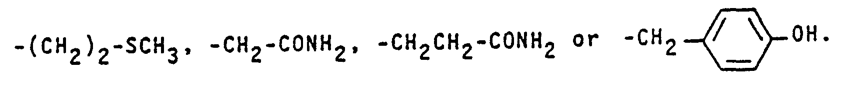 When R⁺ in formula (I) is structure (e), alkylene is preferably -CH₂CH₂-; and R^{v} is preferably H or -CONH₂. Structures (a), (d), and (e) are particularly preferred, especially when one or more of the structural variables therein are as set forth in this paragraph.

In formula (I), it is also preferred that p be equal to n so that n minus p equals zero, in which case the heparin multiplets can be represented by the formula 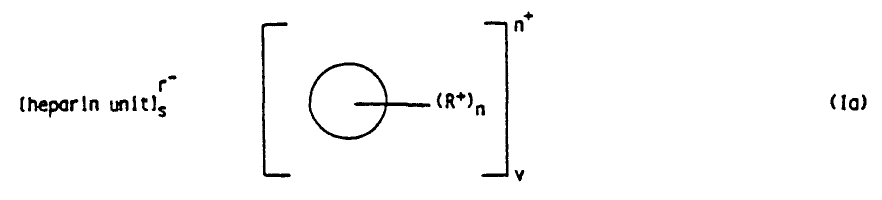 wherein the structural variables are as generally defined above. Especially preferred multiplets of formula (la) are those in which one or more of the structural variables are selected from the preferred variables set forth in the preceding paragraph.

It is also preferred that there be at least four R⁺ groups in the multiplets of formulas (I) and (la). Moreover, it is preferred that r in formulas (I) and (la) be 5, 6 or 7.

In one preferred embodiment of this invention in formula (I), O is the skeleton of inositol, n is 6 and p is 6. In one especially preferred embodiment of this invention, in formula (I), O is the skeleton of inositol, n is 6, p is 6, v is 5, r is 5 and s is 6.

The "ion-paired" complex or multiplet formed by this interaction of the heparinic acid anion and the quaternary ammonium cation is expected to be hydrophobic and absorbable from the intestine. The strong ionic interaction is expected to give high stability to the ion pairs. The complexity and high hydrophobicity of the products are expected to reflect slow dissociation, whether before, after or during absorption and, hence, slow release of heparinic acid and longer duration of action.

The multiplets of formula (I) can be prepared by a variety of conventional synthetic methods. One general method comprises coupling the selected polyol of formula (II) hereinabove with a quaternary acid salt of the formula 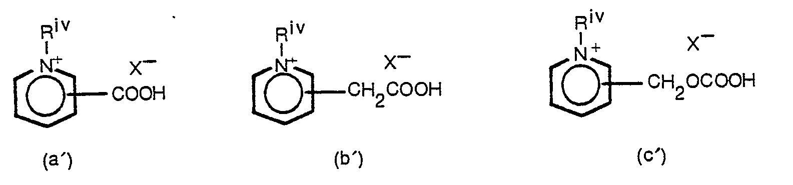 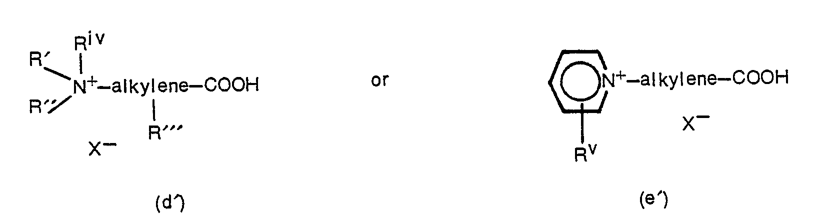 wherein the structural variables are as hereinbefore defined in connection with structures (a), (b), (c), (d) and (e), respectively, and X- is the anion of a pharmaceutically acceptable organic or inorganic acid. The coupling, or esterification, is typically carried out in the presence of a suitable coupling agent such as dicyclohexylcarbodiimide, in an appropriate organic solvent, using excess acid reactant so that as many as possible of the hydroxy groups in the polyol will be esterified. Other esterification procedures will be readily apparent to the skilled organic chemist, e.g. use of a mixed anhydride. The resultant novel intermediates can be represented by the structural formula 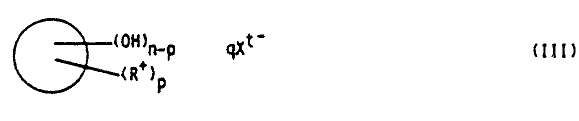 wherein 0, n, p and R⁺ are as defined in connection with formula (I); X- is the anion of a pharmaceutically acceptable acid (e.g. an inorganic acid such as hydrochloric acid, sulfuric acid or hydrobromic acid, or an organic acid such as acetic acid, oxalic acid, succinic acid, maleic acid, fumaric acid, malic acid, tartaric acid, citric acid, malonic acid, methanesulfonic acid or benzoic acid); t is the valence of the acid anion and q is the number which when multiplied by t is equal to p. Thus, when X^{t}- is a monovalent anion, q will be equal to p; when the anion is divalent, q will be equal to 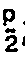 ; and when the anion is trivalent, q will be qual to 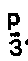 . Further, it is preferred that p be equal to n so that n-p = 0, the quaternary intermediate in such case being represented by the formula 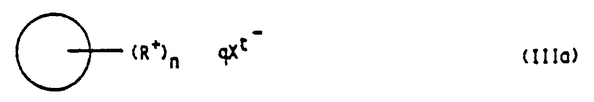 wherein the structural variables are defined as above. Preferred polycationic quaternary intermediates are those which lead to the preferred formula (I) ion pairs already discussed above. Especially preferred formula (III) intermediates are derived from nontoxic naturally-occurring substances, and are themselves non-toxic and will be metabolized to non-toxic moieties. Presently preferred quaternaries can be considered to be derived from inositol as the polyol and trigonelline (which occurs in the seeds of many plants and is excreted in the urine after taking nicotinic acid) and betaine (which is widely distributed in plants and animals) as the quaternized acid portion. The structures of trigonelline and betaine are as follows:

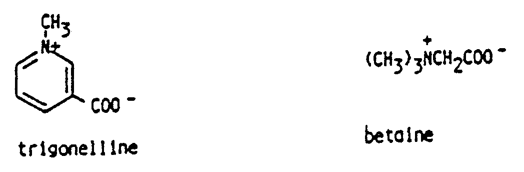

The novel quaternary ester intermediates of formula (III) are then conveniently reacted with heparin sodium, in aqueous medium, to afford the desired multiplets of formula (I). Typically, equivalent amounts of reactants are employed in this step. As many sodium ions as possible will be replaced with quaternary ester groupings in this step, i.e. at least 3, but typically 5 to 7. While not wishing to be bound by this interpretation, it is believed that when heparin sodium has the structure depicted earlier in this specification, of the seven sodium cations per tetrasaccharide unit, those Na⁺'s associated with the CO₂⁻ groupings are more tightly held than those associated with SO₃⁻ groupings and are consequently easier to replace with the instant cationic entities; and that generally at least the five S03 anions will become associated with the new cations.

Nevertheless, other synthetic routes to the instant multiplets are presently preferred. Thus, the presently preferred process for the preparation of the multiplets of formula (I) wherein R⁺ is structure (a), (b), (c) or (d) is to first couple the selected polyol of formula (II) with a tertiary acid of the formula 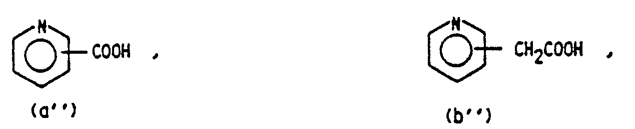 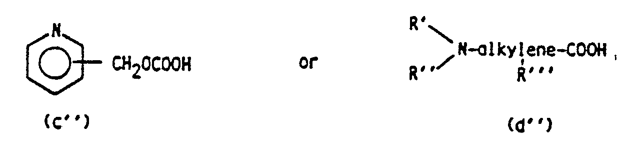 or the corresponding acid halide (e.g. chloride) or anhydride, wherein the structural variables are as previously defined in connection with structures (a), (b), (c) and (d), respectively. When the esterifying agent is an acid, the process is typically carried out in the presence of a suitable coupling agent such as dicyclohexylcarbodimide (DCC) in an appropriate organic solvent such as pyridine, acetonitrile or dichloromethane, the solvent of choice depending upon the particular reactants employed. When the esterifying agent is an acid anhydride (prepared, for example, by reacting the corresponding acid with phosgene), an organic solvent such as pyridine can be employed. When the esterifying agent is an acid chloride (prepared, for example, by reacting the corresponding acid with a chlorinating agent such as thionyl chloride or phosphorous oxychloride), choice of solvent will vary not only with the particular reactants used but also with whether or not the acid chloride is isolated prior to the esterification step. For example, when the acid chloride is isolated as the hydrochloride, then that salt can be conveniently reacted with the polyol in chloroform. On the other hand, if the acid is first reacted with POCI₃ and the polyol subsequently added to the reaction mixture, both steps can be conveniently conducted in pyridine. Whatever the esterifying agent, it will be used in excess so as to esterify as many of the hydroxy groups in the polyol as possible, e.g. when inositol is used as the polyol, a six-fold or greater excess of monocarboxylic acid or acid chloride will be used (i.e. 6 or more moles of acid or acid chloride per mole of polyol). When a starting acid of structure (d") or the corresponding acid chloride or anhydride is used, the R'" group therein preferably does not contain free hydroxy, thiol or carboxy functions which could interfere with coupling with the polyol structure. However, such functions can be conveniently protected, if desired, e.g. with ester groupings (for example, Cₗ-C₇ lower alkyl esters) prior to acylation and subsequently deprotected. Also, carboxy functions contained in the R'" grouping need not be protected if the polyol contains sufficient hydroxy groups; e.g., if R'" is -CH,CH₂COOH, then each amino acid could combine with two hydroxy groups and at least six hydroxy groups would need to be present in the polyol in order to give an acylated derivative in which there are sufficient R groups, i.e. at least three.

The products of the above-described first step of the presently preferred process for the preparation of the formula (I) multiplets in which R⁺ is structure (a), (b), (c) or (d) can be represented by the structural formula 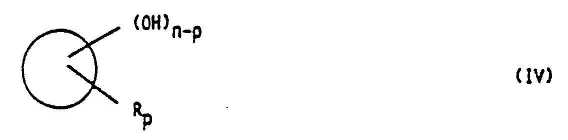 wherein 0, n and p are as defined in connection with formula (I) and R is the residue of one of the aforementioned acids (a") to (d"), i.e. R is 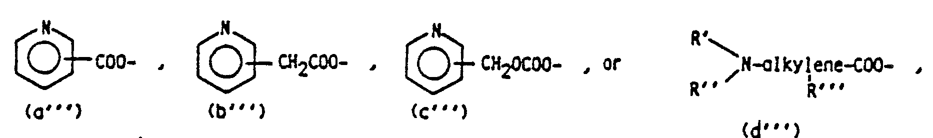 wherein the structural variables are as defined in connection with structures (a), (b), (c) and (d) hereinabove. Preferably, p is equal to n in formula (IV) so that n-p = 0, the acylate in such case being represented by the formula 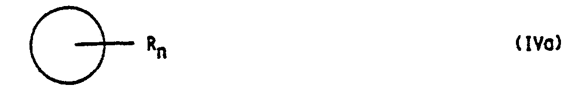 wherein R and n are defined as above.

The acylates of formula (IV) are then quaternized, typically by treatment with a Cₗ-C₃ alkyl halide, R^{iv} -Hal, preferably methyl iodide, in an appropriate solvent such as dimethylformamide. Preferably, the formula (IV) acylate is heated (e.g. at about 50°C) with excess of the alkylating agent in dimethylformamide for at least forty-eight hours. The resultant quaternized acylate wherein the anion is I- or other halide can then be subjected to anion exchange when an anion is desired which is different from the one obtained; such anion exchange may be accomplished via an anion exchange resin or, more conveniently, by use of the method of Kaminski et al, Tetrahedron, Vol. 34, pp. 2857-2859 (1978). According to the Kaminski et al method, a methanolic solution of a pharmaceutically acceptable organic or inorganic acid HX will react with a quaternary ammonium halide to produce the methyl halide and the corresponding quaternary X salt. The quaternization and when desired the subsequent anion exchange afford the novel chemical intermediates of formula (III) hereinabove wherein R⁺ is structure (a), (b), (c) or (d) as defined with formula (1). Again, the preferred compounds of formula (III) are as discussed in connection with the first synthetic route discussed above. And again, those novel intermediates can then be reacted with heparin sodium as already described above to afford the corresponding heparin multiplets of formula (I).

A presently preferred process for the preparation of the multiplets of formula (I) wherein R⁺ is structure (e) utilizes a starting material of the formula 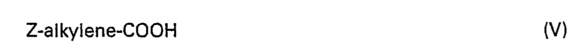 or 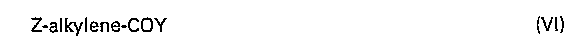 wherein Z is Cl, Br or I; the alkylene group can be straight or branched and contains 1 to 3 carbon atoms; and Y is CI or Br. The formula (V) or (VI) starting material is first reacted with a polyol of formula (II) above to afford an intermediate polyester of the formula 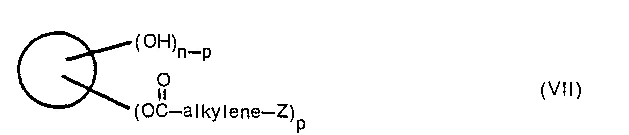 wherein O, n and p are as defined in connection with formula (I) above (i.e. O is the skeleton of a polyol, n is a number from 3 to about 24 which represents the total number of hydroxy groups in the polyol, and p is a number ≥ 3 and ≤ n); and alkylene and Z are defined as in connection with formulas (V) and (VI) above. When a formula (V) starting material is used, the reaction is conducted in the presence of suitable coupling agent such as dicyclohexylcarbodiimide. Alternatively, a mixed anhydride or activated ester may be used. When a formula (VI) starting material is used, the reaction is typically conducted in the presence of an acid scavenger such as triethylamine, in a halogenated hydrocarbon solvent, e.g. chloroform. The resultant polyester of formula (VII) is then converted to the desired quaternary ester intermediate of formula (III) wherein R⁺ is structure (e) by contacting said polyester with excess reactant of the formula 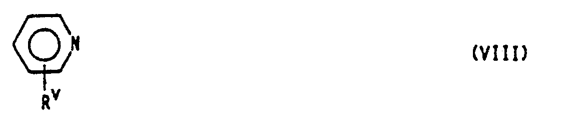 wherein R" is located in the 2-, 3- or 4-position and is H, -CONH₂ or ―COO(C₁―C₇ alkyl), in a polar solvent such as nitromethane, dimethylformamide, acetonitrile, acetone, tetrahydrofuran or ethyl ether, followed by isolation by crystallization. The novel quaternary intermediate thus obtained wherein the anion is CI-, Br⁻ or I- can then be subjected to anion exchange (via an anion exchange resin or the Kaminski et al method described hereinabove), when an intermediate is desired in which a different anion is present. Any of these novel quaternary intermediates can then be reacted with sodium heparin as already described above to give the corresponding heparin multiplets of formula (I).

A highly desirable alternate process for the preparation of the multiplets of formula (I), particularly for the multiplets wherein R⁺ has structure (a) as defined in connection with formula (I), utilizes an activated ester, quaternized activated ester or quaternized anhydride as a starting material.

In order to prepare a multiplet of formula (I) wherein R⁺ has structure (a) using an activated ester starting material, an activated succinimidyl ester or phthalimidyl ester of the formula 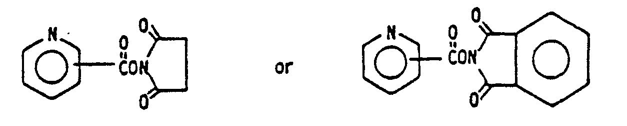 or similar activated ester (prepared, for example, by reacting the appropriate acid such as nicotinic acid with N-hydroxysuccinimide or N-hydroxyphthalimide, respectively), is first reacted with the selected polyol of formula (II). The resultant intermediate of formula (IV) is then converted to a formula (I) derivative by methods fully described hereinabove (i.e. quaternization with R^{iv}-Hal, followed by ion exchange (if desired), and then reaction of the novel formula (III) salt thus obtained with sodium heparin).

In order to prepare a multiplet of formula (I) wherein R⁺ has structure (a) using a quaternized activated ester starting material, an activated ester such as the succinimidyl or phthalimidyl ester depicted above is first quaternized with R^{iv}-Hal; the resultant quaternized activated ester is then subjected to ion exchange (if desired), and subsequently reacted with the selected polyol of formula (II) to give the desired formula (III) intermediate, which is then reacted with sodium heparin to give the formula (I) multiplet.

To prepare a multiplet of formula (I) wherein R⁺ has structure (a) using a quaternized anhydride starting material, a quaternized anhydride of the formula 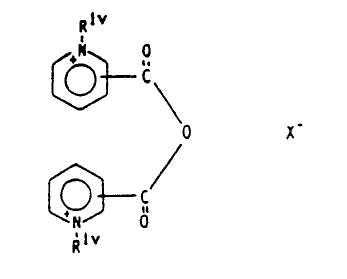 is first prepared by reacting the desired anhydride, e.g. nicotinic anydride, with a 2 molar excess of R^{iv}-Hal in an appropriate solvent, e.g. acetonitrile, the structural variables in the general formula being defined as hereinabove. The reaction mixture typically is stirred at reflux for 48 hours. The resultant quaternized anhydride is then reacted with the selected polyol of formula (II), in a suitable solvent such as pyridine or pyridine/dimethylformamide, to give the desired formula (III) intermediate, which is then reacted with sodium heparin to give the formula (I) multiplet.

In all of the various alternate process schemes described above, the final step comprises reacting a novel quaternary intermediate of formula (III) with sodium heparin to give the corresponding heparin multiplets of formula (I). In any of these schemes, that final step can be replaced with a two step procedure comprising first subjecting the formula (III) quaternary intermediates to ion exchange to replace the X-anions with OH- anions, and then reacting the resultant compound with heparin acid to give the formula (I) derivative. In the first step, a strongly basic anion exchange resin is employed to exchange the I- or other X- groups for OH⁻ groups; the formula (III) intermediate is typically dissolved in water and applied to a column of the resin, and the column is washed with water to elute the corresponding compound of the formula 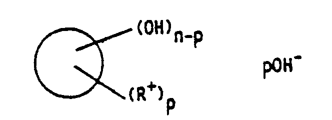 wherein O, n, p and R⁺ are as defined with formula (I) and (II) hereinabove. The resultant intermediate is then reacted with heparinic acid, typically in water or water/alcohol, to afford the corresponding compound of formula (I) wherein the heparin unit has a valence r of 7.

The final products and intermediates prepared by the synthetic procedures detailed above can be readily isolated and purified by usual separation means, for example, solvent extraction, dilution, recrystallization, column chromatography or preparative thin-layer chromatography.

The multiplets of formula (I) can be conveniently administered to warm-blooded animals via conventional oral, nasal or pulmonary (i.e. oral inhalation) administration, preferably by combining the active ingredient of formula (I) with a suitable non-toxic pharmaceutically acceptable inert oral, nasal or pulmonary carrier material, respectively. Such carrier materials are well-known to those skilled in the art of pharmaceutical formulations. For those not skilled in the art, reference is made to the text entitled REMINGTON'S PHARMACEUTICAL SCIENCES, fourteenth edition, 1970.

In a typical unit dosage form for oral administration, e.g. tablet or capsule, any one of the multiplets of formula (I) is combined, in an effective anticoagulant amount, with an oral non-toxic pharmaceutically acceptable inert carrier such as lactose, starch (pharmaceutical grade), dicalcium phosphate, calcium sulfate, kaolin, mannitol or powdered sugar. Additionally, when required or desired, suitable binders, lubricants, wetting agents, surface-active agents, disintegrating agents, coloring agents, flavoring agents and preservatives can also be included. Typical binders include starch, gelatin, sugars such as sucrose, molasses and lactose, natural and synthetic gums such as acacia, sodium alginate, extract of Irish Moss, carboxymethyl cellulose, methyl cellulose, polyvinylpyrrolidione, polyethylene glycol, ethyl cellulose and waxes. Typical lubricants for use in these dosage forms can include, without limitation, boric acid, sodium benzoate, sodium acetate, sodium chloride, leucine and polyethylene glycol. Suitable disintegrators can include, without limitation, starch, methyl cellulose, agar, bentonite, cellulose, wood products, alginic acid, guar gum, citrus pulp, carboxymethyl cellulose and sodium lauryl sulfate. The amount of active ingredient present in the composition will generally be from about 1 to about 70% by weight of the total composition.

Typical dosage forms and carriers for nasal and oral inhalation therapy will obviously depend on the exact nature of the particular dosage form desired, e.g. whether the ion pair is to be formulated into a nasal solution or suspension (typically for use as nose drops or nasal spray), a nasal ointment, cream or gel, or a formulation for oral inhalation. Preferred dosage forms for nasal administration are solutions, which contain a major amount of water in addition to the active ingredient. Minor amounts of other ingredients such as pH adjusters (e.g. a base such as NaOH), emulsifiers or dispersing agents, buffering agents, preservatives, wetting agents and jelling agents (e.g. methylcellulose) may also be present. Most preferably, the nasal composition is a sterile, isotonic, buffered aqueous solution or suspension in polyethylene glycol. The amount of multiplet in the nasal composition will of course vary with the particular multiplet employed and the type of formulation selected. Generally speaking, the composition will contain 0.01 to 5% of a multiplet of formula (I), preferably 0.25 to 2.5%; in other words, each ml of solution or suspension will contain 0.1 to 50 mg, preferably 2.5 to 25 mg, of the formula (I) multiplet. In the case of oral inhalation therapy, compositions typically formulated for such route of administration may be used, a preferred dosage form being an oral inhalation aerosol containing the desired multiplet of formula (I) and fluorochlorohydrocarbons as propellants. Most preferably, the aerosol is provided with a metering mechanism to make accurate dosing possible. Each inhalation will typically deliver 0.1 to 50 mg, preferably 2.5 to 25 mg of the multiplet of formula (I).

The therapeutic dosage range for the multiplets of the instant invention will vary with the size and needs of the animal and the particular multiplet selected for administration. Generally speaking, the present "ion-pairs" can be orally administered in much smaller amounts by weight (e.g. less than one-half and less frequently than would be necessary for orally administered sodium heparin itself. Administration as infrequently as once every 24 to 48 hours may be possible using selected ion pairs of this invention. When nasal or inhalation administration is used, dosages and frequency of administration may be similar to oral dosages and frequency of the instant ion pairs, although smaller, more frequent doses may be preferred for nasal or inhalation therapy. In any case, amounts to be administered by the selected route can be calculated on a unit basis, based on the potency of the heparin sodium used to prepare the instant multiplets, the new unit/mg potency being calculated based on the increase in molecular weight. The number of units given will approximate 2 to 10 times those currently used parenterally in the case of heparin sodium.

In order to further illustrate the present invention and the advantages thereof, specific examples are given below, it being understood that these examples are intended only as illustrative and in no way limitative. In the examples to follow, all melting points are uncorrected and were obtained by using electrothermal capillary melting point apparatus. Elemental analysis were performed at Atlantic Microlabs, Inc., Atlanta, Georgia. In all cases where Anal. C, H, N is indicated, the elementary analysis of the material was found to be within ±0.4 of the calculated value.

### Example 1

To a suspension of 50 g (0.41 mol) of dry nicotinic acid in 135 ml of dry distilled pyridine were added 34 g of phosphorus oxychloride (POCl₃). The reaction mixture was stirred for one hour at 60°C, then 12.3 g (0.068 mol) of myo-inositol were added. The resultant mixture was maintained at 80°C, with stirring, for 3 hours, then was poured into 200 ml of ice cold water. The fine yellowish-white solid which separated was removed from the dark brown mother liquor by filtration, washed well with water and dried in a vacuum oven at 150°C. The solid was crystallized from a chloroform/ether solvent pair to afford 45 g (82% yield) of inositol hexanicotinate (1). The product melts at 258-260°C and can be represented by the structural formula

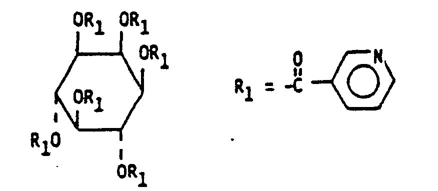

### Example 2

To a solution of 20 g (2.5 mmol) of inositol hexanicotinate (1) in 200 ml of dimethylformamide were added 11.2 g (0.08 mol) of methyl iodide. The mixture was heated at 50°C, under reflux, with stirring, for 48 hours. The fine yellow solid which separated was removed by filtration, washed with acetone and dried in a vacuum at 100°C. The product, obtained in 78% yield (3.2 g), was inositol hexa(1-methyl-3-pyridinium carboxylate)hexaiodide (2), melting at 250253°C with decomposition. NMR (D₂0) 5 9.6-7.96 (ms, 24H, pyridinium protons), 6.7-6.6 (m, 6H, inositol protons), 4.4 (s, 18H, 6 CH₃-N^{*}). Anal. (C₄₈H₄₈I₆N₆O₁₂) C, H, N. The product can be represented by the structural formula 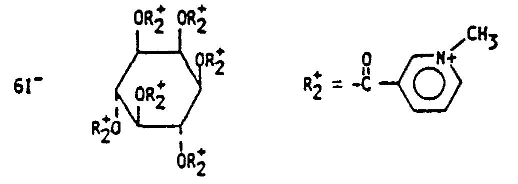 and can also be called hexa(N-methylnicotinoyloxy)-cis-1,2,3,5-trans-4,6-cyclohexane hexaiodide.

### Example 3

To a solution of 2.0 g (2.5 mmol) of inositol hexanicotinate (1) in 200 ml of dimethylformamide were added 6.6 g (0.053 mol) of dimethylsulfate. The mixture was heated, with stirring, at 60°C for forty-eight hours. The sticky, yellowish residue which separated on addition of ether was repeatedly washed with acetone and then dried in a vacuum oven at 50°C for twenty-four hours. There were thus obtained 2.5 g (64% yield) of inositol hexa-(1-methyl-3-pyridinium carboxylate) hexa(methylsulfate) (3) as a yellow, highly hygroscopic solid. NMR (D₂0) 6 9.60-8.03 (ms, 24H, pyridinium protons), 6.6-6.5 (m, 6H, inositol protons), 4.46 (s, 18H, 6 CH₃-N), 3.56 (s, 18H, 6 CH₃-OS0₃). Anal. (C₅₄H₆₆N₆O₃₆S·6H₂O) C, H, N. The product, which can also be named hexa(N-methylnicotinoyloxy)-cis-1,2,3,5-trans-4,6-cyclohexane hexa-(methylsulfate), can be represented by the structural formula

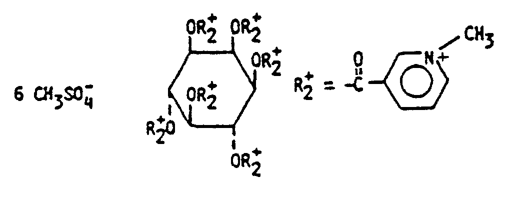

### Example 4

A solution of 1 mmol of the quaternary salt (2) or (3) in 10 ml of water was added, with stirring, to a solution of 1.14 g of heparin sodium (178.6 USP units/mg, 12% Na) in 10 ml of water. No precipitate was observed. On dilution with 100 ml of water, yellow oil droplet separated. The aqueous layer was removed by decantation and the residual oil was washed thoroughly several times with water. The well-drained oil was triturated with 50 ml of acetone. The oil solidified into a glistening yellow solid which was practically insoluble in organic solvents. The product weighed 1.6 g (84% of theoretical) and decomposed at 210°C. Use of the hexaiodide quaternary (2) gave a purer, easier to work with product than use of the hexa(methylsulfate) quaternary (3). Results of microanalysis for C, H, N and S for proposed formula 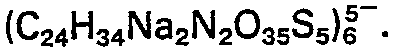 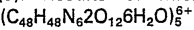 Calc: C, 39:32; H, 4:14; N, 5:01 S, 8:19. Found: C, 39:14; H, 4.72; N, 5.60; S, 7:64. The resultant heparin hexa(N-methylnicotinoyloxy)-cis-1,2,3,5-trans-4,6-cyclohexane multiplet (4) can also be represented by the structural formula

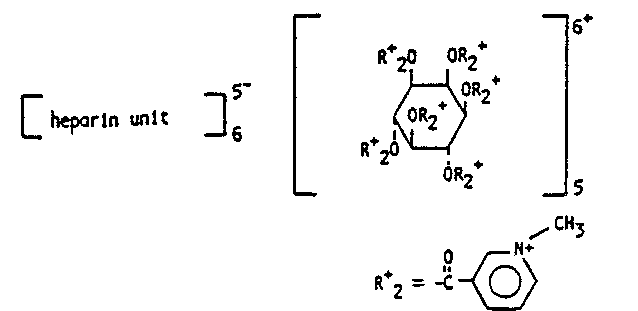

The anticoagulant activity of the product was calculated in relation to the sodium content of heparin sodium to be 107.6 units/mg.

### Example 5

A solution of 6 mmol of N,N-dimethyl-N-dodecyl-N-(a-hydroxyethyl)ammonium bromide in 10 ml of water was added, with stirring, to a solution of 1.14 g of heparin sodium (178.6 unit/mg, 12% Na) in 10 ml of water. A white precipitate was obtained. The mixture was centrifuged and the residue was washed thoroughly with water, then dried in a vacuum desiccator. The white mass was thus converted to a transparent, colorless plastic-like mass of heparin N,N-dimethyl-N-dodecyl-N-(β-hydroxyethyl)ammonium ion pair (5), which was soluble in methanol and ethanol, but insoluble in water, ether, chloroform or dichloromethane.

### Example 6

Substitution of an equivalent quantity of each of the starting materials listed below for the nicotinic acid employed in Example 1 and substantial repetition of the procedures there detailed affords, after suitable isolation, the indicated products of the formula

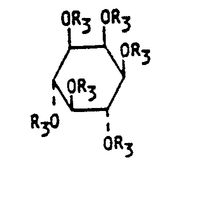

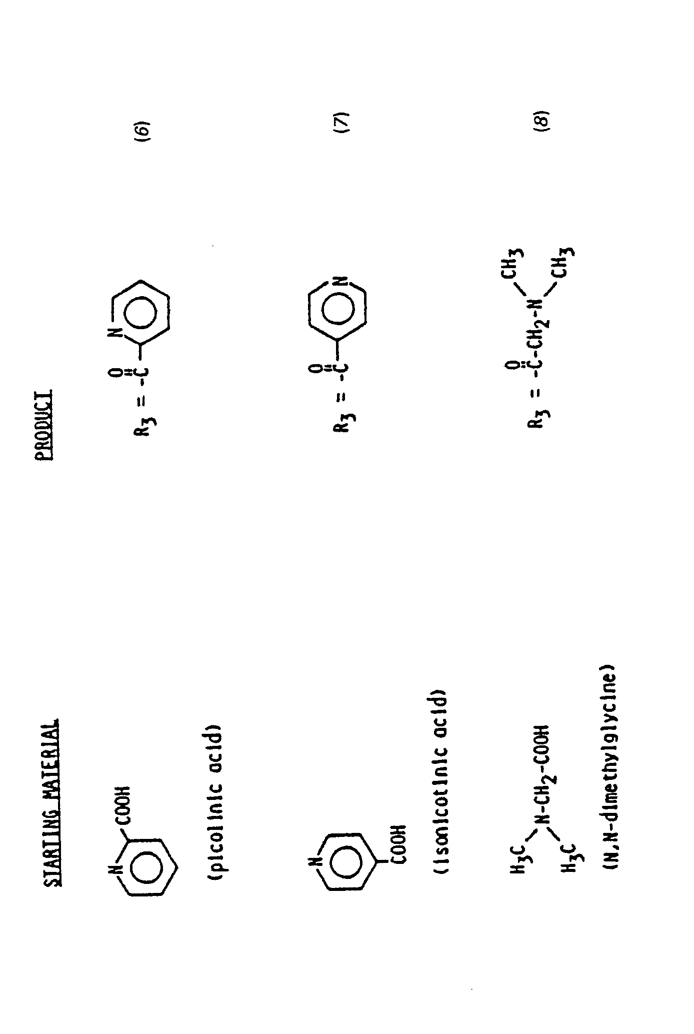

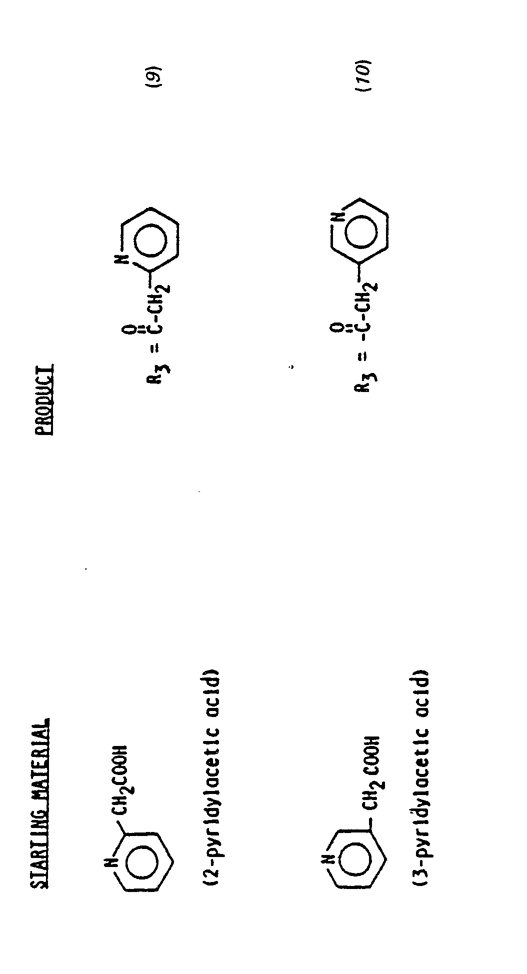

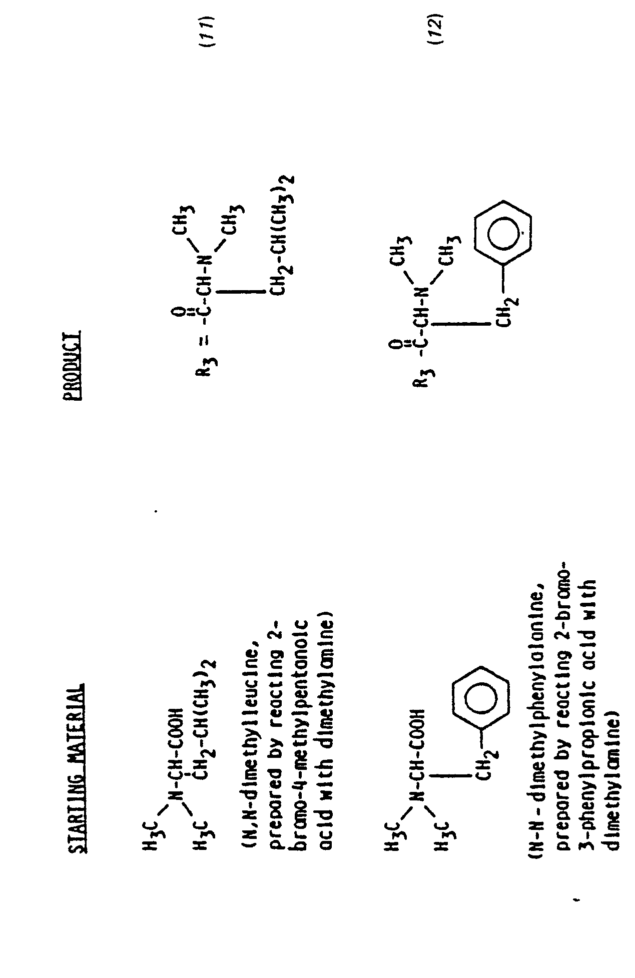

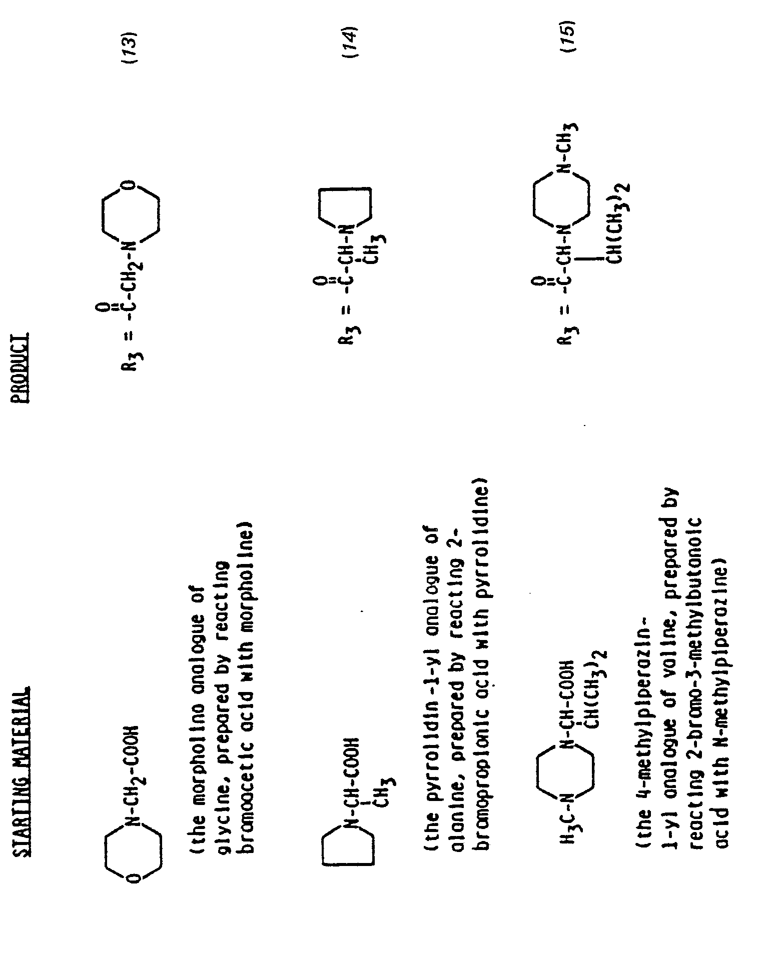

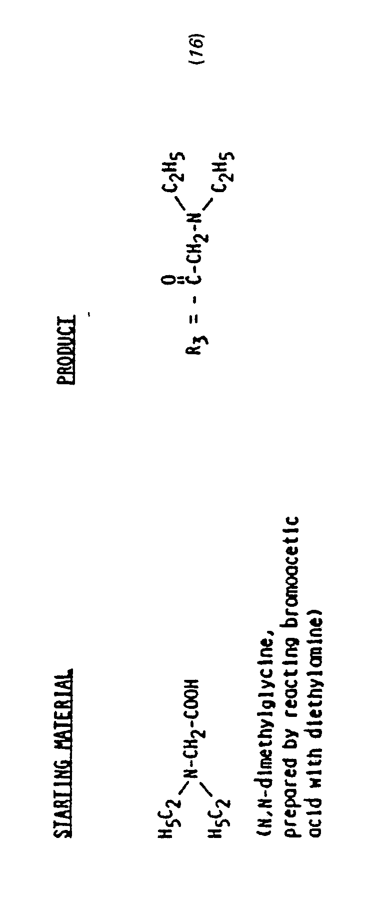

### Example 7

Substitution of an equivalent quantity of each of the starting materials listed below for the inositol employed in Example 1 and substantial repetition of the procedures there detailed affords, after appropriate isolation, the indicated products:

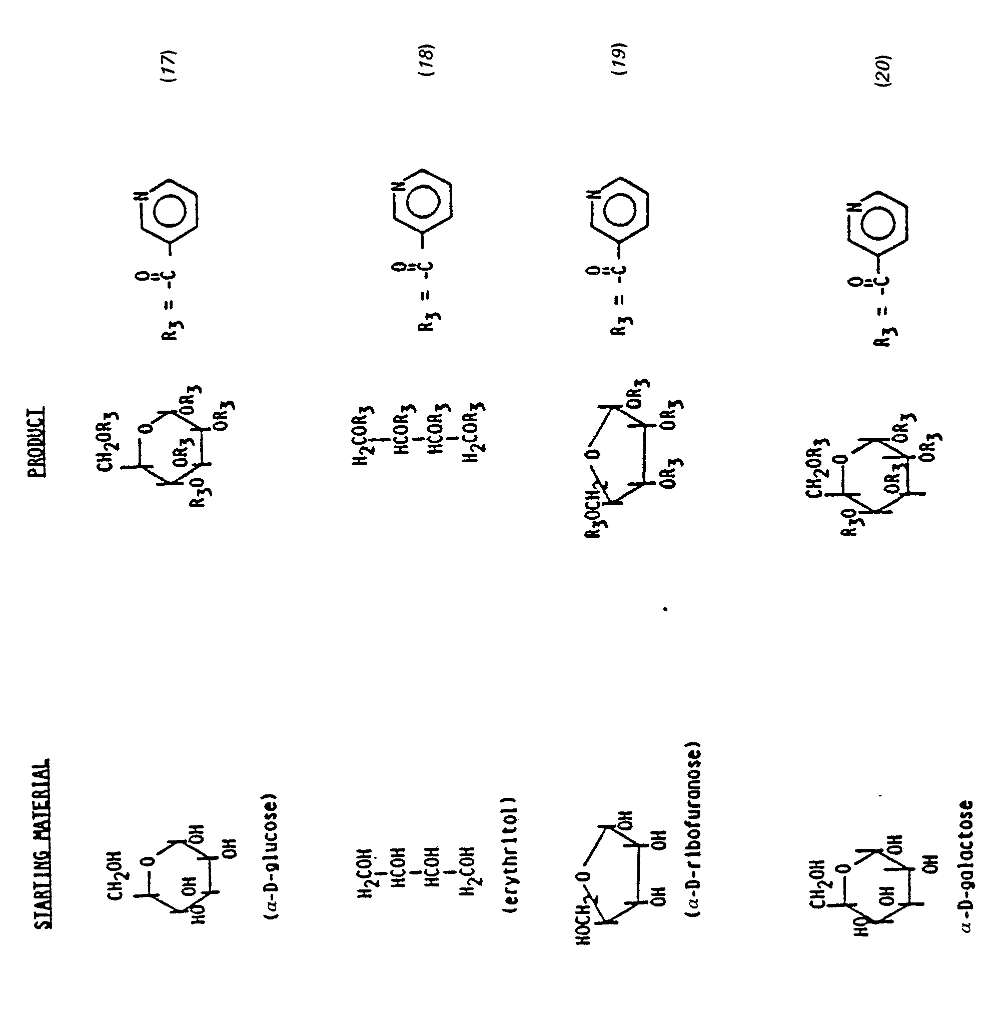

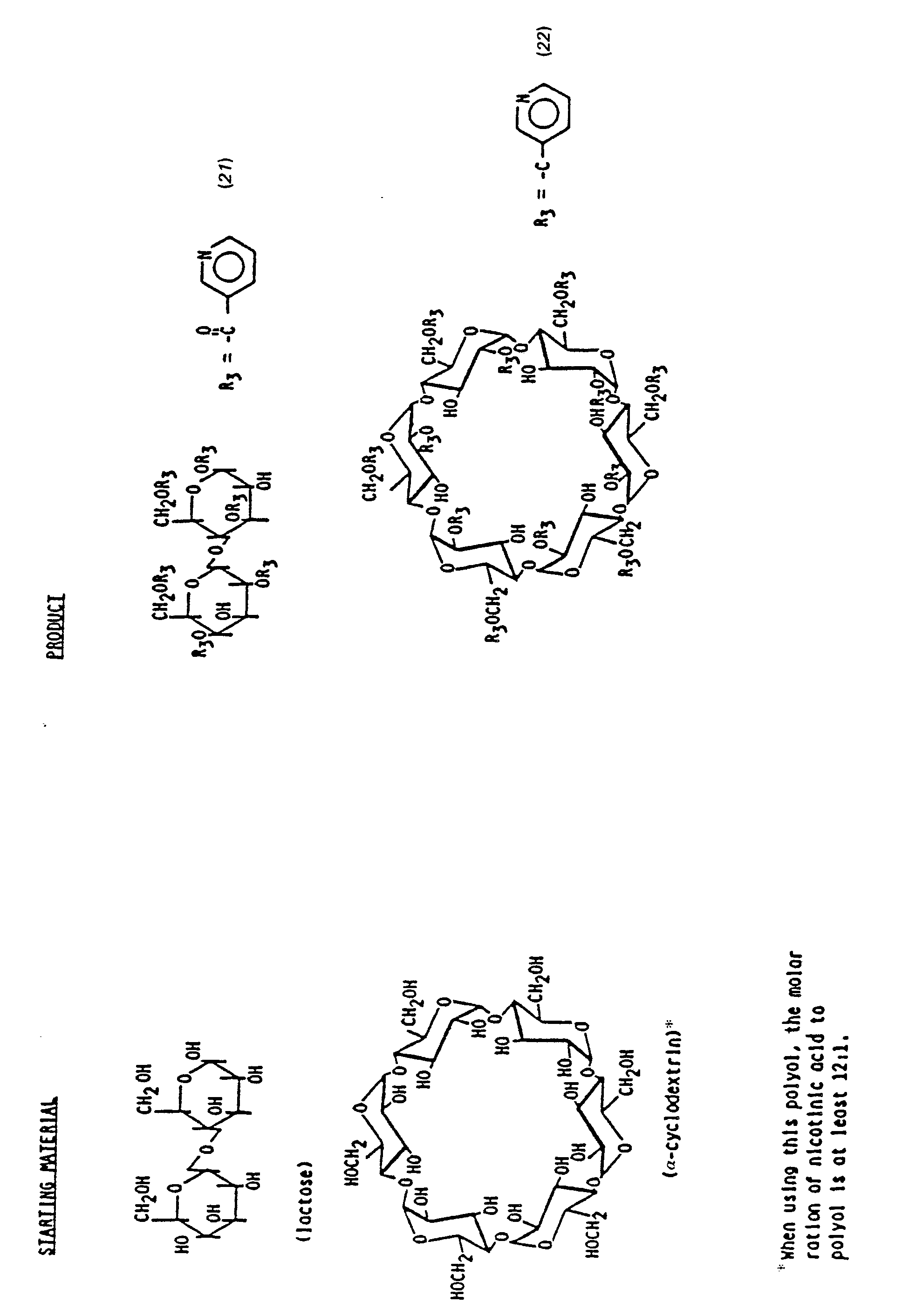

### Example 8

Substitution of an equivalent quantity of each of the starting materials listed in Column A below for the inositol used in Example 1 and substitution of an equivalent quantity of each of the starting materials listed in Column B below for the nicotinic acid used in Example 1 affords, by the procedures of that Example, the following products:

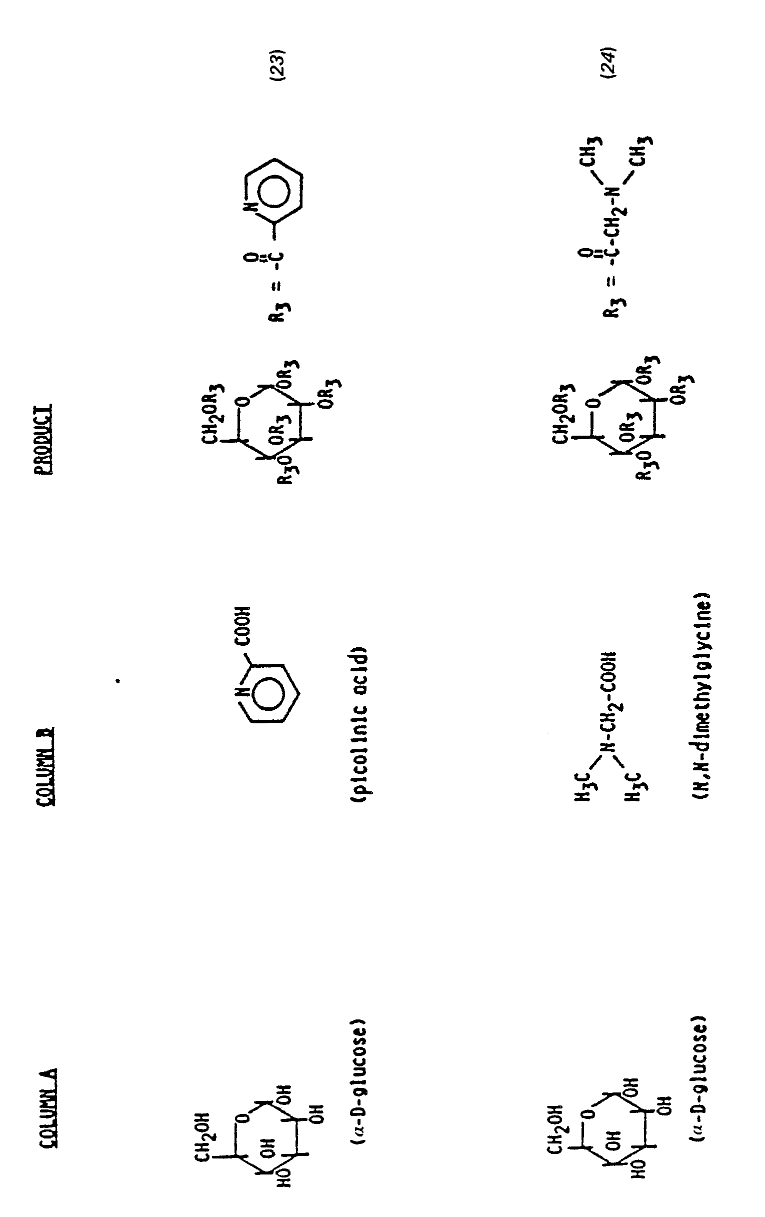

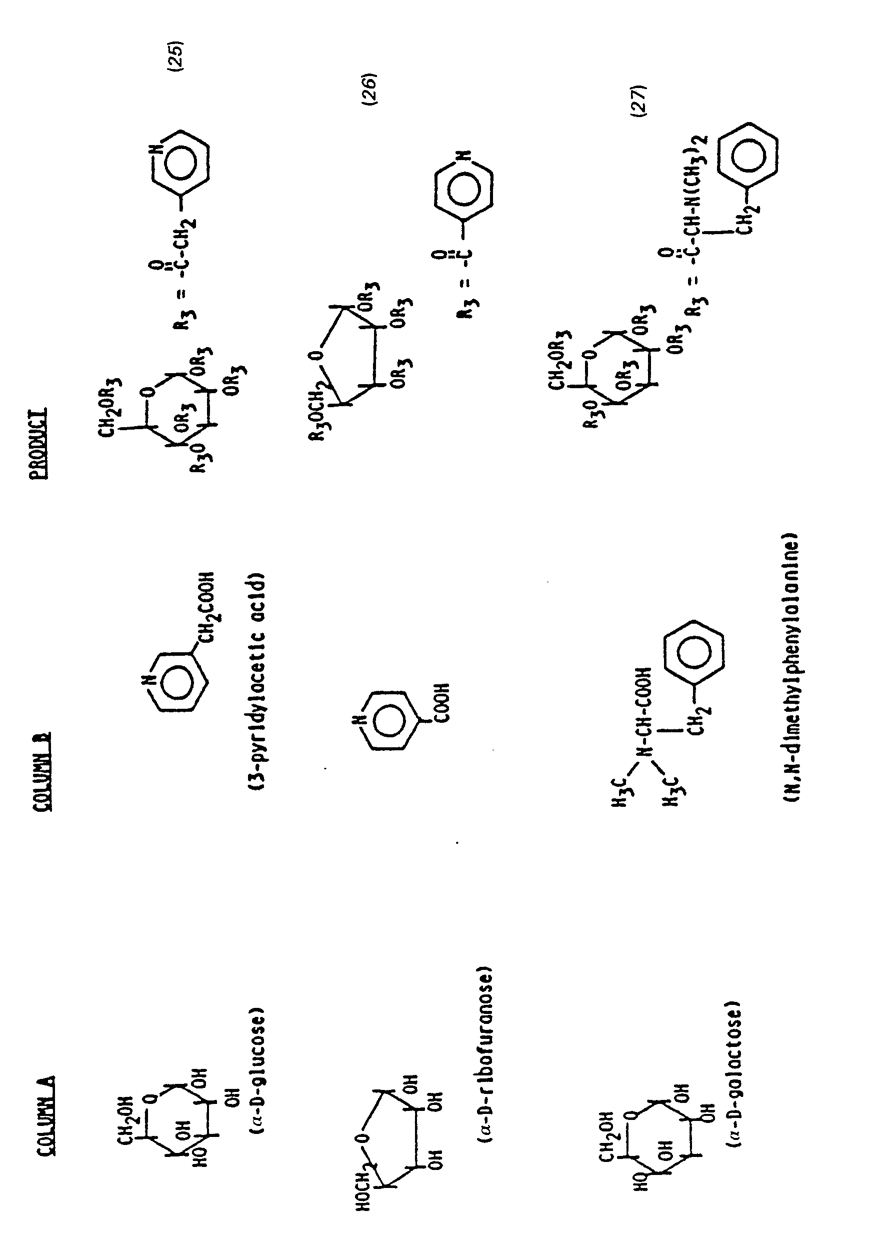

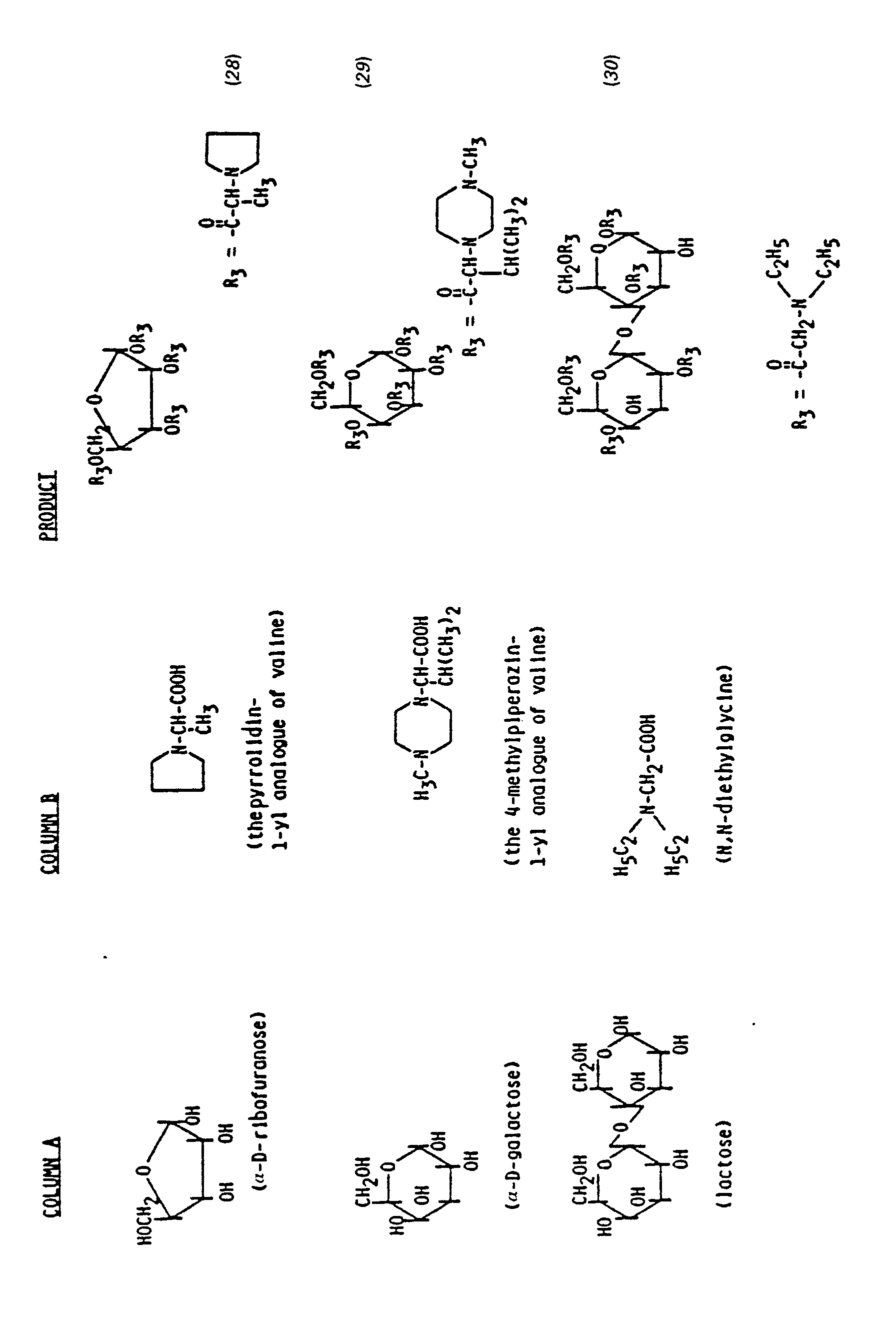

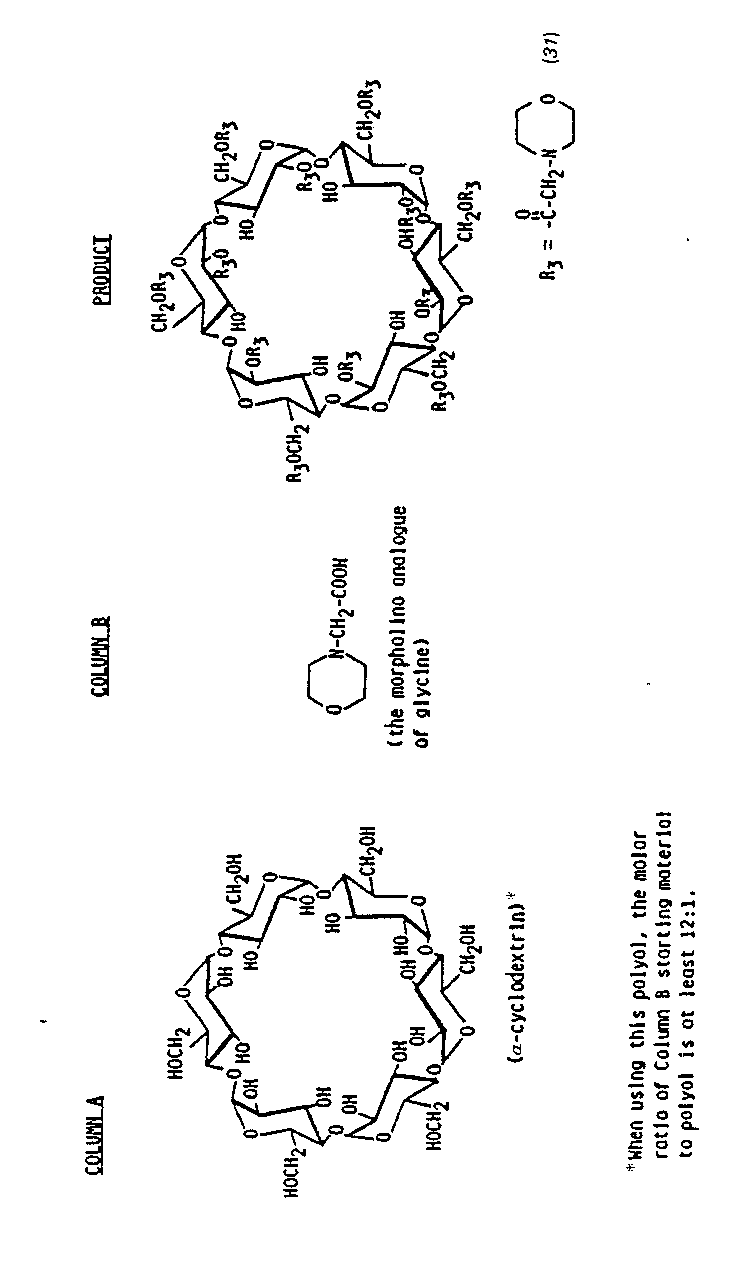

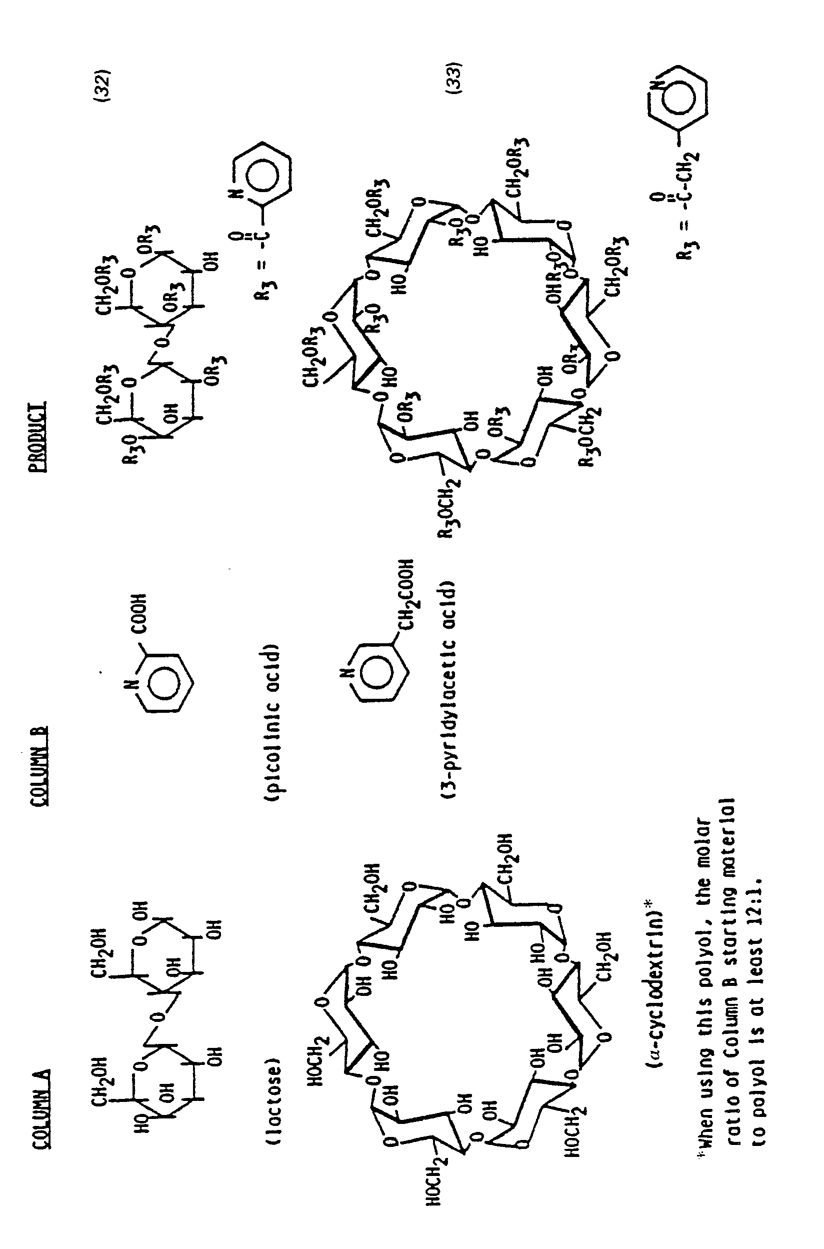

### Example 9

Quaternization of each of the products of Examples 6, 7 and 8 according to the general procedure of Example 2 affords, after appropriate isolation, the following products:

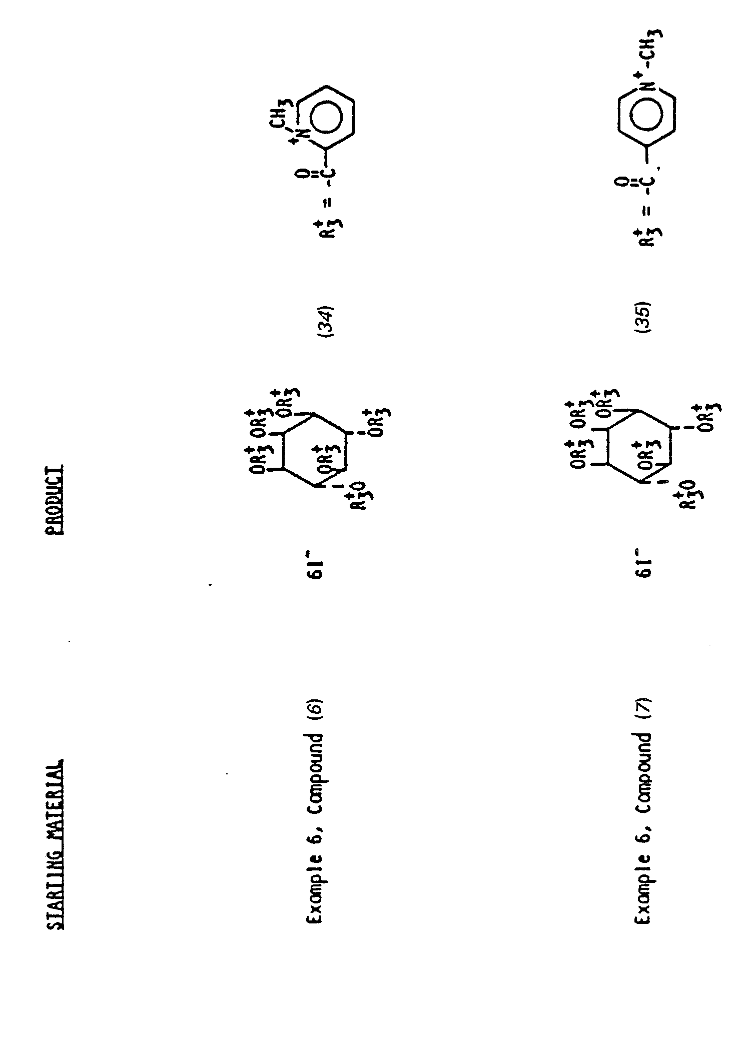

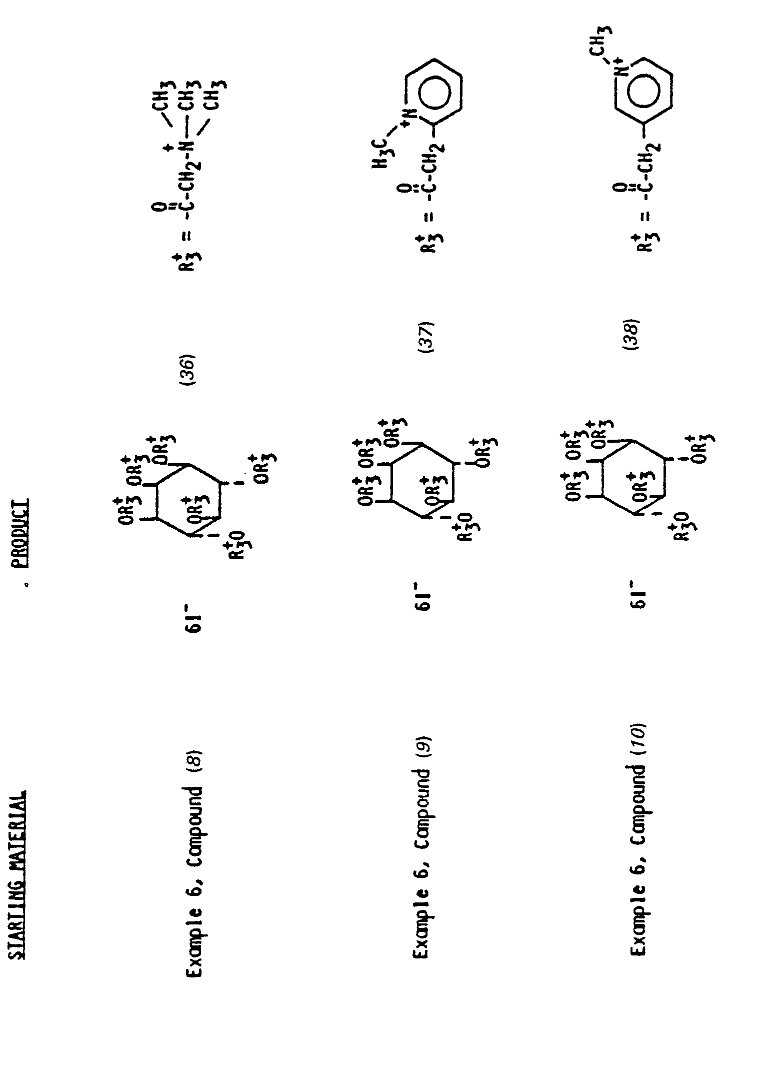

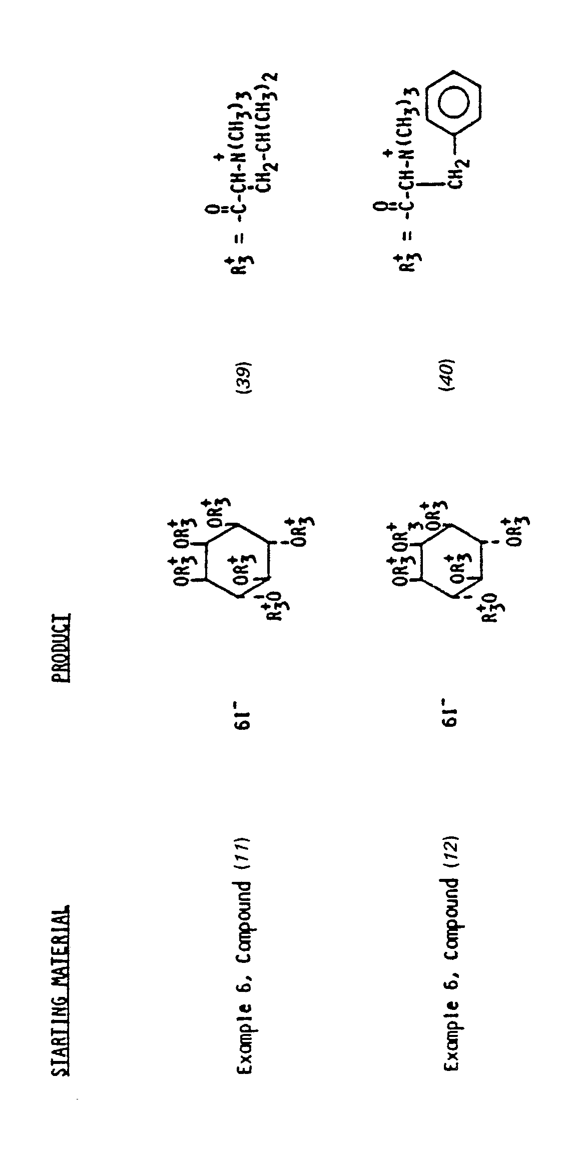

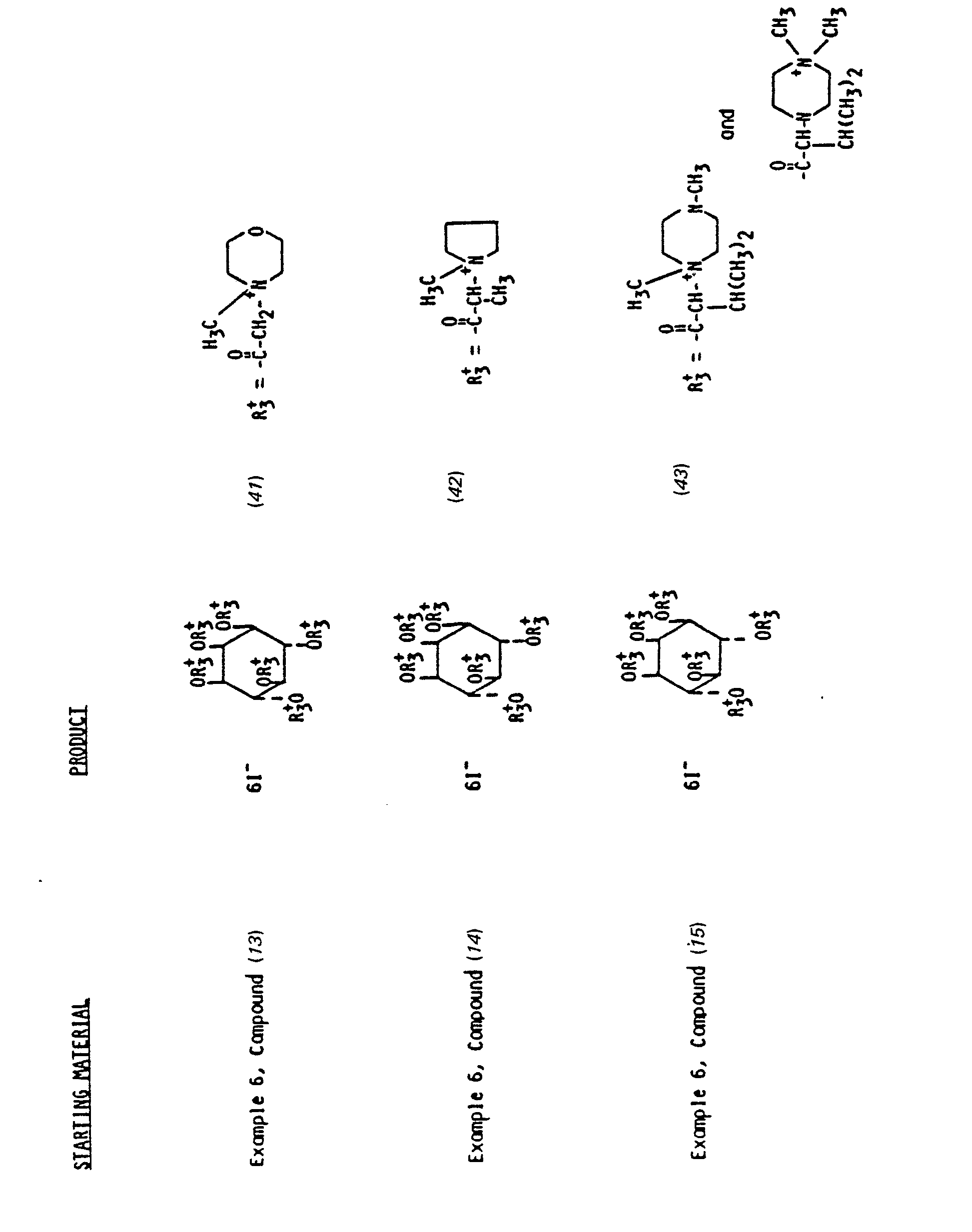

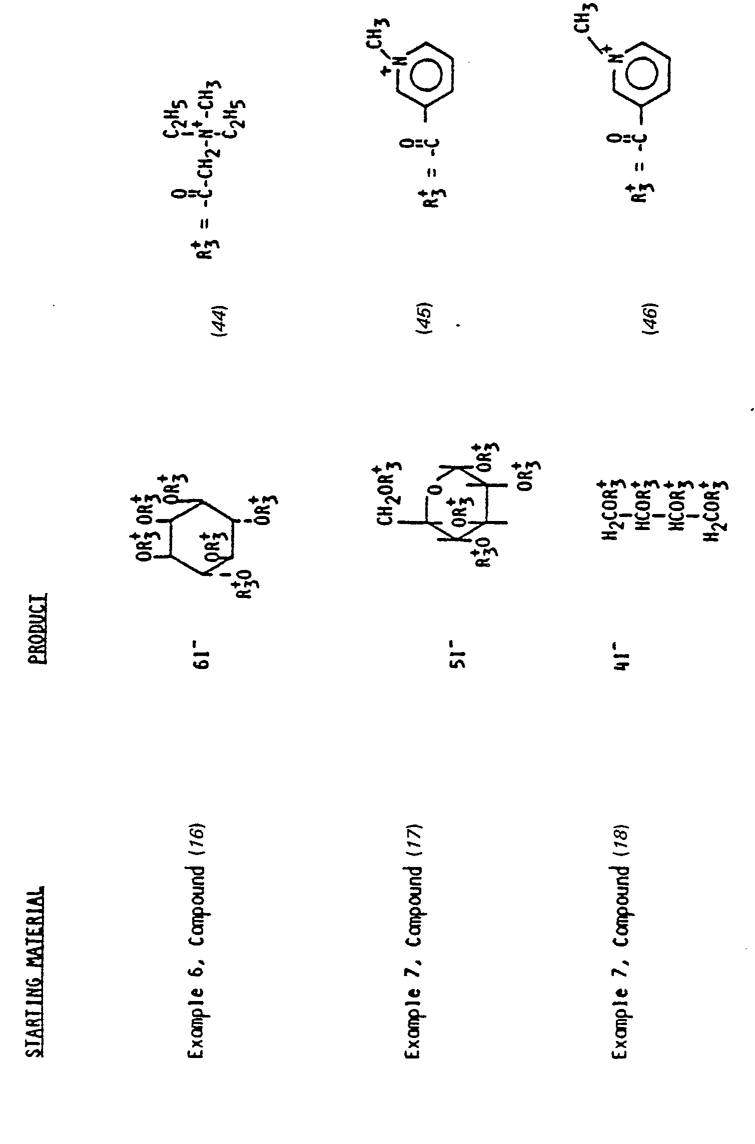

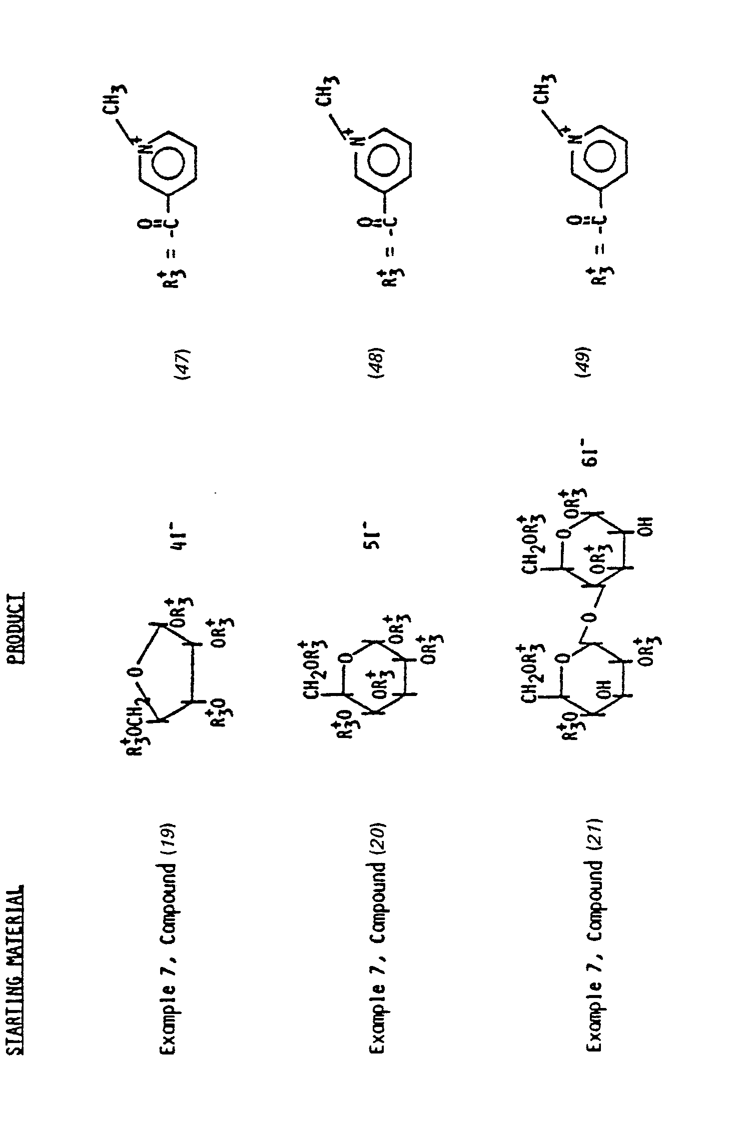

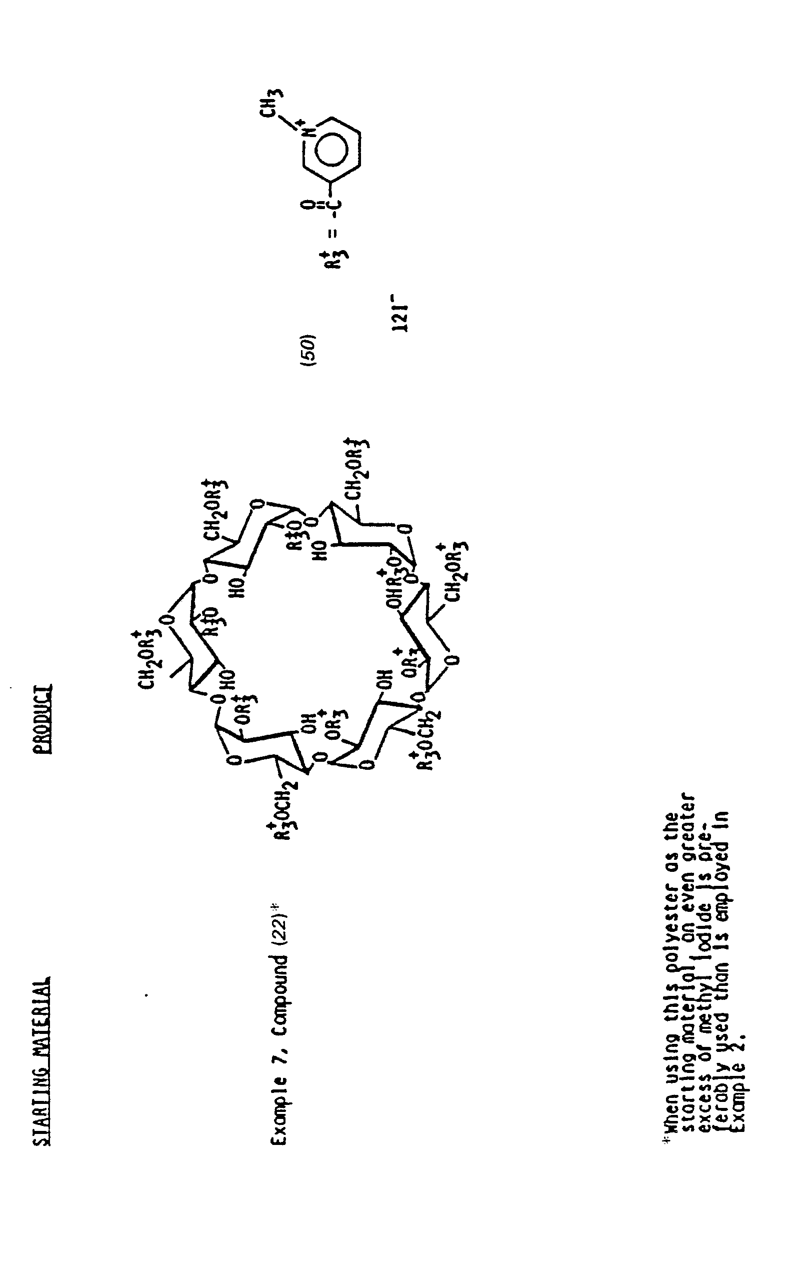

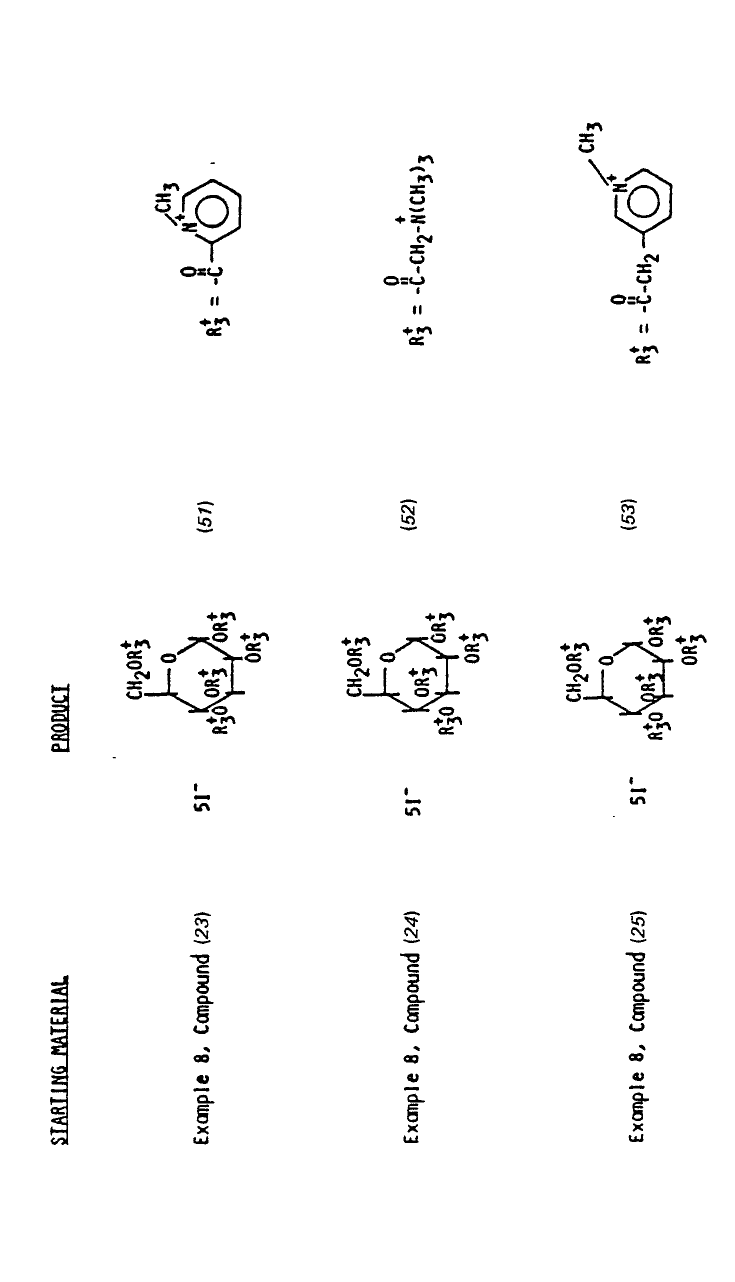

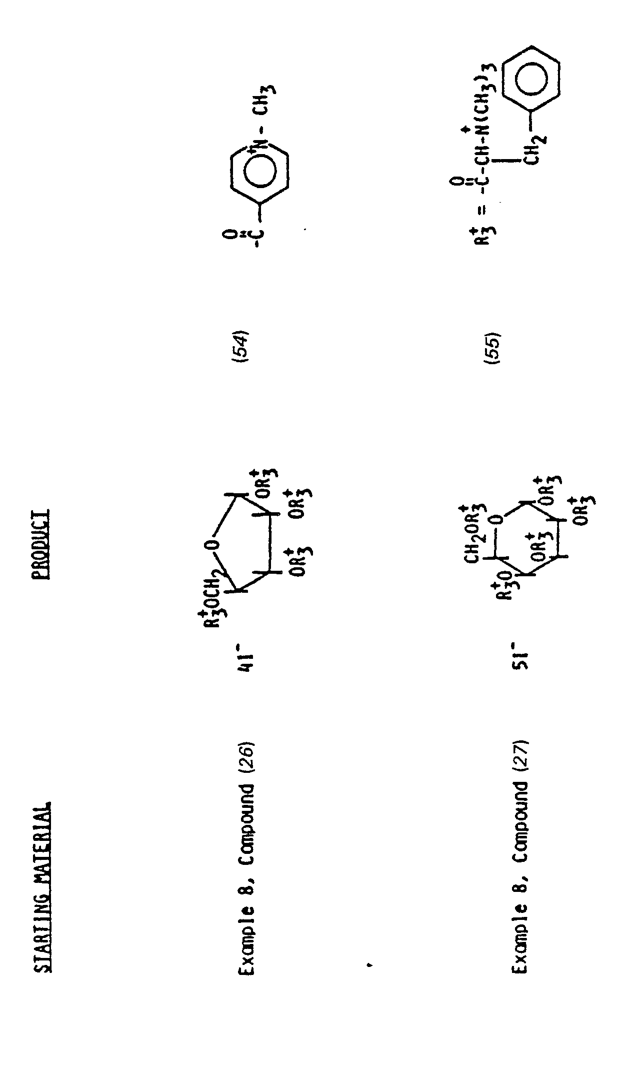

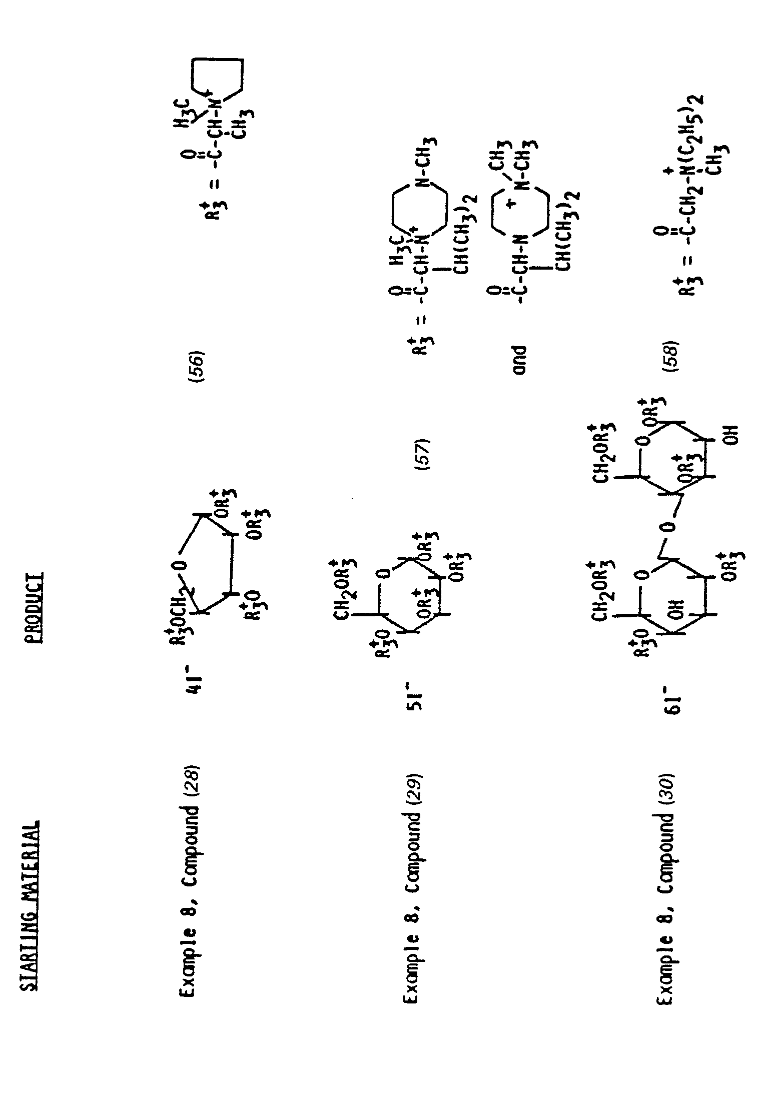

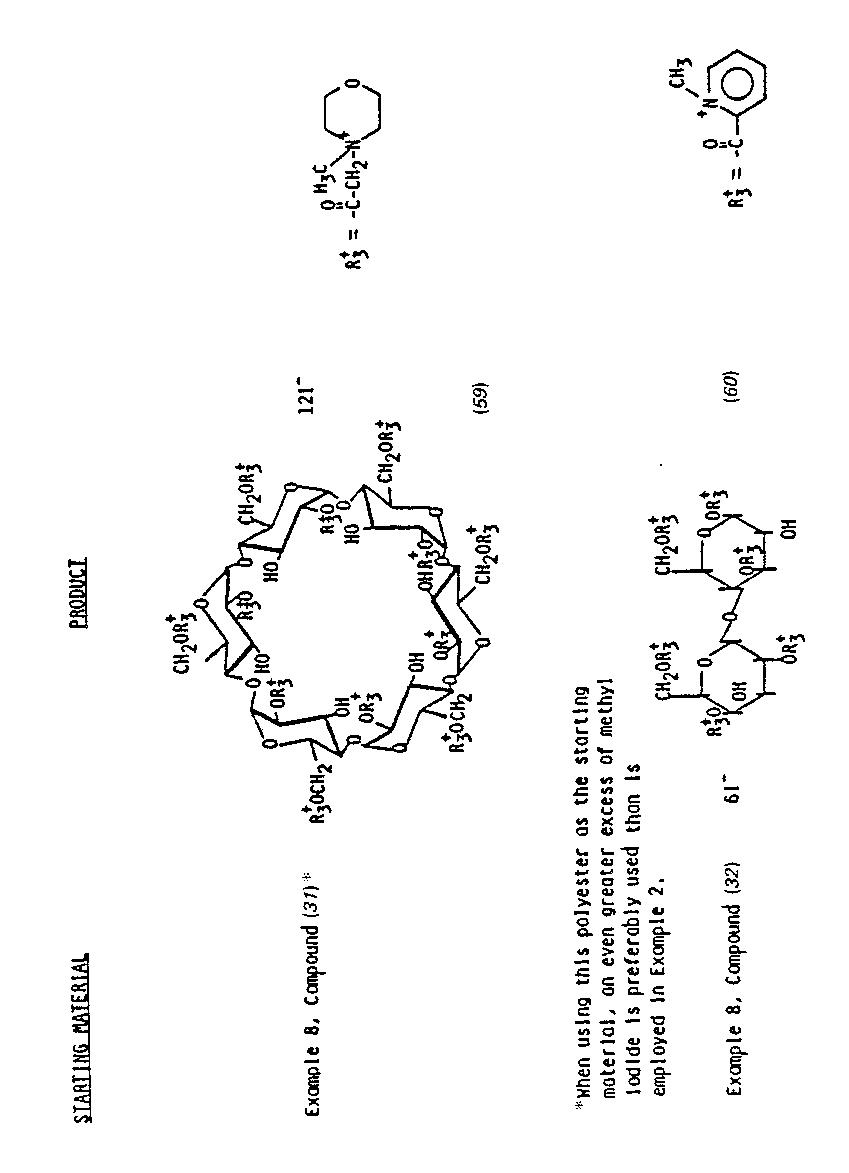

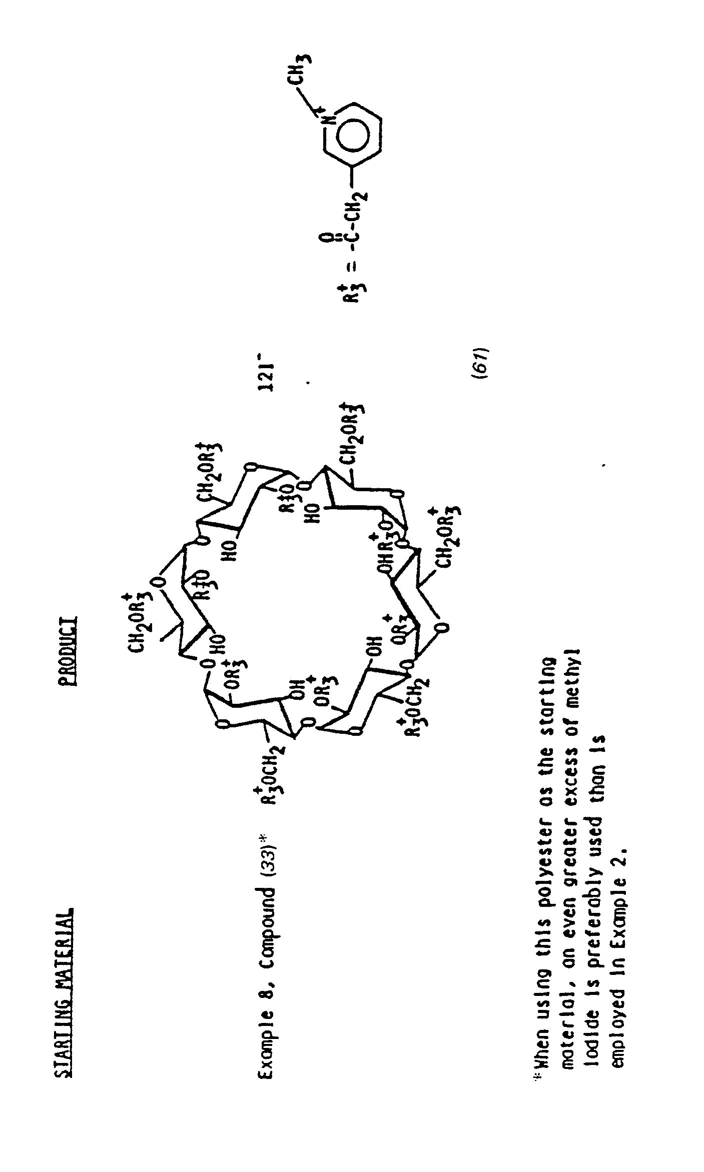

### Example 10

The general procedure of Example 4 is repeated, using an equivalent quantity of each of the quaternary salts prepared in Example 9 in place of the quaternary salt (2) or (3) used in Example 4 and varying reaction conditions appropriately when ion pairs of heparins or heparin⁷⁻ are desired. After suitable isolation, the following heparin multiplets of the invention are obtained:

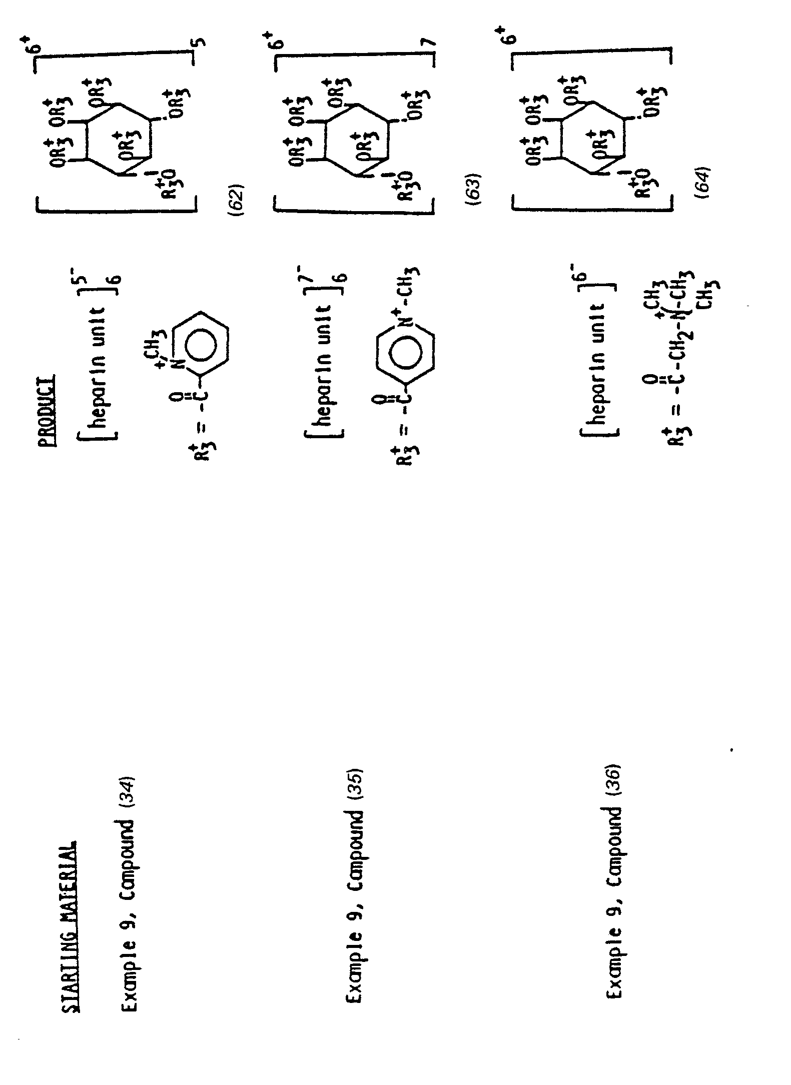

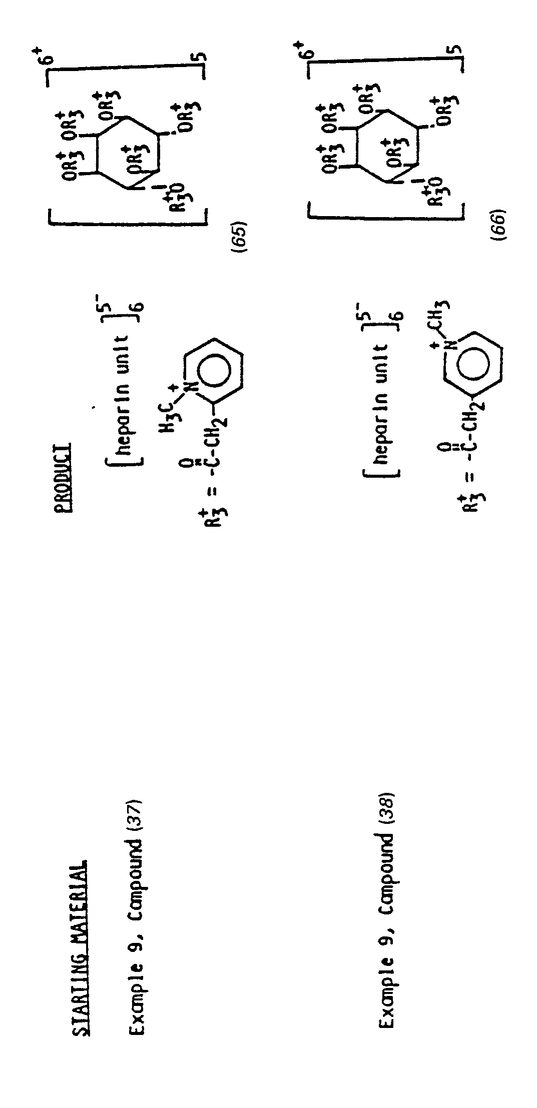

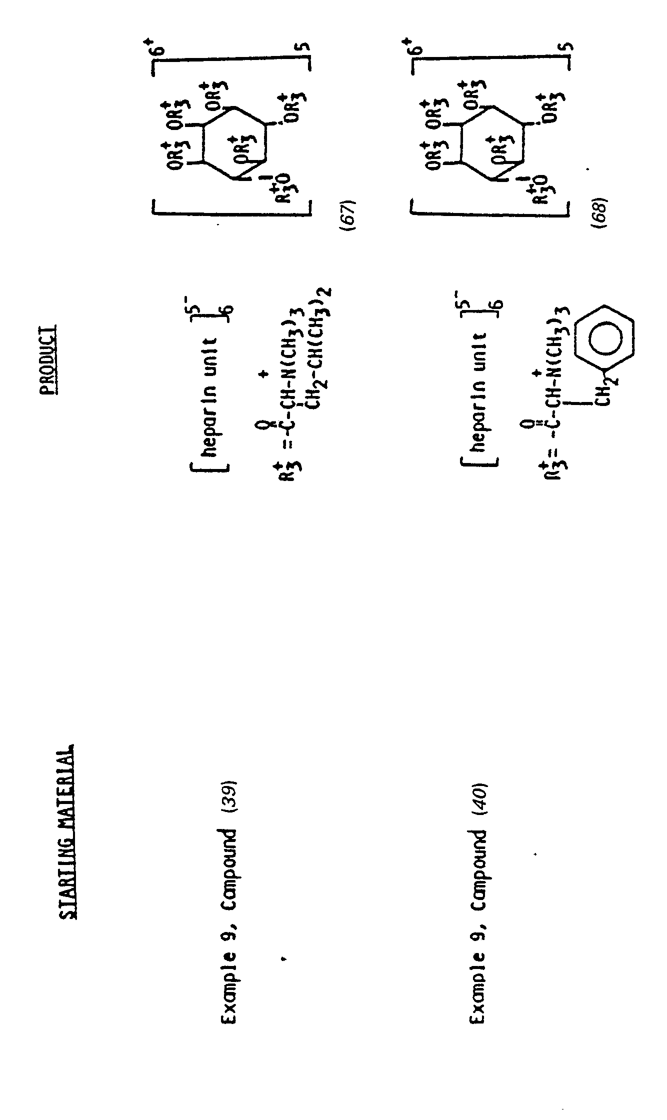

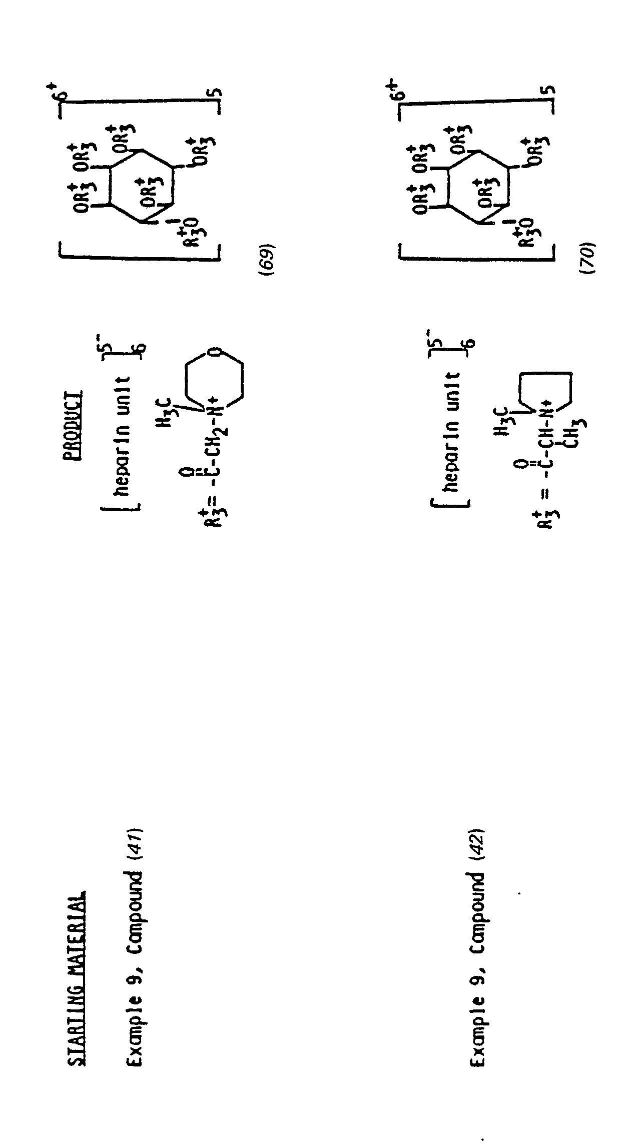

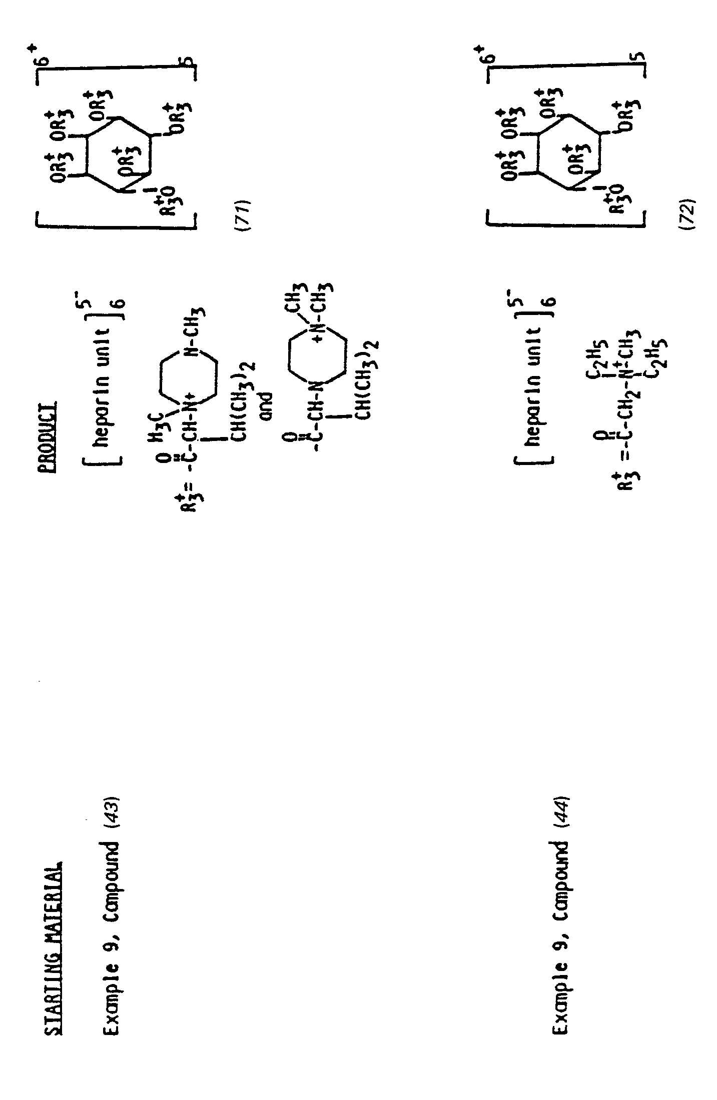

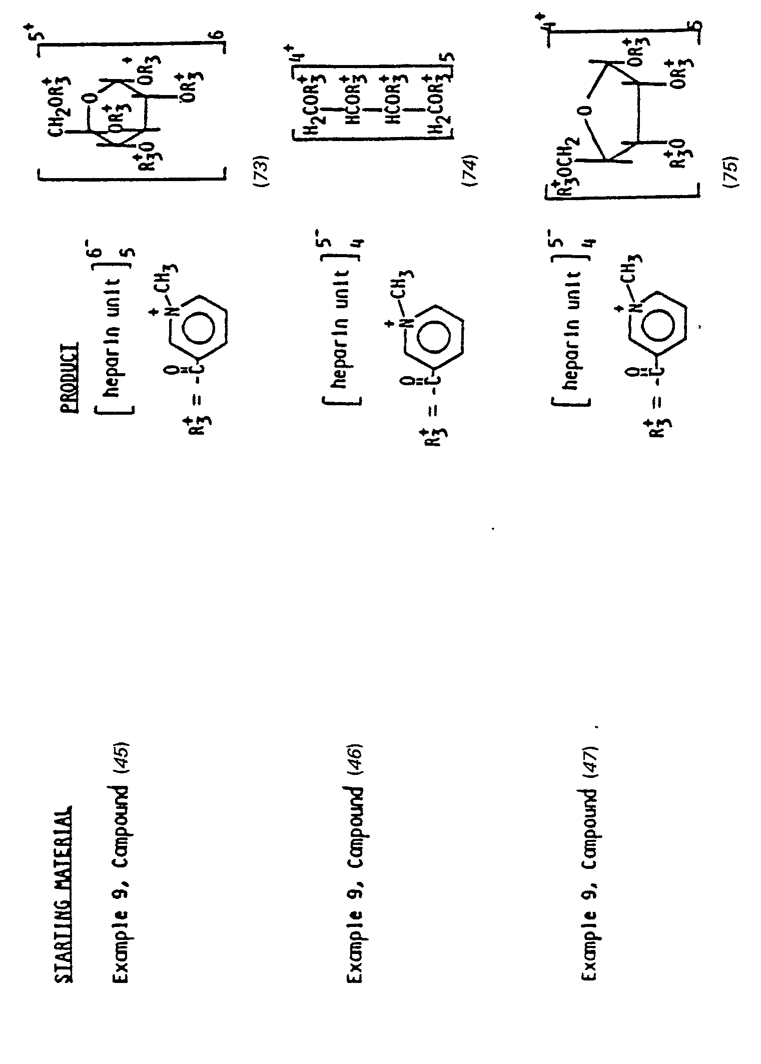

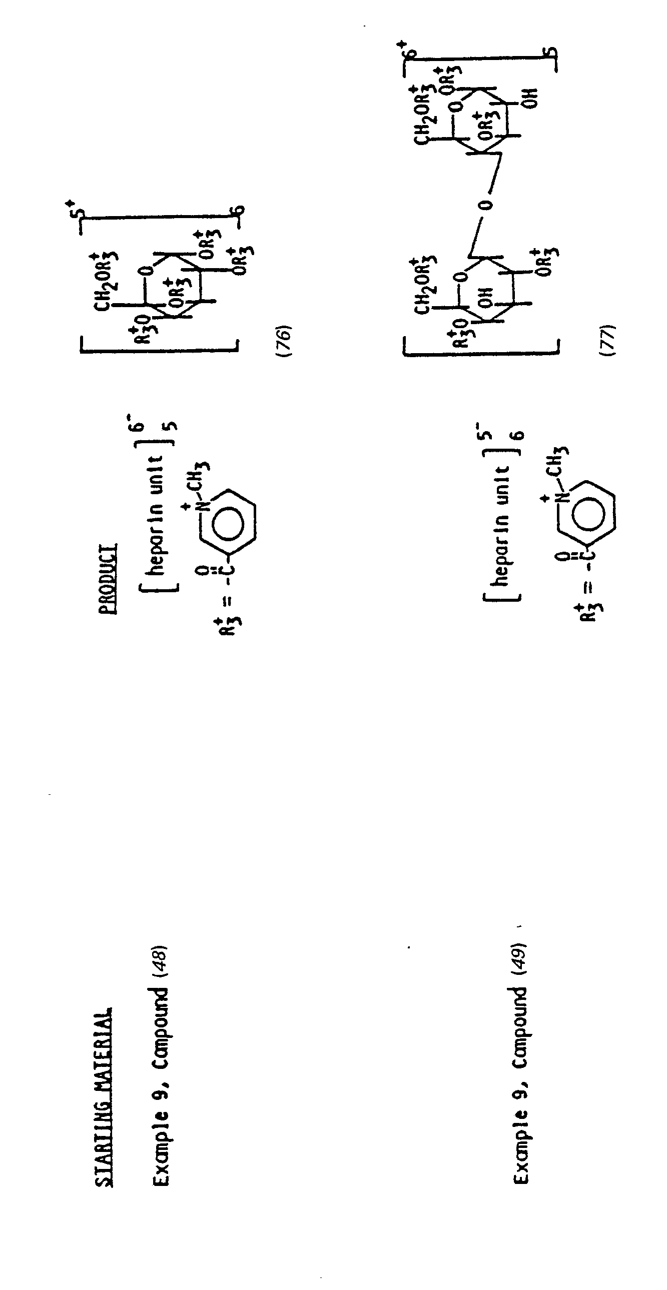

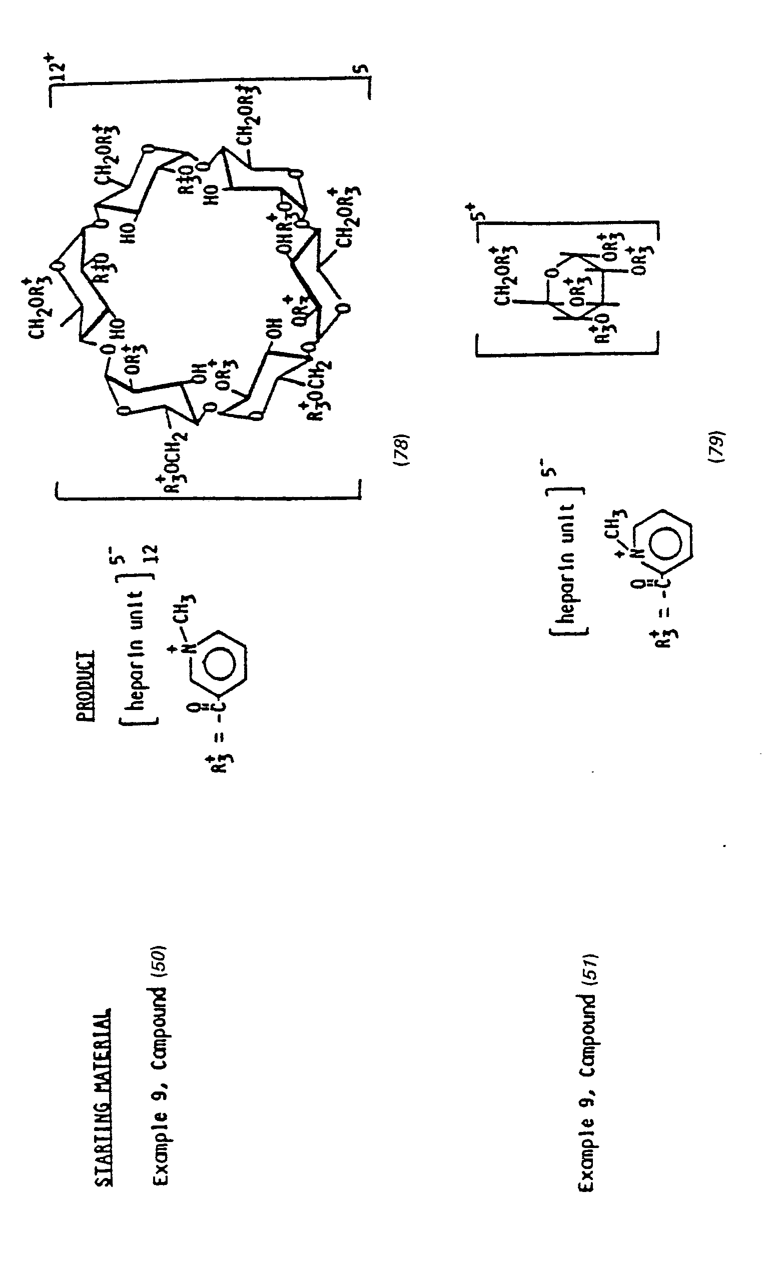

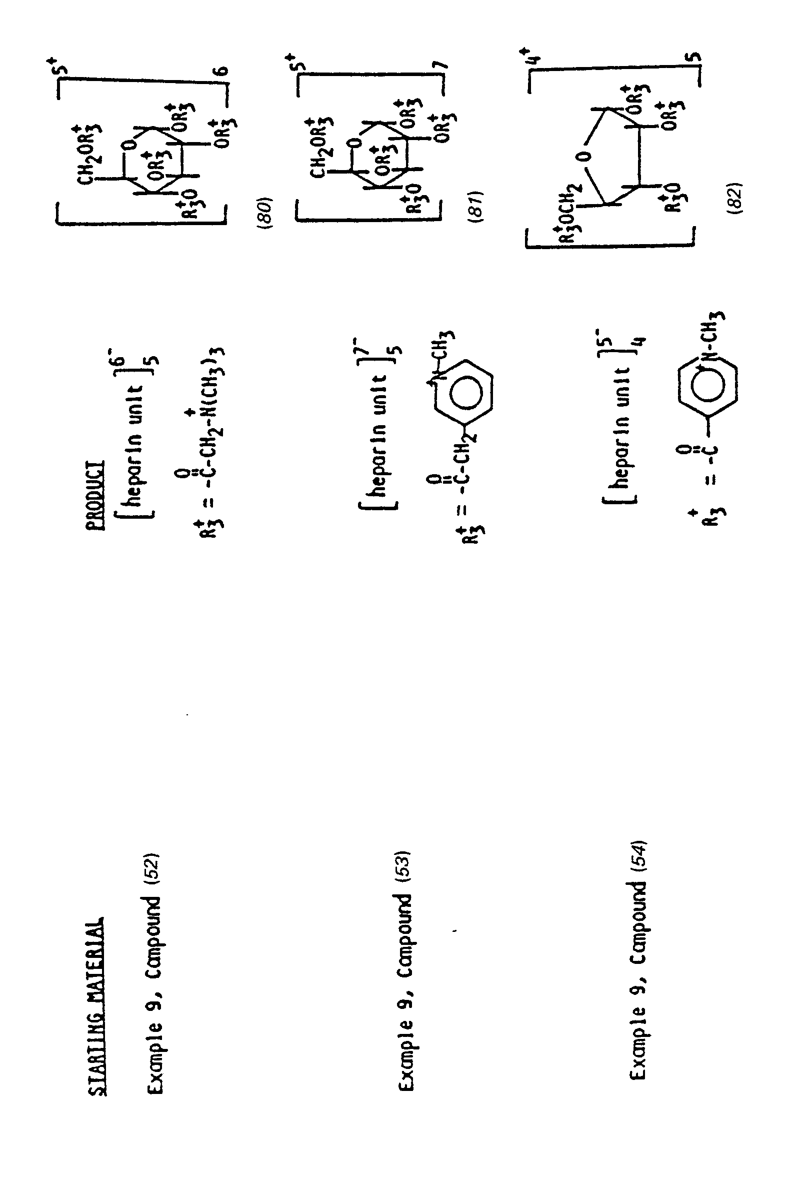

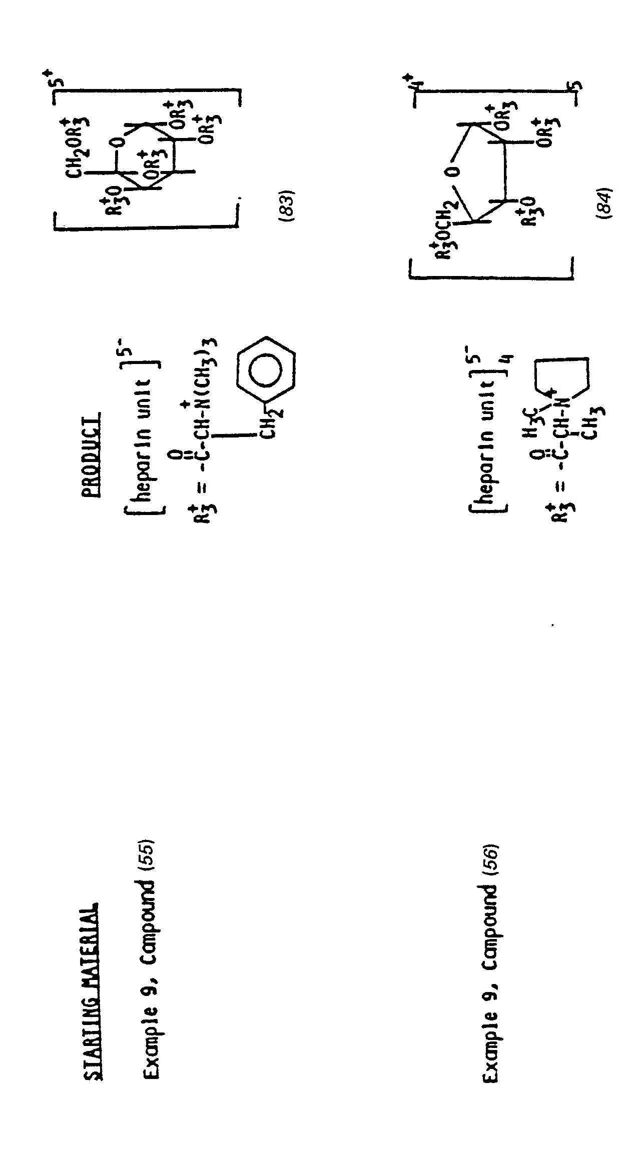

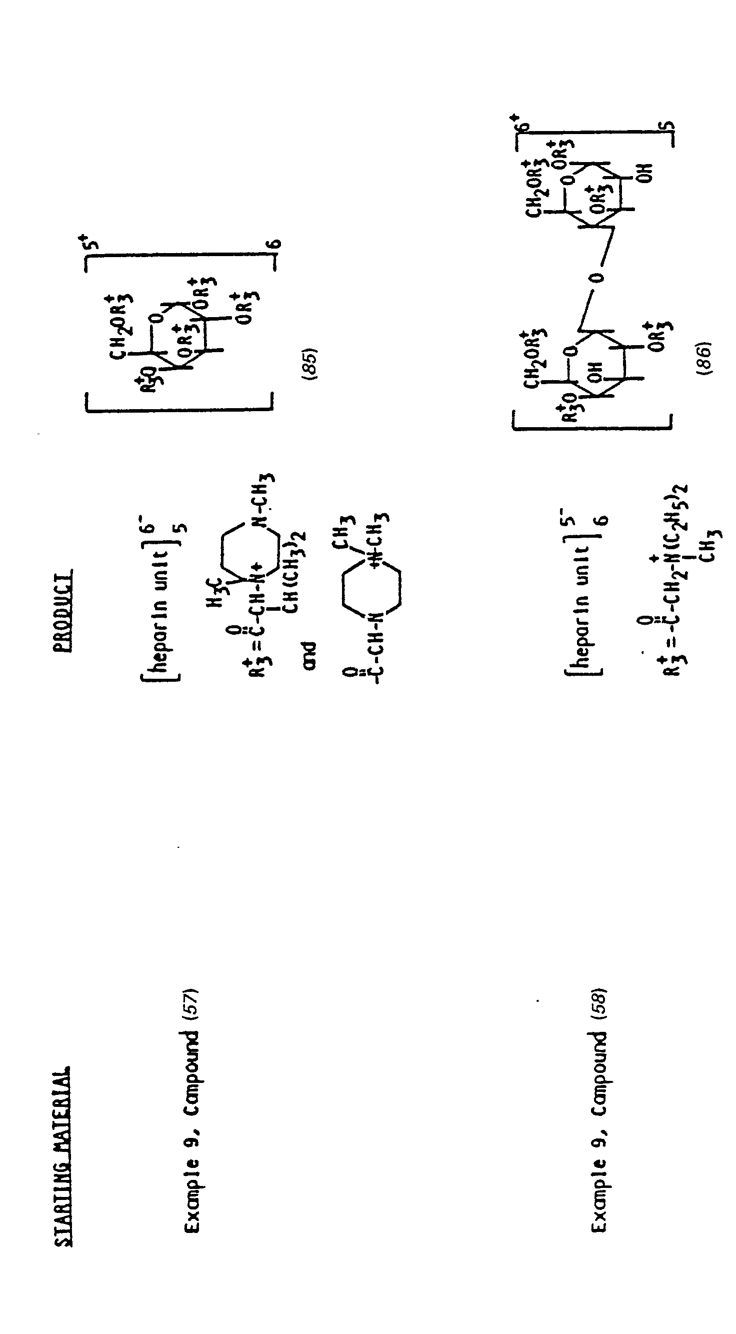

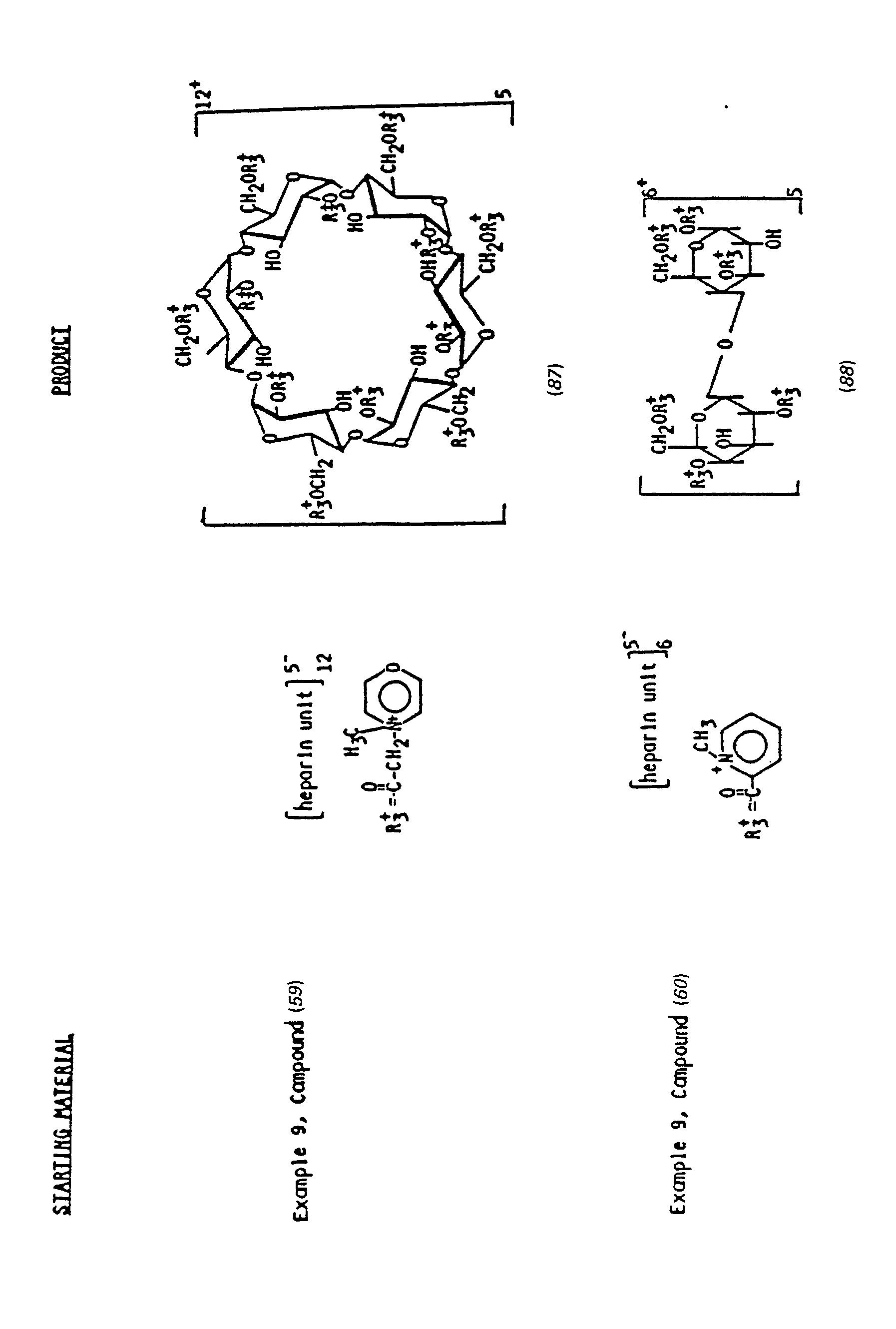

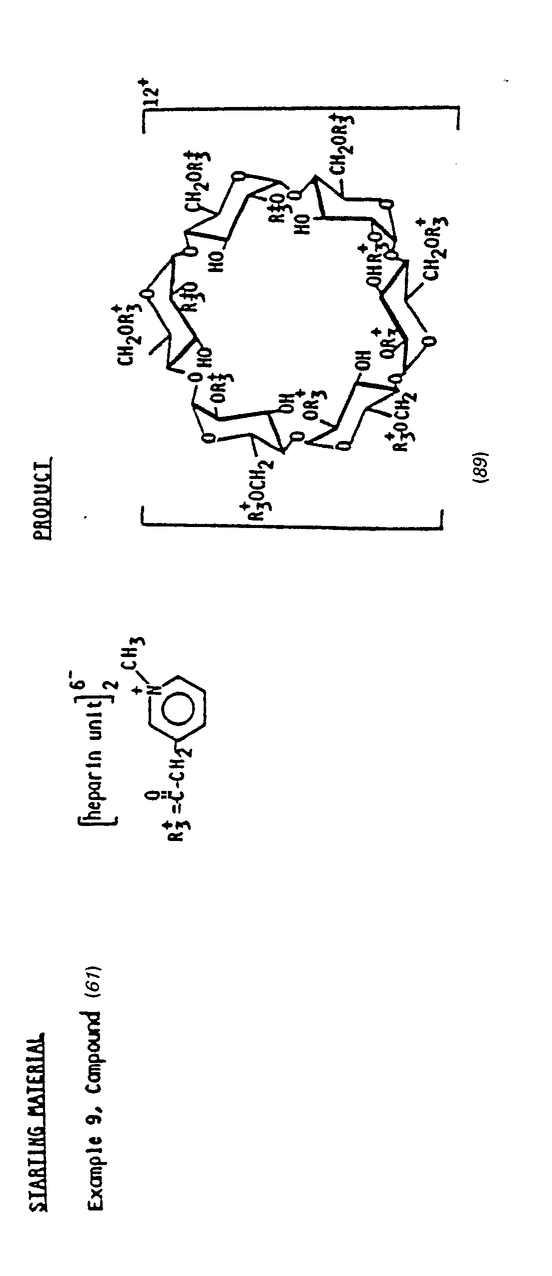

### Example 11

1.0 Mol of erythritol and 5.0 mol of Br-CH₂CH₂COCI are combined in chloroform (25 ml chloroform per 1.0 g erythritol) and 5.0 mol of triethylamine are added. The mixture is heated to the boiling point of chloroform and maintained at that temperature for approximately 4 hours, then washed successively with water, aqueous sodium bicarbonate solution and water and evaporated. The residue, which is the fully bromoacetylated intermediate of the formula 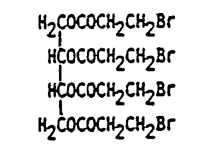 is then dissolved in dimethylformamide (25 ml dimethylformamide per 1.0 g of above intermediate) and excess nicotinamide (8.0 mol per 1.0 mol of above intermediate) is added. The mixture is warmed to 60°-70°C and maintained at that temperature for about 24 hours. Acetonitrile is added to complete precipitation and the precipitate is removed by filtration and dried. The resultant novel intermediate of the formula 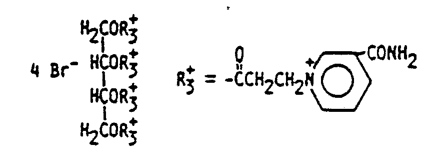 can then be reacted with heparin sodium according to the procedure of Example 4 to afford the following multiplet of the invention:

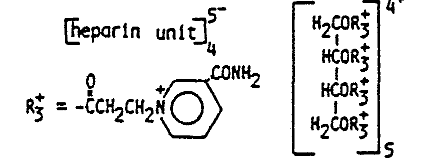

Substantial repetition of the foregoing general procedure utilizing an equivalent quantity of a-D-ribo- furanose in place of the erythritol affords, in the first step, the bromoacetylated intermediate of the formula 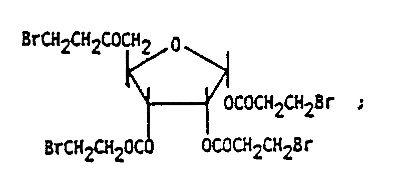 in the second step, the novel quaternary intermediate of the formula 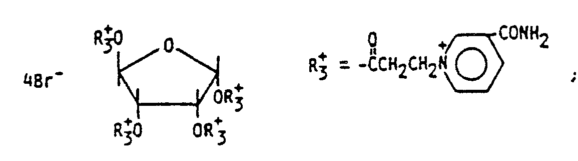 and, in the third step, the following multiplet of the invention:

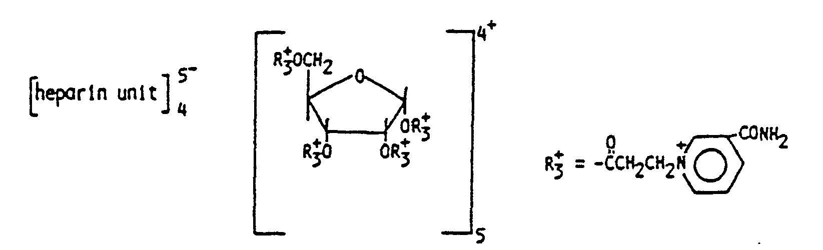

### Example 12

1.0 Mol of inositol, 6.6 mol of pyridinium acetic acid bromide (prepared by reacting pyridine with bromoacetic acid) and 6 mol of dicyclohexylcarbodiimide are combined in pyridine (30 ml per 1.0 g inositol). The mixture is stirred for approximately 4 to 6 hours at room temperature. After appropriate isolation and purification, the following novel quaternary intermediate is obtained:

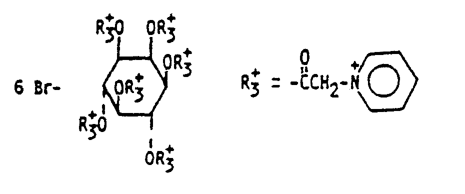

Reaction of that novel intermediate with heparin sodium according to the procedure of Example 4 affords the following multiplet of the invention:

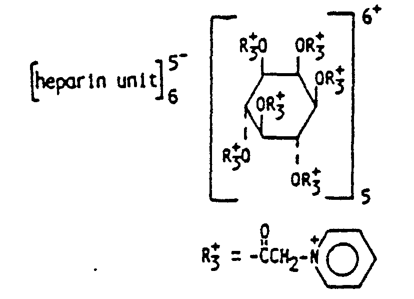

### Example 13

Substitution of equivalent quantities of the starting materials listed below for the inositol and pyridinium acetic acid bromide used in Example 12 and repetition of the general procedure described in the first paragraph of that example affords the indicated products:

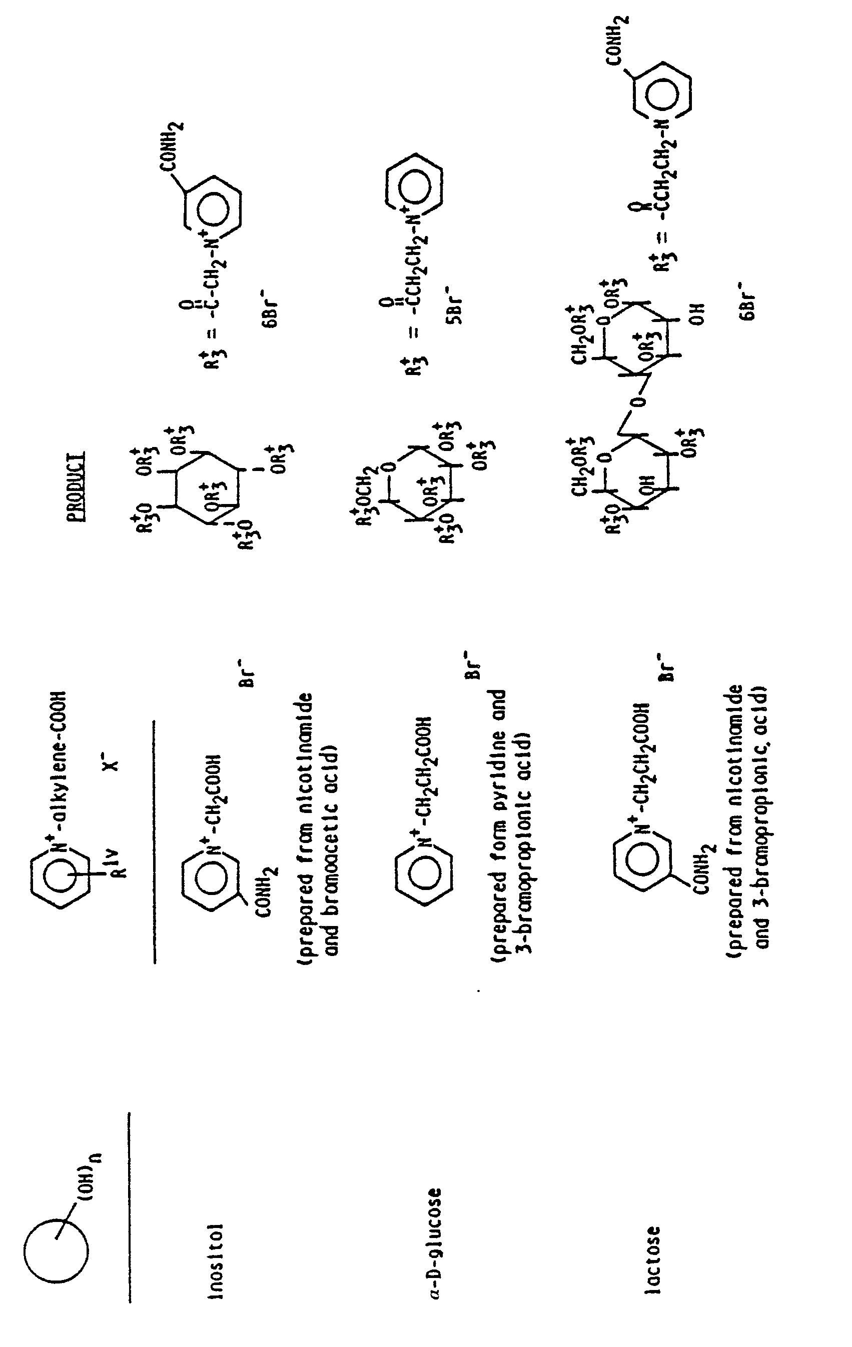

### Example 14

Reaction of each of the products of Example 13 with heparin sodium according to the procedure of Example 4 affords the following multiplets of the invention:

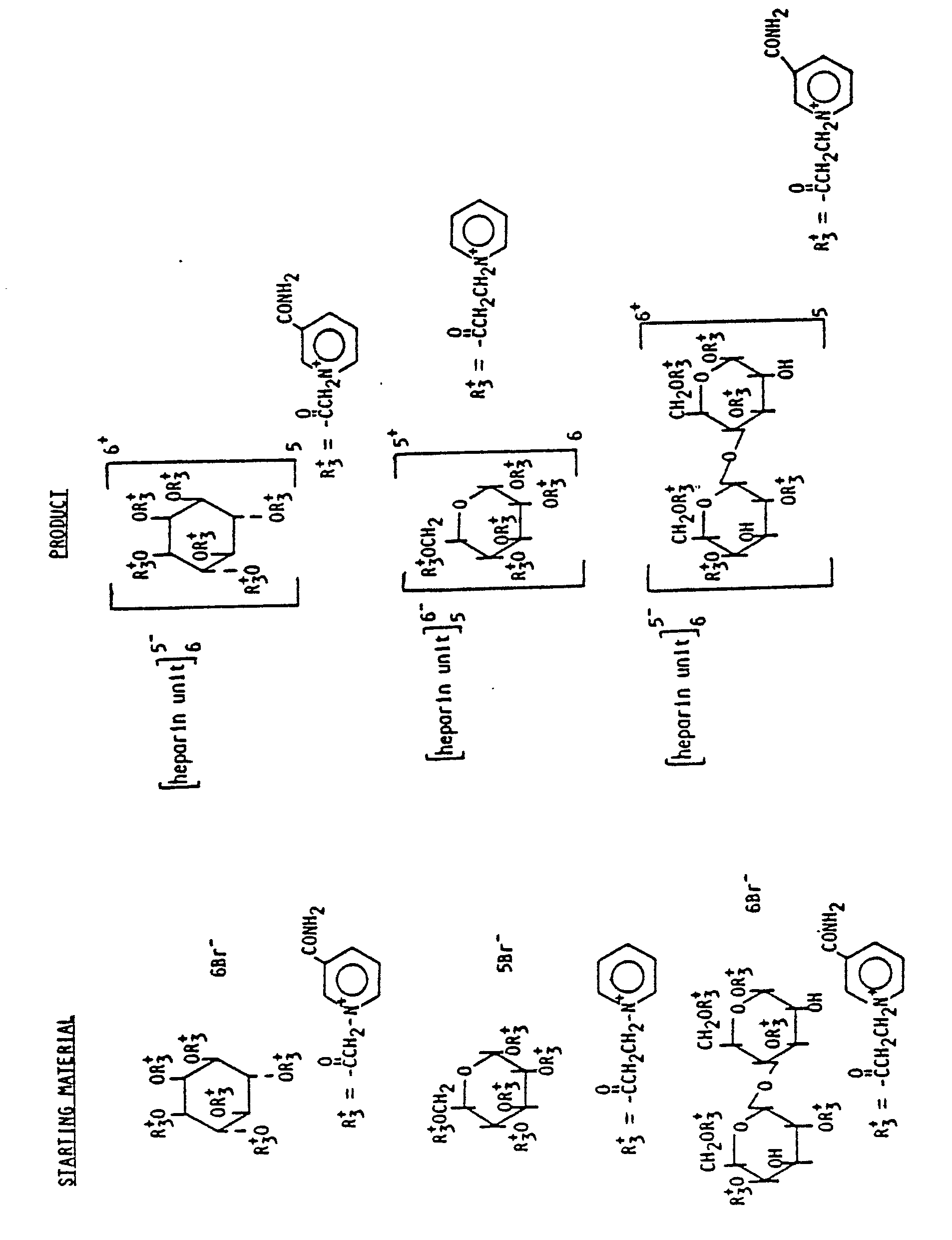

### Example 15

### Stability Studies

A solution of (3) in D₂0 was maintained at room temperature in an NMR sample tube and an NMR run was taken daily. The only observed difference was a broadening of the multiplet at 6 6.6-6.5 corresponding to the protons of the inositol ring which started three days after preparing the solution for study. The broadening increased and the absorption shifted upfield until it lost its characteristic shape (within 12 days).

### Example 16

### Pharmacological Testing

### Determination of Clotting Time

Blood was withdrawn from the treated or control animals, rat or rabbit, by cardiac puncture. Each blood sample was divided into three 1.5 ml polypropylene microcentrifuge tubes. The tubes were capped and the tube containing the first blood withdrawn was inverted once every 30 sec until the blood clotted. At this time, the next tube was inverted in the same way followed by the third tube, which contained the last blood withdrawn. The clotting time taken was the time the blood in the third tube coagulated.

### Testing of Anticoagulant Activity by Oral Administration to Rats

Male Sprague-Dawley rats with an average weight of 450 g were used. The rats were anesthetized with ether and either the test drug or heparin sodium, with a dose of 9000 USP unit/rat, was given orally through a stomach tube as a suspension in 1 ml polyethylene glycol 400. A group of rats were treated in the same way with 1 ml polyethylene glycol 400 only and was used as control. At selected time intervals, a sample of blood was withdrawn by cardiac puncture and the clotting time was determined as described previously.

### Testing of Anticoagulant Activity of Jejunal Administration to Rats and Rabbits

Male Sprague-Dawley rats with an average weight of 450 g and New Zealand white rabbits of average weight 3.0 kg were used for this mode of administration. The animals were fasted for twenty-four hours before the test. The abdomen of the anesthetized animal was entered and the jejunum identified. The finely powdered ion pair compound or heparin sodium, adjusted to contain a dose of 20,000 USP unit/kg, was suspended in polyethylene glycol 400 and injected directly into the jejunum. Blood samples were taken by cardiac puncture at selected time intervals after administration and the clotting time determined as described previously. With rats, no samples could be taken at more than four hours after administration because of the death of rats from internal bleeding.

### Results

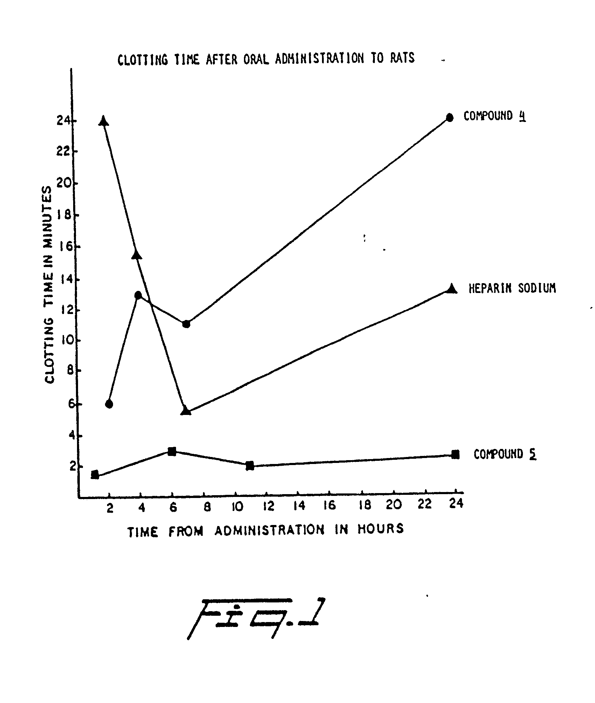

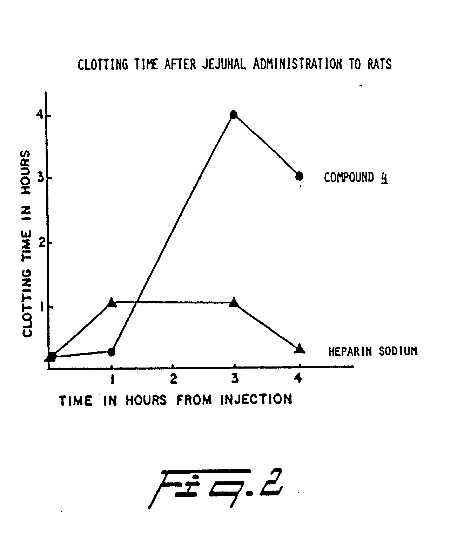

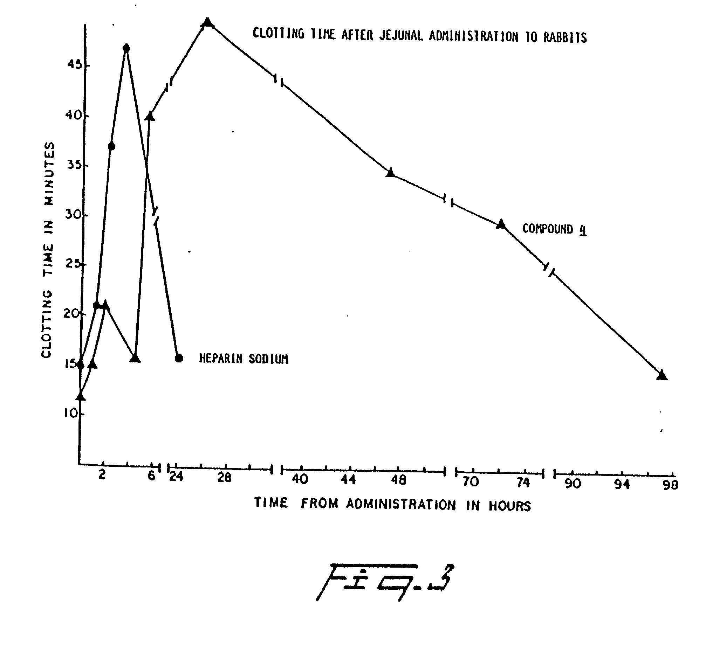

The in vitro anticoagulant activity of the heparin complexes (4) and (5) was tested. Their in vitro activity was found to be comparable to their corresponding content of heparin. Their in vivo anticoagulant activity was tested compared to heparin sodium on rats using oral, rectal and jejunal administration and on rabbits using jejunal administration. The compounds and heparin sodium were each administered as a suspension in polyethylene glycol 400. [Compound (4) changed into a sticky semisolid when it came in contact with water.] Blood samples were taken by heart puncture and the clotting time was determined using the method described above. With rectal administration to rats, no activity was shown, whether for the complexes or for the heparin sodium. With oral administration to rats (Figure 1), compound (5) showed no activity while compound (4), a representative multiplet of this invention, showed higher activity than heparin, almost twice, after twenty-four hours. In the case of jejunal administration to rats (Figure 2), compound (5) showed no activity while compound (4), the representative multiplet of this invention, showed a larger AUC (area under the curve) within the four hours of the experiment. No data could be collected for longer time periods for administration, since the rats died from internal hemorrhage due to the operation. With jejunal administration to rabbits (Figure 3), it is shown that, although compound (4) has slower onset of action than heparin sodium, it is more active and has a much longer duration of action. The clotting time does not return back to the normal value except after about 90 hours from administration. One rabbit was sacrificed and dissected after 48 hours from administration. The internal organs looked normal, except the internal wall of the jejunum which was colored yellow; this indicated still the existence of the drug within the villi.

## Claims (Claims for the following Contracting State(s) : s: BE CH DE FR GB IT LI LU NL SE)

1. A multiplet of the structural formula 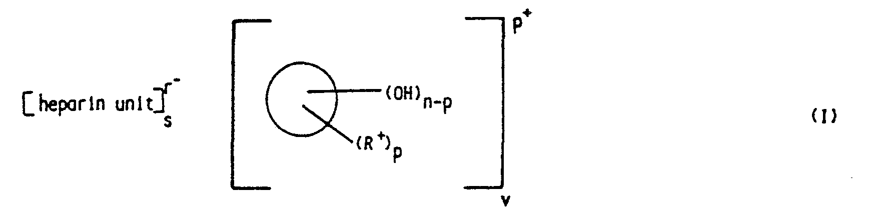 wherein O is the skeleton of a polyol, said skeleton being the portion of said polyol remaining after removal of all hydroxy substituents therefrom; n is a number from 3 to 24 which represents the total number of hydroxy groups in said polyol; p is a number ≥ 3 and ≤ n; r is the available valence of the heparin unit and is ≥ 3 and ≤ 7; s is the number which when multiplied by r is equal to pv; v is the number which when multiplied by p is equal to rs; R⁺ is 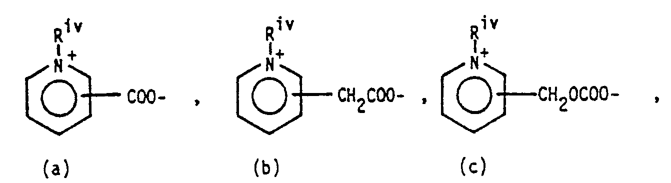 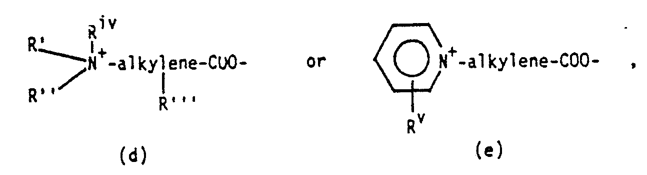 wherein the -COO-, -CH₂COO-, -CH₂0COO- and R" ring substituents can each be in the 2-, 3- or 4- position of the pyridinium ring; R^{iv} is C₁―C₃ alkyl; R' and R", which can be the same or different, are each C₁-C₇ alkyl, or R' and R" are combined with the adjacent nitrogen atom such that 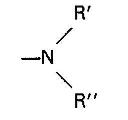 represents the residue of a saturated monocyclic secondary amine; R"' is 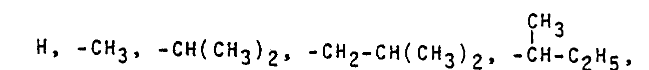 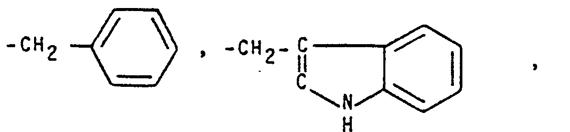 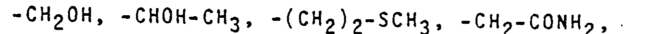 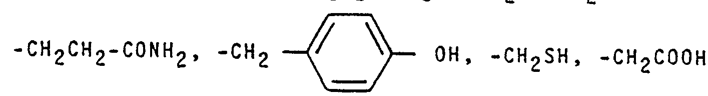 or 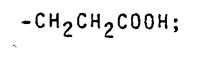 the alkylene groups can be straight or branched and contain 1 to 3 carbon atoms; and R" is H, -CONH₂ or ―COC(C₁―C₇ alkyl).

2. A multiplet as claimed in Claim 1, wherein the polyol is amonosaccharide or an oligosaccharide.

3. A multiplet as claimed in Claim 2, wherein the monosaccharide is a pentose, hexose or heptose, or wherein the oligosaccharide is a disaccharide, a trisaccharide or a cyclodextrin.

4. A multiplet as claimed in Claim 3, wherein the polyol is ribose, arabinose, xylose, lyxose, ribulose, xylulose, glucose, galactose, mannose, fructose, sorbose, tagatose, mannoheptulose, sedoheptulose, sucrose, lactose, maltose, raffinose, a-cyclodextrin, β-cyclodextrin or y-cyclodextrin.

5. A multiplet as claimed in Claim 1, wherein the polyol is a C₃―C₁₅ aliphatic polyhydroxy compound or a C₅―C₁₈ alicyclic polyhydroxy compound.

6. A multiplet as claimed in Claim 5, wherein the aliphatic polyhydroxy compound is a C₃-Cₛ alkyl polyol, or wherein the alicyclic polyhydroxy compound is a fused fully hydrogenated aromatic polyol.

7. A multiplet as claimed in Claim 6, wherein the C₃―C₈ alkyl polyol is glycerol, erythritol or 1,2,6-trihydroxyhexane, or wherein the fused fully hydrogenated aromatic polyol is a decahydronaphthalene polyol.

8. A multiplet as claimed in Claim 5, wherein the alicyclic polyhydroxy compound is a C₅―C₁₀ cycloalkyl polyol.

9. A multiplet as claimed in Claim 8, wherein the C₅―C₁₀ cycloalkyl polyol is a cyclohexane polyol.

10. A multiplet as claimed in Claim 9 wherein the cyclohexane polyol is inositol.

11. A multiplet as claimed in Claim 1, wherein R' and R" are combined with the adjacent nitrogen atom such that 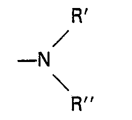 represents the residue of a saturated monocyclic secondary amine having 5 to 7 ring atoms, optionally containing another hetero ring atom ―O―, ―S―, or ―N― in addition to the indicated nitrogen atom, and optionally bearing 1 to 3 methyl substituents.

12. A multiplet as claimed in Claim 11, wherein 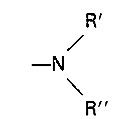 is morpholino, 1-pyrrolidinyl, perhydro-1,2,4-oxathiazin-4-yl, 1- or 4-piperazinyl, 4-methyl-I-piperazinyl, piperidino, hexamethyleneimino, 2-methyl-1-pyrazolidinyl, 1- or 2-pyrazolidinyl, 3-methyl-1-imidazolidinyl or 1- or 3-imidazolidinyl.

13. A multiplet as claimed in Claim 1, wherein:
(i) R⁺ is structure (a), (b) or (c); R^{iv} is methyl; and the ―COO―, ―CH₂COO― or ―CH₂OCOO―group is located in the 3-position; or
(ii) R² is structure (d); R' is methyl or ethyl and R" is identical to R', or R'R"N- represents morpholino, piperidino, 1-pyrrolidinyl or 1-piperazinyl; R^{iv} is methyl; and alkylene is 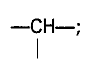 or
(iii) R⁺ is structure (e), alkylene is -CH₂CH₂- and R" is H or -CONH₂.

14. A multiplet as claimed in Claim 13 (ii), wherein R"' is 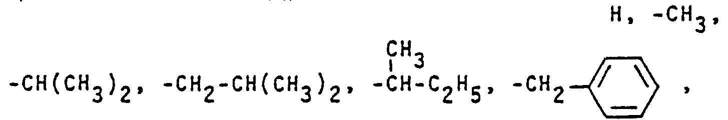 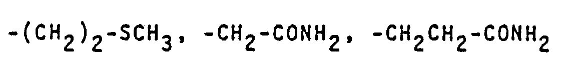 or

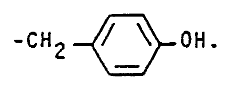

15. A multiplet as claimed in Claim 1, wherein p is equal to n.

16. A multiplet as claimed in Claim 15, wherein p is at least 4.

17. A multiplet as claimed in Claim 1, wherein r is 5, 6 or 7.

18. A multiplet as claimed in Claim 1, wherein O is the skeleton of inositol, n is 6 and p is 6.

19. A multiplet as claimed in Claim 18, wherein v is 5, r is 5 and s is 6.

20. A multiplet as claimed in Claim 1, said multiplet having the structural formula 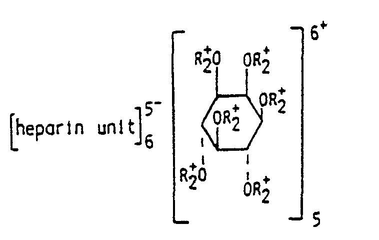 wherein R2 is

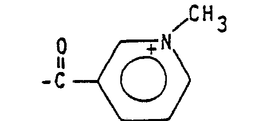

21. A pharmaceutical composition of matter comprising a multiplet of formula (I) as defined by Claim 1 and a non-toxic pharmaceutically acceptable carrier therefor.

22. A pharmaceutical composition of matter, in unit dosage form, for use in eliciting an antiocoagulant response in a warm-blooded animal, said composition comprising, per dosage unit, an effective unit anticoagulant amount of a multiplet of formula (I) as defined by Claim 1 and a non-toxic pharmaceutically acceptable carrier therefor, said composition being suitable for nasal administration, for oral inhalation therapy or for other oral administration.

23. A salt of the structural formula 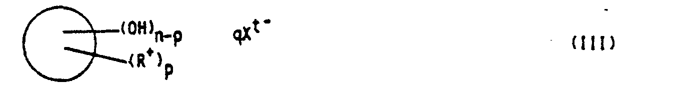 wherein O is the skeleton of a polyol, said skeleton being the portion of said polyol remaining after removal of all hydroxy substituents therefrom; n is a number from 3 to 24 which represents the total number of hydroxy groups in said polyol; p is a number ≥ 3 and ≤ n; R⁺ is 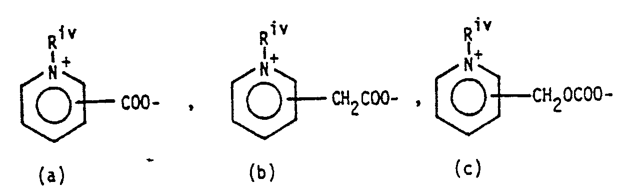 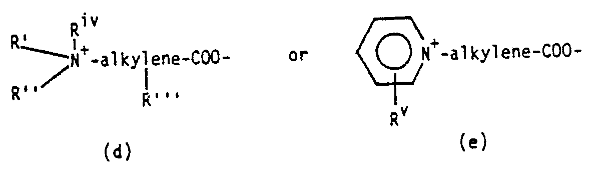 wherein the -COO-, -CH₂COO-, -CH₂0COO- and R" ring substituents can each be in the 2-, 3- or 4- position of the pyridinium ring; R^{iv} is C₁-C₃ alkyl; R' and R", which can be the same or different, are each C₁-C₇ alkyl, or R' and R" are combined with the adjacent nitrogen atom such that 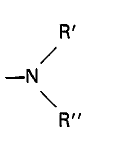 represents the residue of a saturated monocyclic secondary amine; R"' is 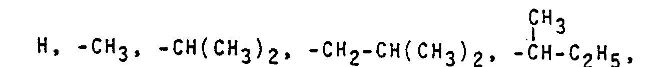 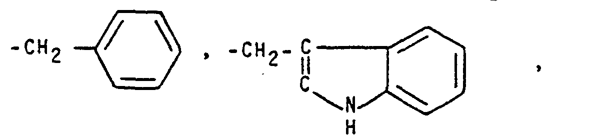 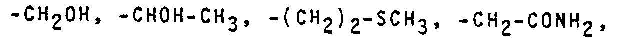 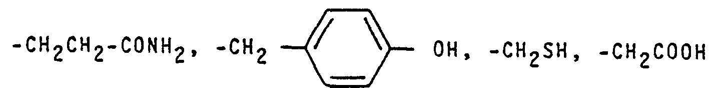 or 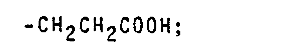 the alkylene groups can be straight or branched and contain 1 to 3 carbon atoms; R" is H, -CONH₂ or ―COO(C₁―C₇); X- is the anion of a pharmaceutically acceptable organic or inorganic acid; t is the valence of the acid anion; and 1 is the number which when multiplied by t is equal to p.

24. A salt as claimed in Claim 23, wherein the polyol is a monosaccharide or an oligosaccharide.

25. A salt as claimed in Claim 24, wherein the monosaccharide is a pentose, hexose or heptose, or wherein the oligosaccharide is a disaccharide, a trisaccharide or a cyclodextrin.

26. A salt as claimed in Claim 23, wherein the polyol is a C₃―C₁₅ aliphatic polyhydroxy compound or a C₅―C₁₈ alicyclic polyhydroxy compound.

27. A salt as claimed in Claim 26, wherein the aliphatic polyhydroxy compound is a C₃―C₈ alkyl polyol, or wherein the alicyclic polyhydroxy compound is a C₅―C₁₀ cycloalkyl polyol or a fused fully hydrogenated aromatic polyol.

28. A salt as claimed in Claim 27, wherein the C₅―C₁₀ cycloalkyl polyol is a cyclohexane polyol.

29. A salt as claimed in Claim 28, wherein the cyclohexane polyol is inositol.

30. A salt as claimed in Claim 23, wherein R' and R" are comined with the adjacent nitrogen atom such that 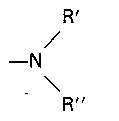 represents the residue of a saturated monocyclic secondary amine having 5 to 7 ring atoms, optionally containing another hetero ring atom -0-, -S- or-N- in addition to the indicated nitrogen atom, and optionally bearing 1 to 3 methyl substituents.

31. A salt as claimed in Claim 23, wherein:
(i) R⁺ is structure (a), (b) or (c); R^{iv} is methyl; and the ―COO―, ―CH₂COO― or ―CH₂OCOO― group is located in the 3-position; or
(ii) R⁺ is structure (d); R' is methyl or ethyl and R" is identical to R', or R'R"N- represents morpholino, piperidino, 1-pyrrolidinyl or 1-piperazinyl; R^{iv} is methyl; and alkylene is 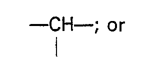
(iii) R⁺ is structure (e), alkylene is ―CH₂CH₂― and R^{v} is H or -CONH₂.

32. A salt as claimed in Claim 23, wherein p is equal to n, or wherein t is 1, or wherein X- is I-, Br⁻ or CI-.

33. A salt as claimed in Claim 23, wherein O is the skeleton of inositol, n is 6 and p is 6.

34. A salt as claimed in Claim 33, having the structural formula 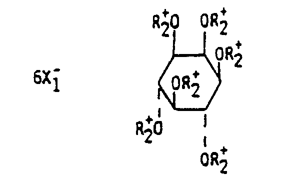 wherein X-₁ is I⁻, Br⁻ or Cl⁻ and R2 is

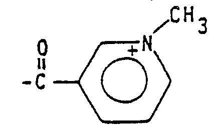

## Claims (Claims for the following Contracting State(s) : AT)

1. A process for the preparation of a multiplet of the structural formula 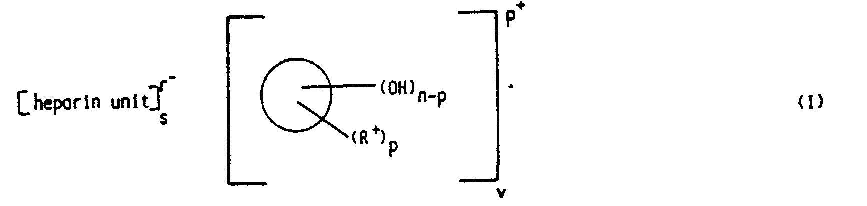 wherein O is the skeleton of a polyol, said skeleton being the portion of said polyol remaining after removal of all hydroxy substituents therefrom; n is a number from 3 to 24 which represents the total number of hydroxy groups in said polyol; p is a number ≥ 3 and ≤ n; r is the available valence of the heparin unit and is ≥ 3 and ≤ 7; s is the number which when multiplied by r is equal to pv; v is the number which when multiplied by p is equal to rs; R⁺ is 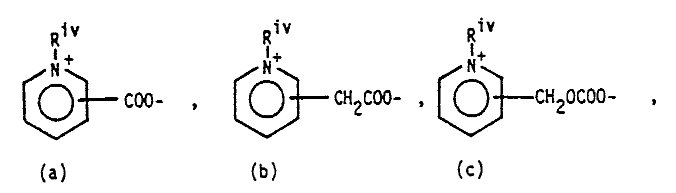 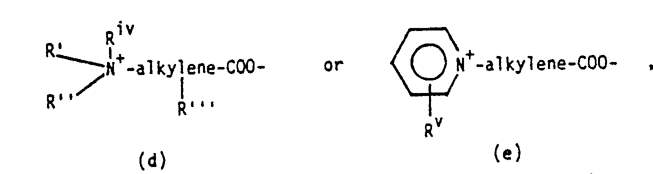 wherein the -COO-, -CH₂COO-, -CH₂0COO- and R" ring substituents can each be in the 2-, 3- or 4- position of the pyridinium ring; R^{iv} is C₁―C₃ alkyl; R' and R", which can be the same or different, are each C₁―C₇ alkyl, or R' and R" are combined with the adjacent nitrogen atom such that 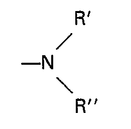 represents the residue of a saturated monocyclic secondary amine; R"' is 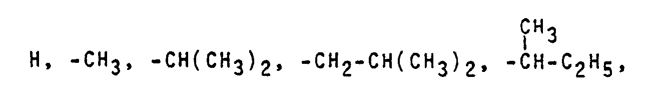 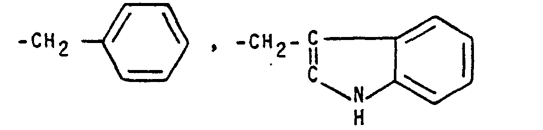 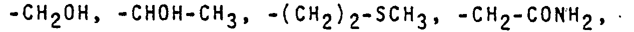 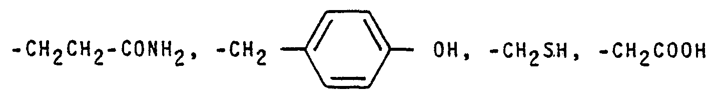 or 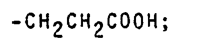 the alkylene groups can be straight or branched and contain 1 to 3 carbon atoms; and R" is H, -CONH₂ or ―COO(C₁―C₇ alkyl); said process comprising:
(a) reacting the corresponding salt of the structural formula 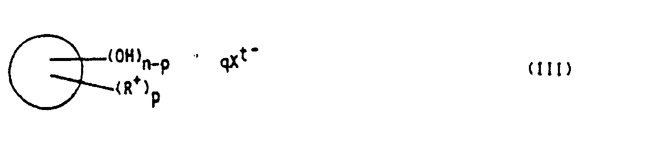 wherein 0, n, p and R⁺ are as defined with formula (I) above, X- is the anion of a pharmaceutically acceptable organic or inorganic acid, t is the valence of the acid anion, and q is the number which when multiplied by t is equal to p, with sodium heparin in aqueous medium; or
(b) subjecting the corresponding salt of formula (III) above to anion exchange to replace the X- anions in the salt with OH- anions, followed by reacting the resultant intermediate with heparinic acid.

2. A process according to Claim 1, wherein the polyol skeleton is that of a monosaccharide or of an oligosaccharide.

3. A process according to Claim 2, wherein the monosaccharide is a pentose, hexose or heptose, or wherein the oligosaccharide is a disaccharide, a trisaccharide or a cyclodextrin.

4. A process according to Claim 1, wherein the polyol skeleton is that of ribose, arabinose, xylose, lyxose, ribulose, xylulose, glucose, galactose, mannose, fructose, sorbose, tagatose, mannoheptulose, sedoheptulose, sucrose, lactose, maltose, raffinose, a-cyclodextrin, β-cyclodextrin or y-cyclodextrin.

5. A process according to Claim 1, wherein the polyol skeleton is that of a C₃―C₁₅ aliphatic polyhydroxy compound or a C₅―C₁₈ alicyclic polyhydroxy compound.

6. A process according to Claim 5, wherein the aliphatic polyhydroxy compound is a C₃―C₈ alkyl polyol, or wherein the alicyclic polyhydroxy compound is a fused fully hydrogenated aromatic polyol.

7. A process according to Claim 6, wherein the C₃―C₈ alkyl polyol is glycerol, erythritol or 1,2,6-trihydroxyhexane, or wherein the fused fully hydrogenated aromatic polyol is a decahydronaphthalene polyol.

8. A process according to Claim 5, wherein the alicyclic polyhydroxy compound is a C₅―C₁₀ cycloalkyl polyol.

9. A process according to Claim 8, wherein the C₅―C₁₀ cycloalkyl polyol is a cyclohexane polyol.

10. A process according to Claim 9, wherein the cyclohexane polyol is inositol.

11. A process according to Claim 1, wherein R' and R" are combined with the adjacent nitrogen atom such that 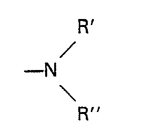 represents the residue of a saturated monocyclic secondary amine having 5 to 7 ring atoms, optionally containing another hetero ring atom ―O―, ―S―, or ―N― in addition to the indicated nitrogen atom, and optionally bearing 1 to 3 methyl substituents.

12. A process according to Claim 11, wherein 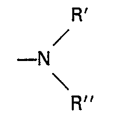 is morpholino, 1-pyrrolidinyl, perhydro-1,2,4-oxathiazin-4-yl, 1- or 4-piperazinyl, 4-methyl-1-piperazinyl, piperidino, hexamethyleneimino, 2-methyl-1-pyrazolidinyl, 1- or 2-pyrazolidinyl, 3-methyl-1-imidazolidinyl or 1- or 3-imidazolidinyl.

13. A process according to Claim 1, wherein:
(i) R⁺ is structure (a), (b) or(c); R^{iv} is methyl; and the ―COO―,―CH₂COO―or ―CH₂OCOO― group is located in the 3-position; or
(ii) R² is structure (d); R' is methyl or ethyl and R" is identical to R', or R'R"N- represents morpholino, piperidino, 1-pyrrolidinyl or 1-piperazinyl; R^{iv} is methyl; and alkylene is 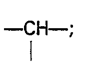 or
(iii) R⁺ is structure (e), alkylene is -CH₂CH₂- and R^{v} is H or-CONH₂.

14. A process accordina to Claim 13 (ii). wherein R"' is 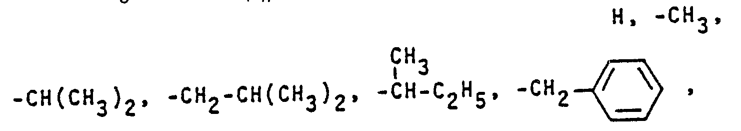 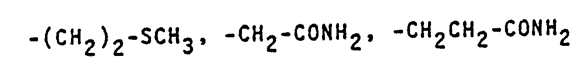 or

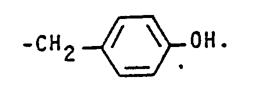

15. A process according to Claim 1, wherein p is equal to n.

16. A process according to Claim 15, wherein p is at least 4.

17. A process according to Claim 1, wherein r is 5, 6 or 7.

18. A process according to Claim 1, wherein O is the skeleton of inositol, n is 6 and p is 6.

19. A process according to Claim 18, wherein v is 5, r is 5 and s is 6.

20. A process according to Claim 1, said multiplet of formula (I) has the structural formula 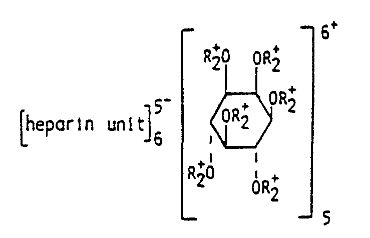 wherein R₂⁺ is

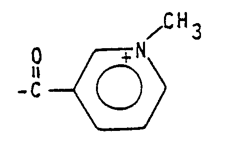

21. A process for the preparation of a salt of the structural formula 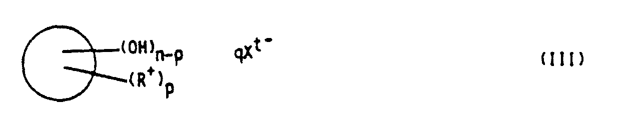 wherein O is the skeleton of a polyol, said skeleton being the portion of said polyol remaining after removal of all hydroxy substituents therefrom; n is a number from 3 to 24 which represents the total number of hydroxy groups in said polyol; p is a nuymber ≥ 3 and ≤ n; r⁺ is 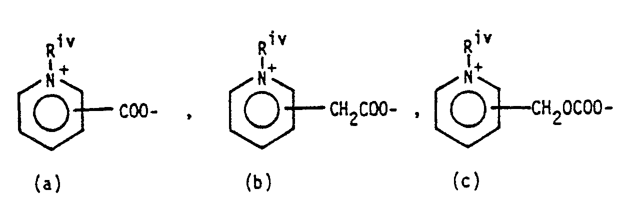 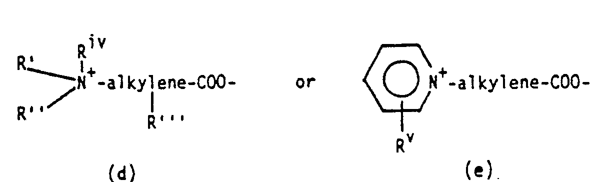 wherein the -COO-, -CH₂COO-, ―CH₂OCOO― and R" ring substituents can each be in the 2-, 3- or 4- position of the pyridinium ring; R'" is C₁―C₃ alkyl; R' and R", which can be the same or different, are each C₁-C₇ alkyl, or R' and R" are combined with the adjacent nitrogen atom such that 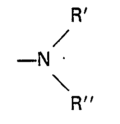 represents the residue of a saturated monocyclic secondary amine; R"' is 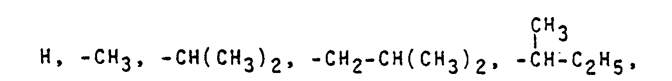 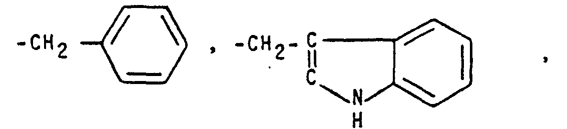 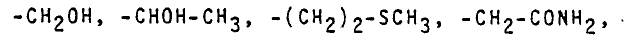 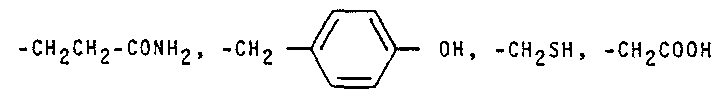 or 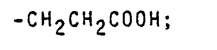 he alkylene groups can be straight or branched and contain 1 to 3 carbon atoms; R" is H, -CONH₂ or -COO(C₁―C₇alkyl); X- is the anion of a pharmaceutically acceptable organic or inorganic acid; t is the valence of the acid anion; and q is the number which when multiplied by t is equal to p; said process comprising:
(a) esterifying a polyol of the formula 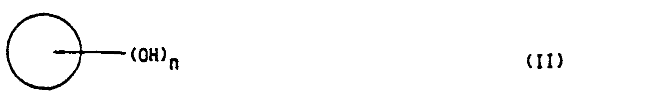 wherein O and n are as defined above, with a quaternary acid salt of the formula 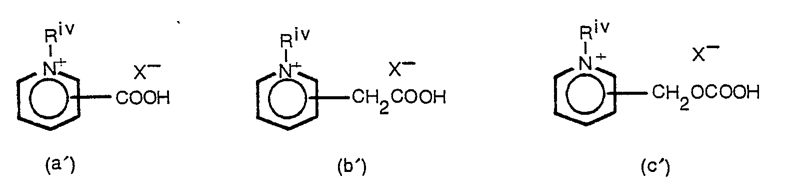 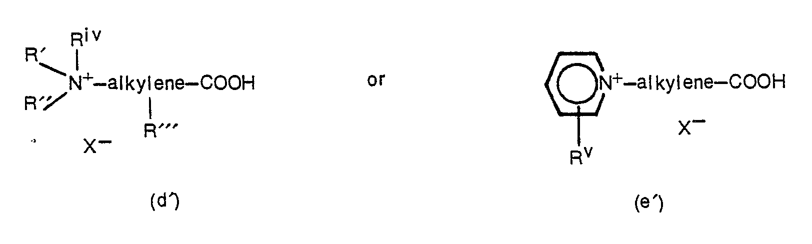 wherein the structural variables are as defined in connection with structures (a), (b), (c), (d) and (e), respectively, above and X- is as defined above, or with the corresponding salt of formula (III);
(b) esterifying a polyol of formula (II) above with a tertiary acid of the formula 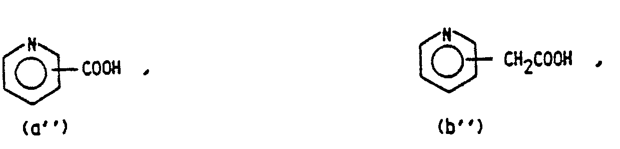 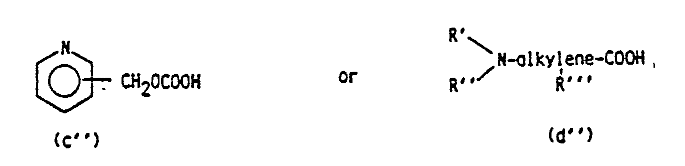 or with the corresponding acid halide or anhydride, wherein the structural variables are as defined in connection with structures (a), (b), (c) and (d), respectively, above, to afford the corresponding compound of the formula 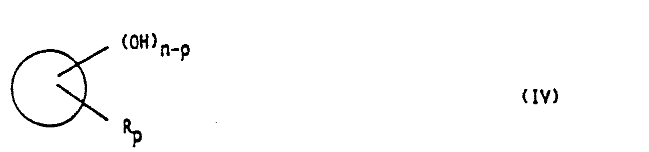 wherein O, n and p are as defined above and R is 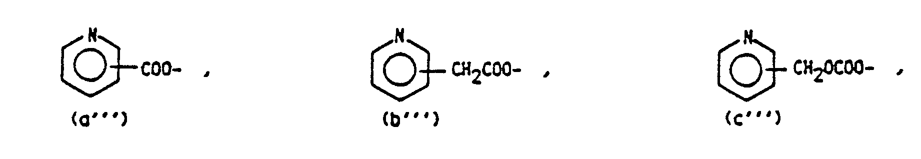 or 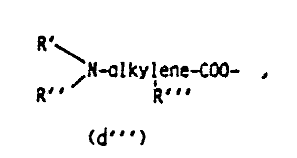 respectively, wherein the structural variables are as defined in connection with structures (a), (b), (c) and (d) above; followed by quaternizing the resultant compound of formula (IV) with an alkyl halide, R^{iv}-Hal, wherein R" is as defined above, to afford the corresponding salt of formula (III) wherein X- is a halide anion and R⁺ is structure (a), (b), (c) or (d), respectively;
(c) reacting a polyester of the formula 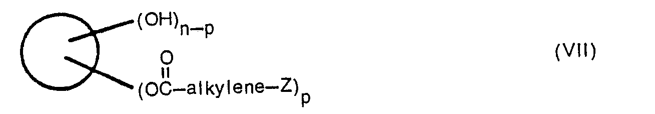 wherein O, n and p are as defined above, the alkylene group can be straight or branched and contains 1 to 3 carbon atoms and Z is Cl, Br or I, with excess reactant of the formula 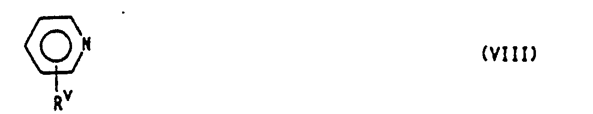 wherein R" is located in the 2-, 3- or 4-position of the pyridine ring and can be H, ―CONH₂ or ―COO(C₁―C₇ alkyl), to afford the corresponding salt of formula (III) wherein X- is Cl⁻, Br⁻ or I⁻ and R⁺ has structure (e);
(d) reacting an activated ester of the formula 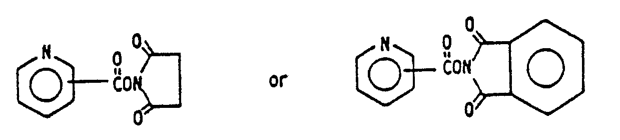 with a polyol of formula (II) above, followed by quaternizing the resultant compound of formula (IV) above wherein R is structure (a"') with an alkyl halide, R^{iv}-Hal, wherein R^{iv} is as defined above, to afford the corresponding salt of formula (III) wherien X- is a halide ion and R⁺ is structure (a);
(e) reacting a quaternized activated ester of the formula 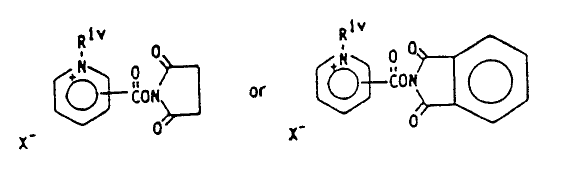 wherein R^{iv} and X- are as defined above, with a polyol of formula (II) above, to afford the corresponding salt of formula (III) wherein R⁺ is structure (a);
(f) reacting a quaternized anhydride of the formula 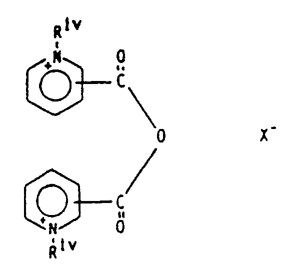 wherein X- and R^{iv} are as defined above, with a polyol of formula (II) above, to afford the corresponding salt of formula (III) wherein R⁺ is structure (a); or
(g) subjecting a salt of formula (III) to ion exchange to afford a salt of formula (III) containing a different X- anion.

22. A process according to Claim 21, wherein the polyol skeleton is that of a monosaccharide or an oligosaccharide.

23. A process according to Claim 22, wherein the monosaccharide is a pentose, hexose or heptose, or wherein the oligosaccharide is a disaccharide, a trisaccharide or a cyclodextrin.

24. A process according to Claim 21, wherein the polyol skeleton is that of a C₃―C₁₅ aliphatic polyhydroxy compound or a C₅―C₁₈ alicyclic polyhydroxy compound.

25. A process according to Claim 24, wherein the aliphatic polyhydroxy compound is a C₃―C₈ alkyl polyol, or wherein the alicyclic polyhydroxy compound is a C₅―C₁₀ cycloalkyl polyol or a fused fully hydrogenated aromatic phenol.

26. A process according to Claim 25, wherein the C_{S}-C₁ₒ cycloalkyl polyol is a cyclohexane polyol.

27. A process according to Claim 26, wherein the cyclohexane polyol is inositol.

28. A process according to Claim 21, wherein R' and R" are combined with the adjacent nitrogen atom such that 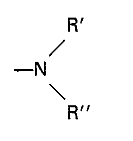 represents the residue of a saturated monocyclic secondary amine having 5 to 7 ring atoms, optionally containing another hetero ring atom ―O―, ―S― or ―N― in addition to the indicated nitrogen atom, and optionally bearing 1 to 3 methyl substituents.

29. A process according to Claim 21, wherein:
(i) R⁺ is structure (a), (b) or (c); R^{iv} is methyl; and the―COO―,―CH₂COO―or―CH₂OCOO―group is located in the 3-position; or
(ii) R² is structure (d); R' is methyl or ethyl and R" is identical to R', or R'R"N- represents morpholino, piperidino, 1-pyrrolidinyl or 1-piperazinyl; R^{iv} is methyl; and alkylene is 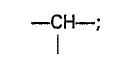 or
(iii) R⁺ is structure (e), alkylene is ―CH₂CH₂― and R^{v} is H or ―CONH₂.

30. A process according to Claim 21, wherein p is equal to n, or wherein t is 1, or wherein X⁻ is I⁻, Br or CI-.

31. A process according to Claim 21, wherein O is the skeleton of inositol, n is 6 and p is 6.

32. A process according to Claim 21, wherein the salt of formula (III) has the structural formula 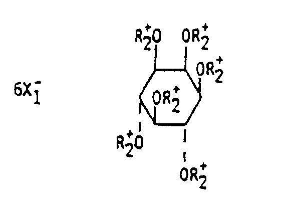 wherein X⁻₁, is I⁻, Br- or CI- and R2 is

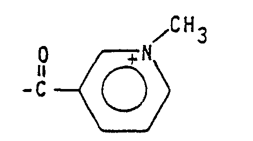

## Patentansprüche (Patentansprüche für folgende(n) Vertragsstaat(en) : BE CH DE FR GB IT LU NL SE)

1. Multiplett mit der Strukturformel 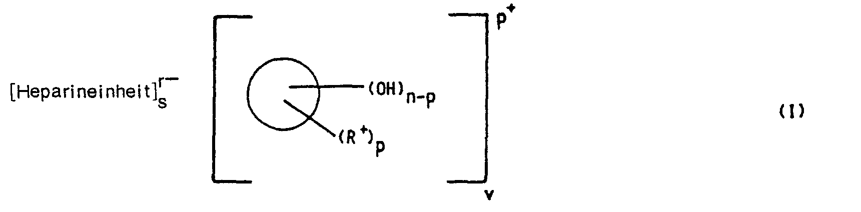 worin O das Gerüst eines Polyols its, wobei das Gerüst derjenige Teil des Polyols ist, welcher nach Entfernung aller Hydroxy-Substituenten aus diesem übrig bleibt; n eine Zahl von 3 bis 24 ist, welche die Gesamtzahl von Hydroxygruppen in dem Polyol darstellt; p eine Zahl ≥3 und ≤n ist; r die verfügbare Wertigkeit der Heparineinheit ist und ≥3 und ≤7 ist; s diejenige Zahl ist, welche, wenn sie mit r multipliziert wird, gleich pv ist; v diejenige Zahl ist, welche, wenn sie mit p multipliziert wird, gleich rs ist; R⁺ bedeutet 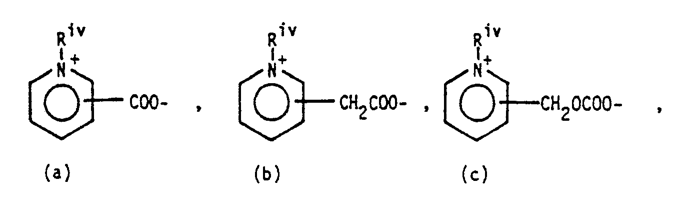 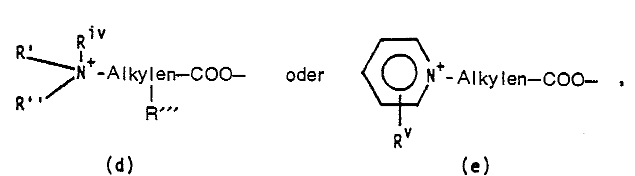 wobei sich die ―COO―, ―CH₂COO―, ―CH₂OCOO― und R^{v}- Ring-Substituten jeweils in der 2-, 3- oder 4-Stellung des Pyridiniumrings befinden können; R^{iv} bedeutet C₁―C₃-Alkyl; R' und R", die gleich oder verschieden sein können, jeweils ein Cₗ-C₇-Alkyl sind, oder R' und R" mit dem benachbarten Stickstoffatom verbunden sind, so daß 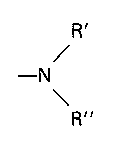 den Rest eines gesättigten monozyklischen sekundären Amins darstellt;
R"' bedeutet 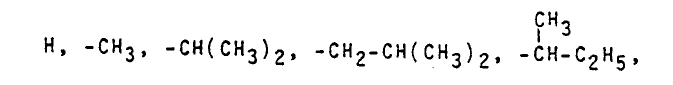 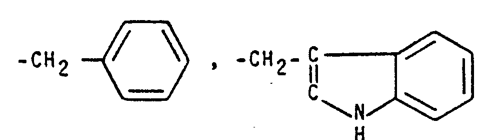 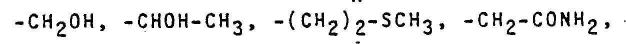 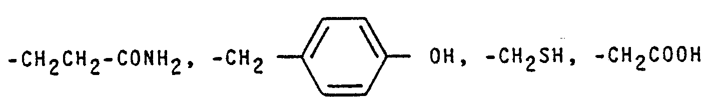 oder 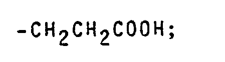 die Alklylengruppen gerade oder verzweigt sein und 1 bis 3 Kohlenstoffatome enthalten können; und R" bedeutet H, -CONH₂ oder ―COO(C₁―C₇-Alkyl).

2. Multiplett nach Anspruch 1, worin das Polyol ein Monosacharid oder ein Oligosacharid ist.

3. Multiplett nach Anspruch 2, worin das Monosacharid eine Pentose, Hexose oder Heptose ist, oder wobei das Oligosacharid ein Disacharid, ein Trisacharid oder ein Zyklodextrin ist.

4. Multiplett nach Anspruch 3, worin das Polyol eine Ribose, Arabinose, Xylose, Lyxose, Ribulose, Xylulose, Glukose, Galaktose, Mannose, Fruktose, Sorbose, Tagatose, Mannoheptulose, Sedoheptulose, Sucrose, Lactose, Maltose, Raffinose, ein a-Zyklodextrin, β-Zyklodextrin oder y-Zyklodextrin ist.

5. Multiplett nach Anspruch 1, worin das Polyol eine C₃―C₁₅-aliphatische Polyhydroxyverbindung oder eine C₅―C₁₈-alizyklische Polyhydroxyverbindung ist.

6. Multiplett nach Anspruch 5, worin die aliphatische Polyhydroxyverbindung ein C₃―C₈-Alkylpolyol ist, oder wobei die alizyklische Polyhydroxyverbindung ein kondensiertes, vollständig hydriertes aromatisches Polyol ist.

7. Multiplett nach Anspruch 6, worin das C₃―C₈-Alkylpolyol Glyzerin, Erythrit oder 1,2,6-Trihydroxyhexan ist, oder wobei das kondensierte, vollständig hydrierte aromatische Polyol ein Dekahydronaphthalinpolyol ist.

8. Multiplett nach Anspruch 5, worin die alizyklische Polyhydroxy-Verbindung ein C₅-C₁₀-Zykloalkyl- polyol ist.

9. Multiplett nach Anspruch 8, worin das C₅―C₁₀-Zykloalkylpolyol ein Zyklohexanpolyol ist.

10. Multiplett nach Anspruch 9, worin das Zyklohexanpolyol Inositol ist.

11. Multiplett nach Anspruch 1, worin R' und R" mit dem benachbarten Stickstoffatom verbunden sind, so daß 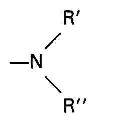 den Rest eines gestättigten monozyklischen sekundären Amins darstellt, das 5 bis 7 Ringatome aufweist und wahlweise ein weiteres Heteroringatom -0-, ―S― oder -N- zusätzlich zu dem indizierten Stickstoffatom enthält sowie wahlweise 1 bis 3 Methylsubstituenten trägt.

12. Multiplett nach Anspruch 11, worin 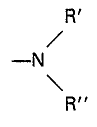 einen Morpholin-, 1-Pyrrolidinyl-, Perhydro-1,2,4-Oxathiazin-4-yl-, 1- oder 4-Piperazinyl, 4-Methyl-1-Piperazinyl-, Piperidin-, Hexamethylenimin-, 2-Methyl-1-Pyrazolidinyl-, 1- oder 2-Pyrazolidinyl-, 3-Methyl-1-Imidazolidinyl- oder 1- oder 3-Imidazolidinyl-Rest bedeutet.

13. Multiplett nach Anspruch 1, worin
(i) R⁺ bedeutet Struktur (a), (b) oder (c); R^{iv} bedeutet einen Methyl-Rest; und die―COO―,―CH₂COO― oder ―CH₂OCOO― -Gruppe in der 3-Stellung angeordnet ist; oder
(ii) R⁺ bedeutet Struktur (d); R' einen Methyl- oder Ethyl-Rest bedeutet und R." mit R' identisch ist, oder R'R"N- einen Morpholin-, Piperidin-, 1-Pyrrolidinyl- oder 1-Piperazinyl-Rest; R'" Methyl; und Alkylen -CH- bedeutet; oder
(iii) R⁺Struktur (e), Alkylen -CH₂CH₂- und R" H oder -CONH₂ bedeutet.

14. Multiplett nach Anspruch 13 (ii) worin R'" bedeutet 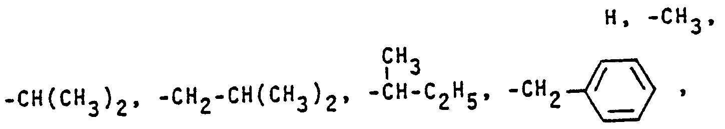 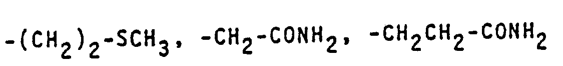 oder

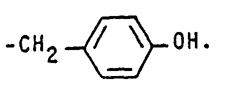

15. Multiplett nach Anspruch 1, worin p gleich n ist.

16. Multiplett nach Anspruch 15, worin p mindestens gleich 4 ist.

17. Multiplett nach Anspruch 1, worin r bedeutet 5, 6 oder 7.

18. Multiplett nach Anspruch 1, worin 0 bedeutet das Skelett von Inositol, n bedeutet 6 und p bedeutet 6.

19. Multiplett nach Anspruch 18, worin v bedeutet 5, r bedeutet 5 und s bedeutet 6.

20. Multiplett nach Anspruch 1, worin das Multiplett die Strukturformel 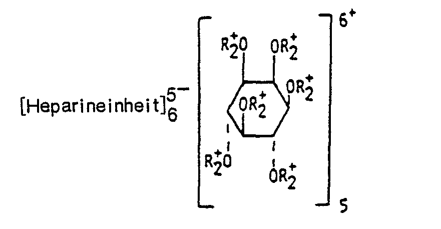 aufweist, wobei R₂⁺ bedeutet

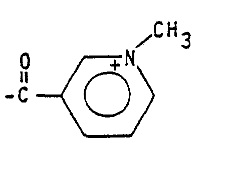

21. Pharmazeutische Zusammensetzung von Substanzen mit einem Multiplett nach der Formel (I), wie in Anspruch 1 definiert, und einer nichttoxischen, pharmazeutisch annehmbaren Trägersubstanz für dieses.

22. Pharmazeutische Zusammensetzung von Substanzen in Form von Dosierungseinheiten zur Verwendung bei der Auslösung einer antikoagulierenden Reaktion in einem warmblütigen Tier, wobei die Zusammensetzung pro Dosierungseinheit eine wirksame Einheit einer Antikoagulansmenge eines Multipletts nach Formel (I), wie in Anspruch 1 definiert, und eine nichttoxische, pharmazeutisch annehbare Trägersubstanz für diese aufweist, wobei die Zusammensetzung für nasale Applikation, für orale Inhalationstherapie oder für eine andere orale Applikation geeignet ist.

23. Salz mit der Strukturformel 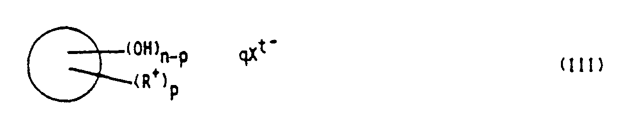 worin O das Gerüst eines Polyols ist, wobei das Gerüst derjenige Teil des Polyols ist, welcher nach Entfernung aller Hydroxy-Substituenten aus diesem übrig bleibt; n eine Zahl von 3 bis 24 ist, welche die Gesamtzahl von Hydroxygruppen in dem Polyol darstellt; p eine Zahl ≥ 3 und ≤ n ist; R⁺ bedeutet 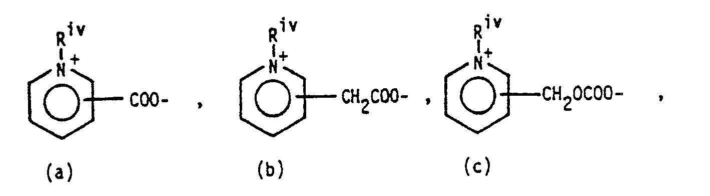 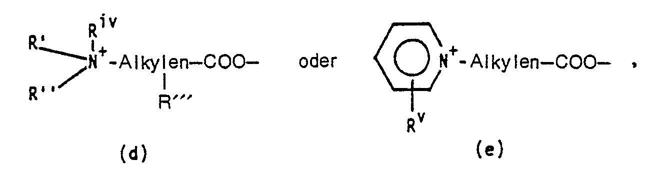 wobei sich die ―COO―,―CH₂COO―, ―CH₂OCOO― und R^{V-}Ring-Substituenten jeweils in der 2-, 3- oder 4-Stellung des Pyridiniumrings befinden können, R^{iv} bedeutet C₃―C₃-Alkyl; R' und R", die gleich oder verschieden sein können, jeweils ein Cₗ-C₇-Alkyl sind, oder R' und R" mit dem benachbarten Stickstoffatom verbunden sind, so daß 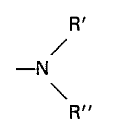 den Rest eines gesättigten monozyklischen sekundären Amins darstellt; R"' bedeutet 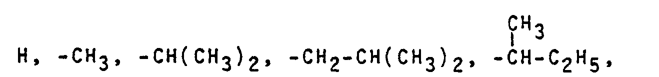 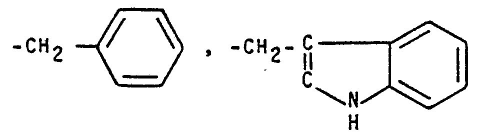 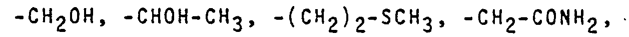 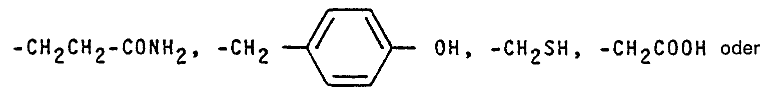 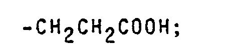 die Alkylgruppen gerade oder verzweigt sein und 1 bis 3 Kohlenstoffatome enthalten können; R^{v} bedeutet H, -CONH₂ oder ―COO(C₁―C₇); X- das Anion einer pharmazeutisch annehmbaren organischen oder anorganischen Säure ist; t die Wertigkeit des Säureanions ist; und q diejenige Zahl ist, welche, wenn sie mit t multipliziert wird, gleich p ist.

24. Salz nach Anspruch 23, worin das Polyol ein Monosacharid oder ein Oligosacharid ist.

25. Salz nach Anspruch 24, worin das Monosacharid eine Pentose, Hexose oder Heptose ist, oder wobei das Oligosacharid ein Disacharid, ein Trisacharid oder ein Zyklodextrin ist.

26. Salz nach Anspruch 23, worin das Polyol eine C₃―C₁₅-aliphatische Polyhydroxyverbindung oder eine C₅―C₁₈-alizyklische Polyhydroxyverbindung ist.

27. Salz nach Anspruch 26, worin die aliphatische Polyhydroxyverbindung ein C₃―C₈-Alkylpolyol ist, oder wobei die alizykische Polyhydroxyverbinding ein C₅―C₁₀-Zylkoalkylpolyol oder ein kondensiertes, vollständig hydriertes aromatisches Polyol ist.

28. Salz nach Anspruch 27, worin das C₅―C₁₀-Zykloalkylpolyol ein Zyklohexanpolyol ist.

29. Salz nach Anspruch 8, worin das Zyklohexanpolyol Inositol ist.

30. Salz nach Anspruch 23, worin R' und R" mit dem benachbarten Stickstoffatom verbunden sind, so daß 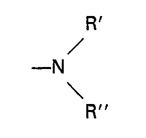 den Rest eines gesättigten monozyklischen sekundären Amins darstellt, das 5 bis 7 Ringatome aufweist und wahlweise ein weiteres Heteroringatom -0-, -S- oder -N- zusätzlich zu dem indizierten Stickstoffatom enthält sowie wahlweise 1 bis 3 Methylsubstituenten trägt.

31. Salz nach Anspruch 23, worin:
(i) R⁺ bedeutet Struktur (a), (b) oder (c); R^{iv} bedeutet Methyl; und die -COO-, -CH₂-COO oder ―CH₂OCOO― -Gruppe in der 3-Stellung angeordnet ist; oder
(ii) R⁺ bedeutet Struktur (d); R' bedeutet einen Methyl- oder Ethyl-Rest und R" mit R' identisch ist, oder R'R"N- bedeutet einen Morpholin-, Piperidin-, 1-Pyrrolidinyl- oder 1-Piperazinyl-rest; R^{iv} bedeutet einen Methyl-Rest; und Alkylen bedeutet -CH-; oder
(iii) R⁺ bedeutet Struktur (e), Alkylen bedeutet―CH₂CH₂― und R" bedeutet H oder―CONH₂.

32. Salz nach Anspruch 23, worin p gleich n ist oder wobei t gleich 1 ist oder wobei X- bedeutet I⁻ Br⁻ oder CI-.

33. Salz nach Anspruch 23, worin O bedeutet das Skelett von Inositol, n bedeutet 6 und p bedeutet⁶.

34. Salz nach Anspruch 33 mit der Strukturformel 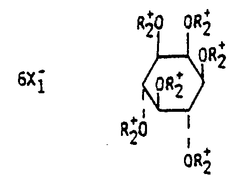 wobei X₁⁻ bedeutet I⁻, Br⁻ oder Cl⁻ und R₂⁺ bedeutet

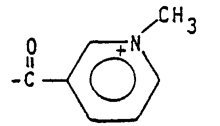

## Patentansprüche (Patentansprüche für folgende(n) Vertragsstaat(en) : AT)

1. Verfahren zur Herstellung eines Multipletts mit der Strukturformel 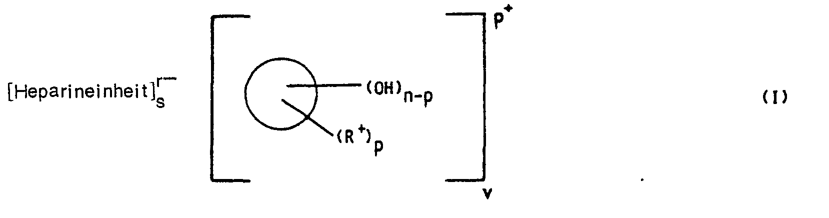 worin O das Gerüst eines Polyols ist, wobei das Gerüst derjenige Teil des Polyols ist, welcher nach Entfernung aller Hydroxy-Substituenten aus diesem übrig bleibt; n eine Zahl von 3 bis 24 ist, welche die Gesamtzahl von Hydroxygruppen in dem Polyol darstellt; p eine Zahl ≥3 und ≤n ist; r die verfügbare Wertigkeit der Heparineinheit ist und ≥3 und ≤7 ist; s diejenige Zahl ist, welche, wenn sie mit r multipliziert wird, gleich pv ist; v diejenige Zahl ist, welche, wenn sie mit p multipliziert wird, gleich rs ist; R⁺ bedeutet 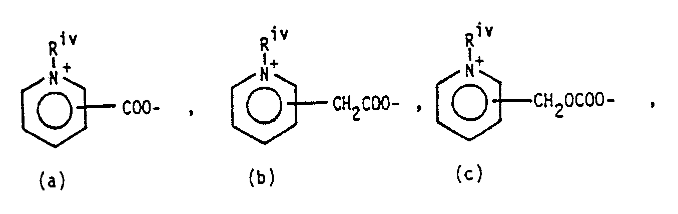 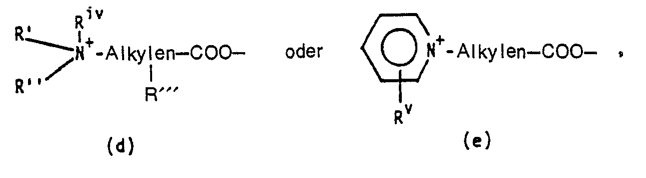 wobei sich die ―COO―, ―CH₂COO―, ―CH₂OCOO― und R^{v}- Ring-Substituten jeweils in der 2-, 3- oder 4-Stellung des Pyridiniumrings befinden können; R^{iv} bedeutet C₁―C₃-Alkyl; R' und R", die gleich oder verschieden sein können, jeweils ein C₁―C₇-Alkyl sind, oder R' und R" mit dem benachbarten Stickstoffatom verbunden sind, so daß 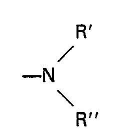 den Rest eines gesättigten monozyklischen sekundären Amins darstellt;
R"' bedeutet 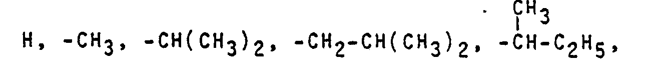 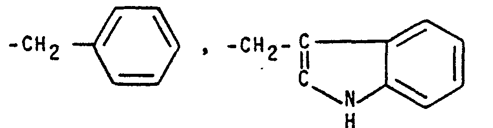 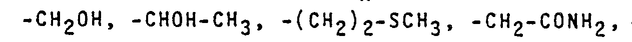 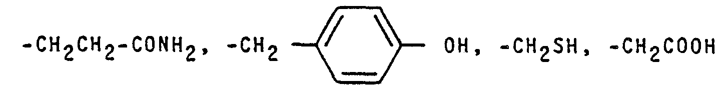 oder 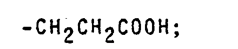 die Alklylengruppen gerade oder verzweigt sein und 1 bis 3 Kohlenstoffatome enthalten können; und R" bedeutet H,―CONH₂ oder―COO(C₁―C₇-Alkyl); wobei dem Verfahren
(a) das entsprechende Salz mit der Strukturformel 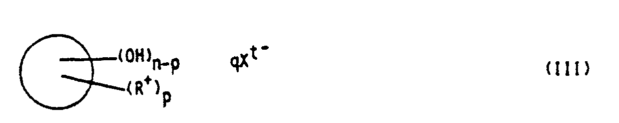 wobei O, n, p und R⁺ wie in der oben genannten Formel (I) definiert sind, X- das Anion einer pharmazeutisch annehmbaren organischen oder anorganischen Säure ist, t die Wertigkeit des Säureanions ist; und q diejenige Zahl ist, welche, wenn sie mit t multipliziert wird, gleich p ist, mit Natriumheparin in einem wässrigen Medium zur Reaktion gebracht wird; oder
(b) das entsprechende Salz mit der oben genannten Formel (III) einem Anionenaustausch unterzogen wird, um die X--Anionen im Salz durch OH--Anionen zu erzetzen, wonach die sich ergebende Zwischenverbindung mit Heparinsäure zur Reaktion gebracht wird.

2. Verfahren nach Anspruch 1, wobei das Polyolgerüst dasjenige eines Monosacharids oder eines Oligosacharids ist.

3. Verfahren nach Anspruch 2, wobei das Monosacharid eine Pentose, Hexose oder Heptose ist, oder wobei das Oligosacharid ein Disacharid, ein Trisacharid oder ein Zyklodextrin ist.

4. Verfahren nach Anspruch 1, wobei das Polyolgerüst dasjenige von Ribose, Arabinose, Xylose, Lyxose, Ribulose, Xylulose, Glukose, Galaktose, Mannose, Fruktose, Sorbose, Tagatose, Mannoheptulose, Sedoheptulose, Sucrose, Lactose, Maltose, Raffinose, a-Zkylodextrin, β-Zyklodextrin oder y-Zyklodextrin ist.

5. Verfahren nach Anspruch 1, wobei das Polyolgerüst dasjenige einer C₃―C₁₅-aliphatischen Polyhydroxyverbindung oder einer C₅―C₁₈-alizyklischen Polyhydroxyverbindung ist.

6. Verfahren nach Anspruch 5, wobei die aliphatische Polyhydroxyverbindung ein C₃―C₈-Alkylpolyol ist, oder wobei die alizyklische Polyhydroxyverbindung ein kondensiertes, vollständig hydriertes aromatisches Polyol ist.

7. Verfahren nach Anspruch 6, wobei das C₃―C₈-Alkylpolyol Glyzerin, Erythrit oder 1,2,6-Trihydroxyhexan ist, oder wobei das kondensierte, vollständig hydrierte aromatische Polyol ein Dekahydronaphthalinpolyol ist.

8. Verfahren nach Anspruch 5, wobei die alizyklische Polyhydroxy-Verbindung ein C₅―C₁₀-Zykloalkyl- polyol ist.

9. Verfahren nach Anspruch 8, wobei das C₅―C₁₀-Zykloalkylpolyol ein Zyklohexanpolyol ist.

10. Verfahren nach Anspruch 9, wobei das Zyklohexan Inositol ist.

11. Verfahren nach Anspruch 1, wobei R' und R" mit dem benachbarten Stickstoffatom verbunden sind, so daß 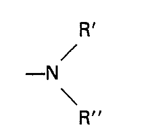 den Rest eines gestättigten monozyklischen sekundären Amins darstellt, das 5 bis 7 Ringatome aufweist und wahlweise ein weiteres Heteroringatom -0-, -S- oder -N- zusätzlich zu dem indizierten Stickstoffatom enthält sowie wahlweise 1 bis 3 Methylsubstituenten trägt.

12. Verfahren nach Anspruch 11, wobei 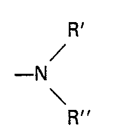 einen Morpholin-, 1-Pyrrolidinyl-, Perhydro-1,2,4-Oxathiazin-4-yl-, 1- oder 4-Piperazinyl, 4-Methyl-1-Piperazinyl-, Piperidin-, Hexamethylenimin-, 2-Methyl-1-Pyrazolidinyl-, 1- oder 2-Pyrazolidinyl-, 3-Methyl-1- lmidazolidinyl- oder 1- oder 3-Imidazolidinyl-Rest bedeutet.

13. Verfahren nach Anspruch 1, wobei
(i) R⁺ bedeutet Struktur (a), (b) oder (c); R^{iv} bedeutet einen Methyl-Rest; und die―COO―, ―CH₂COO― oder―CH₂OCOO―-Gruppe in der 3-Stellung angeordnet ist; oder
(ii) R⁺ bedeutet Struktur (d); R' einen Methyl- oder Ethyl-Rest bedeutet und R" mit R' identisch ist, oder R'R"N- einen Morpholin-, Piperidin-, 1-Pyrrolidinyl- oder 1-Piperazinyl-Rest; R^{iv} Methyl; und Alkylen 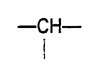 bedeutet; oder
(iii) R⁺ Struktur (e), Alkylen -CH₂CH₂- und R" H oder -CONH₂ bedeutet.

14. Verfahren nach Anspruch 13 (ii) wobei R'" bedeutet 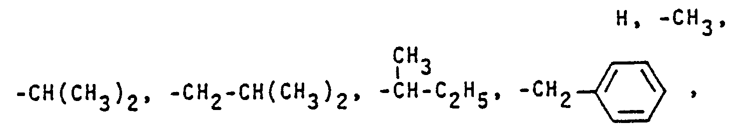 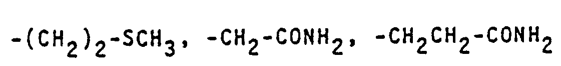 oder

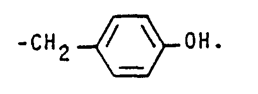

15. Verfahren nach Anspruch 1, wobei p gleich n ist.

16. Verfahren nach Anspruch 15, wobei p mindestens gleich 4 ist.

17. Verfahren nach Anspruch 1, wobei r bedeutet 5, 6 oder 7.

18. Verfahren nach Anspruch 1, wobei O bedeutet das Skelett von Inositol, n bedeutet 6 und p bedeutet 6.

19. Verfahren nach Anspruch 18, wobei v bedeutet 5, r bedeutet 5 und s bedeutet 6.

20. Verfahren nach Anspruch 1, wobei das Multiplett von Formel (I) die Strukturformel 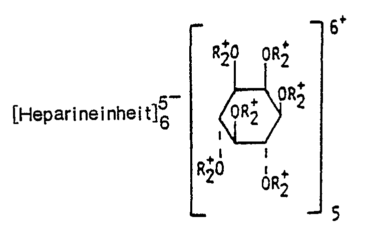 aufweist, wobei R₂⁺ bedeutet

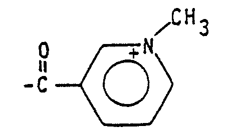

21. Verfahren zur Herstellung eines Salzes mit der Strukturformel 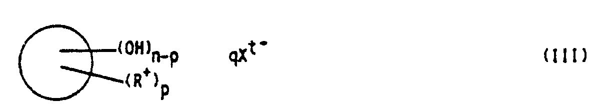 worin O das Gerüst eines Polyols ist, wobei das Gerüst derjenige Teil des Polyols ist, welcher nach Entfernung aller Hydroxy-Substituenten aus diesem übrig bleibt; n eine Zahl von 3 bis 24 ist, welche die Gesamtzahl von Hydroxygruppen in dem Polyol darstellt; p eine Zahl ≥ 3 und ≤ n ist; R⁺ bedeutet 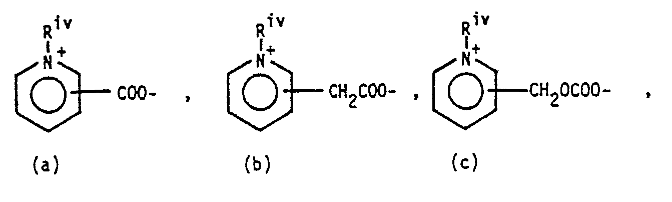 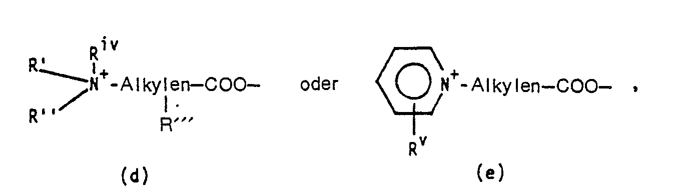 wobei sich die―COO―, ―CH₂COO―, ―CH₂OCOO― und R^{v}-Ring-Substituenten jeweils in der 2-, 3- oder 4-Stellung des Pyridiniumrings befinden können, R'" bedeutet C₁―C₃-Alkyl; R' und R", die gleich oder verschieden sein können, jeweils ein C₁-C₇₋Alkyl sind, oder R' und R" mit dem benachbarten Stickstoffatom verbunden sind, so daß 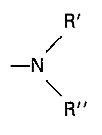 den Rest eines gesättigten monozyklischen sekundären Amins darstellt; R"' bedeutet 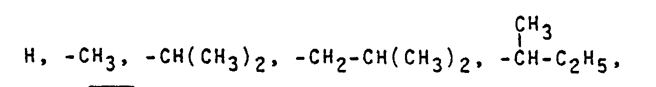 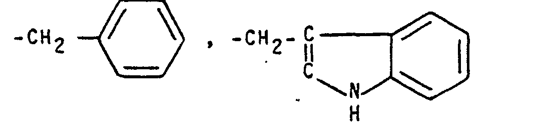 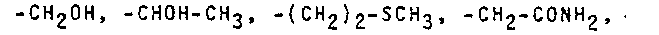 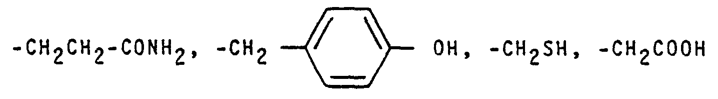 oder 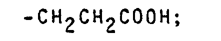 die Alkylgruppen gerade oder verzweigt sein und 1 bis 3 Kohlenstoffatome enthalten können; R^{v} bedeutet H, -CONH₂ oder ―COO(C₁―C₇-Alkyl); X- das Anion einer pharmazeutisch annehmbaren organischen oder anorganischen Säure ist; t die Wertigkeit des Säureanions ist; und q diejenige Zahl ist, welche, wenn sie mit t multipliziert wird, gleich p ist; wobei bei dem Verfahren
(a) ein Polyol mit der Formel 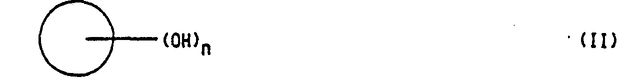 wobei O und n wie oben definiert sind, mit einem quaternären sauren Salz mit der Formel 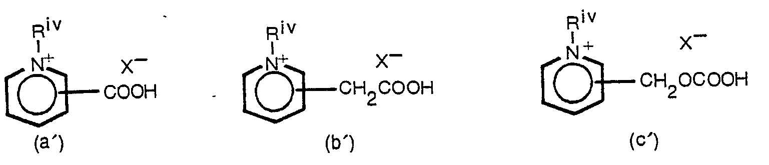 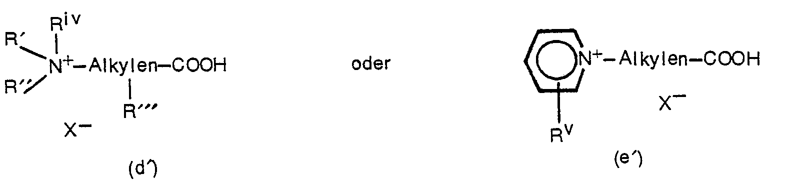 verestert wird, wobei die Strukturvariablen wie oben in Verbindung mit den jeweiligen Strukturen (a), (b), (c), (d) und (e) definiert sind und X- wie oben oder mit dem entsprechenden Mischanhydrid definiert ist, um das entsprechende Salz von Formel (III) zu erhalten;
(b) ein Polyol mit der oben genannten Formel (II) mit einer tetiären Säure mit der Formel 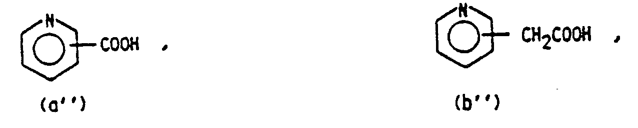 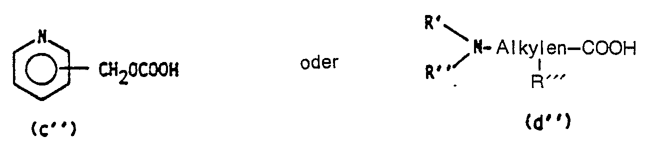 oder mit dem entsprechenden Säurehalogenid oder -anhydrid verestert wird, wobei di Strukturvariablen wie oben in Verbindung mit den jeweiligen Strukturen (a), (b), (c), und (d) definiert sind, um die entsprechende Verbindung mit der Formel 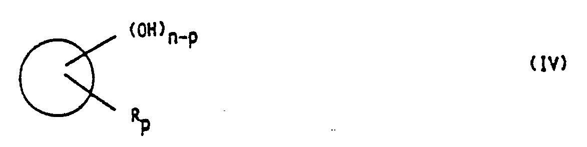 zu erhalten, wobei O, n und p wie oben definiert sind und R jeweils 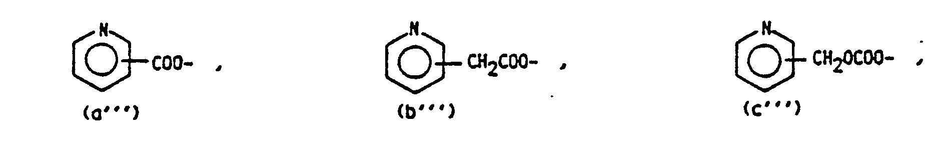 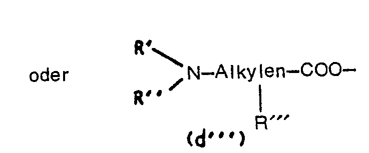 bedeutet, wobei die Strukturvariablen wie oben in Verbindung mit den Strukturen (a), (b), (c) und (d) definiert sind; wonach die sich ergebende Verbindung von Formel (IV) mit einem Alkylhalogenid, R'^{v-}Hal, quaternisiert wird, wobei R" wie oben definiert ist, um das entsprechende Salz von Formel (III) zu erhalten, wobei X- ein Halogenenidanion ist und R⁺ jeweils Struktur (a), (b), (c) oder (d) ist,
(c) ein Polyester mit der Formel 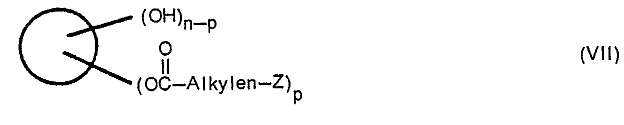 wobei O, n und p wie oben definiert sind, die Alkylengruppe gerade oder verzweigt sein kann und 1 bis 3 Kohlenstoffatome enthält, und Z bedeutet CI, Br oder 1, mit einem Überschußreaktionsmittel mit der Formel 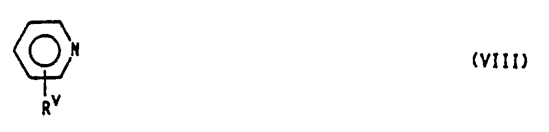 zur Reaktion gebracht wird, wobei R^{v} in der 2-, 3- oder 4-Stellung des Pyridinrings angeordnet ist und H, -CONH₂ oder -COO(C₁-C₇-Alkyl) bedeuten kann, um das entsprechende Salz mit der Formel (III) zu erhalten, wobei X- bedeutet CI-, Br⁻ oder I⁻ und R⁺ die Struktur (e)⁻aufweist;
(d) ein aktivierter Ester mit der Formel 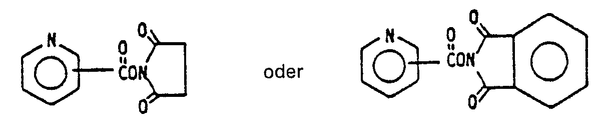 mit einem Polyol der oben genannten Formel (II) zur Reaktion gebracht wird, wonach die sich ergebende Verbindung mit der oben genannten Formel (IV), wobei R bedeutet Struktur (a"') mit einem Alkylhalogenid, R^{iv}-Hal, quarternisiert wird, wobei R^{iv} wie oben definiert ist, um das entsprechende Salz von Formel (III) zu erhalten, wobei X- ein Halogenidion bedeutet und R⁺ bedeutet Struktur (a);
(e) ein quaternisierter aktivierter Ester mit der Formel 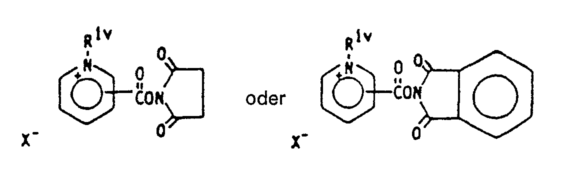 wobei R^{iv} und X- wie oben definiert sind, mit einem Polyol der oben genannten Formel (II) zur Reaktion gebracht wird, um das entsprechende Salz von Formel (III) zu erhalten, wobei R⁺ bedeutet Struktur (a);
(f) ein quaternisiertes Anhydrid mit der Formel 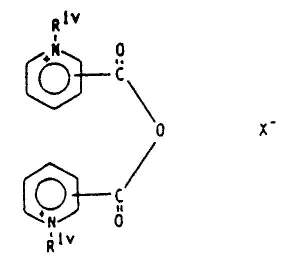 wobei X- und R^{iv} wie oben definiert sind, mit einem Polyol der oben genannten Formel (II) zur Reaktion gebracht wird, um das entsprechende Salz von Formel (III) zu erhalten, wobei R⁺ bedeutet Struktur (a); oder
(g) ein Salz von Formel (III) einem lonenaustausch unterzogen wird, um ein Salz von Formel (III) zu erhalten, das ein anderes X--Anion enthält.

22. Verfahren nach Anspruch 21, wobei das Polyolgerüst dasjenige eines Monosacharids oder eines Oligosacharids ist.

23. Verfahren nach Anspruch 22, wobei das Monosacharid eine Pentose, Hexose oder Heptose ist, oder wobei das Oligosacharid ein Disacharid, ein Trisacharid oder eine Zyklodextrin ist.

24. Verfahren nach Anspruch 21, wobei das Polyolgerüst dasjenige einer C₃―C₁₅-aliphatischen Polyhydroxyverbindung oder einer C₅―C₁₈-alizyklischen Polyhydroxyverbindung ist.

25. Verfahren nach Anspruch 24, wobei die aliphatische Polyhydroxyverbindung ein C₃―C₈-Alkylpolyol ist, oder wobei die alizyklische Polyhydroxyverbindung ein C₅―C₁₀-Zykloalkylpolyol oder ein kondensiertes, vollständig hydriertes aromatisches Polyol ist.

26. Verfahren nach Anspruch 25, wobei das C₅―C₁₀-Zykloalkylpolyol ein Zyklohexanpolyol ist.

27. Verfahren nach Anspruch 26, wobei das Zyklohexanpolyol Inositol ist.

28. Verfahren nach Anspruch 21, wobei R' und R" mit dem benachbarten Stickstoffatom verbunden sind, so daß 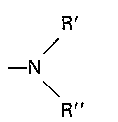 den Rest eines gesättigten monozyklischen sekundären Amins darstellt, das 5 bis 7 Ringatome aufweist und wahlweise ein weiteres Heteroringatom -0-, -S-, oder -N- zusätzlich zu dem indizirten Stickstoffatom enthält sowie wahlweise 1 bis Methylsubstituenten trägt.

29. Verfahren nach Anspruch 21, wobei:
(i) R⁺ bedeutet Struktur (a), (b) oder (c); R^{iv} bedeutet einen Methyl-Rest, und die ―COO―, ―CH₂COO― oder ―CH₂OCOO-Gruppe in der 3-Stellung angeordnet ist; oder
(ii) R⁺ bedeutet Struktur (d); R' bedeutet einen Methyl- oder Ethyl-Rest und R" mit R' identisch ist, oder R'R"N- einen Morpholin-, Piperidin-, 1-Pyrrolidinyl- oder 1-Piperazinyl-Rest; R'" einen Methyl-Rest; und Alkylen 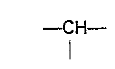 bedeutet; oder
(iii) R⁺ bedeutet Struktur (e), Alkylen bedeutet -CH₂CH₂- und R" bedeutet H oder -CONH₂.

30. Verfahren nach Anspruch 21, wobei p gleich n ist oder wobei t gleich 1 ist oder wobei X- bedeutet I⁻, Br⁻ oder CI-.

31. Verfahren nach Anspruch 21, wobei O bedeutet das Skelett von Inositol, n bedeutet 6 und p bedeutet 6.

32. Verfahren nach Anspruch 21, wobei das Salz von Formel (III) die Strukturformel 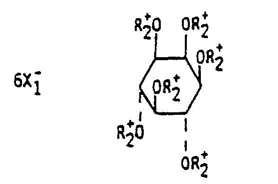 aufweist, wobei X⁻₁ bedeutet I⁻, Br oder CI- und R2 bedeutet

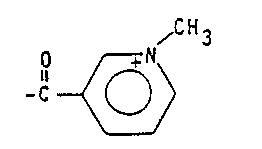

## Revendications (Revendications pour l'(les) Etat(s) contractant(s) suivant(s) : BE CH DE FR GB IT LU NL SE)

1. Multiplet à formule de structure 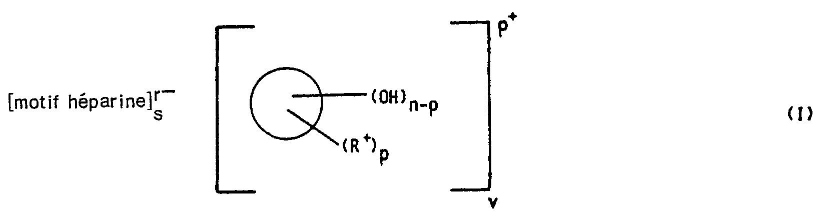 où O est le squelette d'un polyol, ce squelette étant la partie dudit polyol demeurant après qu'on ait éliminé de celui-ci tous les substituants hydroxy; n est un nombre de 3 à 24 qui représente le nombre total de groupes hydroxy présents dans ledit polyol; p est un nombre ≥3 et ≤N; r est la valence disponible du motif héparine et est ≥3 et ≤7; s est le nombre qui multiplié par r est égal à pv; v est le nombre qui multiplié par p est égal à rs; R⁺ est 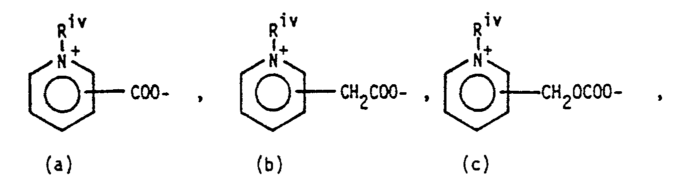 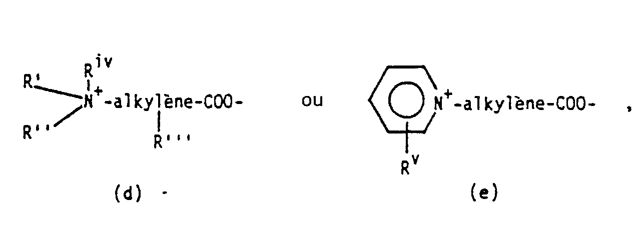 où les substituants de noyau -COO-, ―CH₂COO―, -CH₂0COO- et R^{v} peuvent se trouver chacun en 2, en 3 ou en 4 sur le noyau pyridinium; R^{iv} est alkyle en C₁―C₃; R' et R", qui peuvent être semblables ou différents, sont chacun alkyle en C₁―C₇, ou R' et R" sont combinés avec l'atome d'azote voisin de sorte que 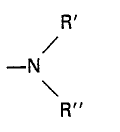 représente le résidu d'une amine secondaire monocyclique saturée; R"' est 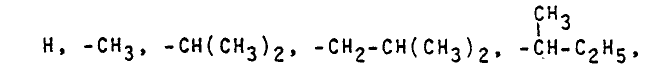 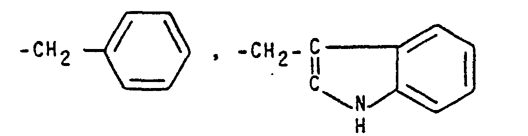 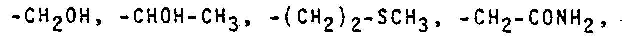 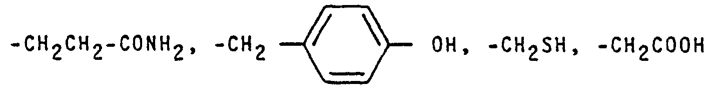 ou 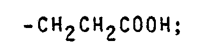 les groupes alkyléniques peuvent être rectilignes ou ramifiés et contiennent 1 à 3 atomes de carbone; et R" est H, -CONH₂ ou -COO(alkyle en Cₗ-C₇).

2. Multiplet selon la revendication 1, dans lequel le polyol est un monosaccharide ou un oligosaccharide.

3. Multiplet selon la revendication 2, dans lequel le monosaccharide est un pentose, hexose ou heptose, ou dans lequel l'oligosaccharide est un disaccharide, un trisaccharide ou une cyclodextrine.

4. Multiplet selon la revendication 3, dans lequel le polyol est le ribose, l'arabinose, le xylose, le lyxose, le ribulose, le xylulose, le glucose, le galactose, le mannose, le fructose, le sorbose, le tagatose, le mannoheptulose, le sédoheptulose, le saccharose, le lactose, le maltose, le raffinose, l'a-cyclodextrine, la β-cyclodextrine ou la y-cyclodextrine.

5. Multiplet selon la revendication 1, dans lequel le polyol est un composé polyhydroxy en C₃―C₁₅ ou un composé polyhydroxy alicyclique en C₅―C₁₈.

6. Multiplet selon la revendication 5, dans lequel le composé polyhydroxy aliphatique est un alkylpolyol en C₃―C₈, ou dans lequel le composé polyhydroxy alicyclique est un polyol aromatique complètement hydrogéné condensé.

7. Multiplet selon la revendication 6, dans lequel l'alkylpolyol en C₃―C₈ est le glycérol, l'érythritol ou le 1,2,6-trihydroxyhexane, ou dans lequel le polyol aromatique complètement hydrogéné condensé est un décahydronaphtalène polyol.

8. Multiplet selon la revendication 5, dans lequel le composé polyhydroxy alicyclique est un phénol cycloalkylique en C₅―C₁₀.

9. Multiplet selon la revendication 8, dans lequel le polyol cycloalkylique en C₅―C₁₀ est un polyol cyclohexanique.

10. Multiplet selon la revendication 9, dans lequel le cyclohexane polyol est l'inositol.

11. Multiplet selon la revendication 1, dans lequel R' et R" sont combinés avec l'atome d'azote voisin de sorte que 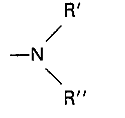 représente le résidu d'une amine secondaire monocyclique saturée comptant 5 à 7 atomes de noyau, contenant facultativement un autre atome hétérocyclique -0-, -S-, ou -N- en plus de l'atome d'azote indiqué, et portant facultativement 1 à 3 substituants méthyle.

12. Multiplet selon la revendication 11, dans lequel 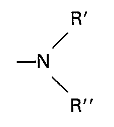 est morpholino, 1-pyrrolidinyl, perhydro-1,2,4-oxathiazine-4-yl, 1- ou 4-pipérazinyl, 4-méthyl-1-pipérazinyl, pipéridino, hexaméthylèneimino, 2-méthyl-1-pyrazolidinyl, 1- ou 2-pyrazolidinyl, 3-méthyl-1-imidazolidinyl ou 1- ou 3-imidazolidinyl.

13. Multiplet selon la revendication 1, dans lequel:
(i) R⁺ est de structure (a), (b) ou (c); R^{iv} est méthyle; et le groupe -COO-, -CH₂COO- ou -CH₂0COO- est situé en 3; ou
(ii) R⁺ est de structure (d); R' est méthyle ou éthyle et R" est identique à R', ou R'R"N- représente morpholino, pipéridino, 1-pyrrolidinyl ou 1-pipérazinyl; R^{iv} est méthyle; et l'alkylène est 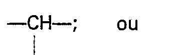
(iii) R⁺ est de structure (e), l'alkylène est ―CH₂CH₂― et R^{v} est H ou -CONH₂.

14. Multiplet selon la revendication 13(ii), dans lequel R'" est 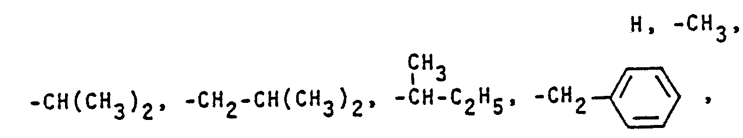 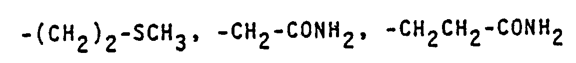 ou

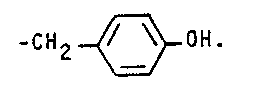

15. Multiplet selon la revendication 1, dans lequel p est égal à n.

16. Multiplet selon la revendication 15, dans lequel p est d'au moins 4.

17. Multiplet selon la revendication 1, dans lequel r est de 5, 6 ou 7.

18. Multiplet selon la revendication 1, dans lequel 0 est le squelette de l'inositol, n est de 6 et p est de 6.

19. Multiplet selon la revendication 18, dans lequel v est de 5, r est de 5 et s est de 6.

20. Multiplet selon la revendication 1, ledit multiplet ayant la formule de structure

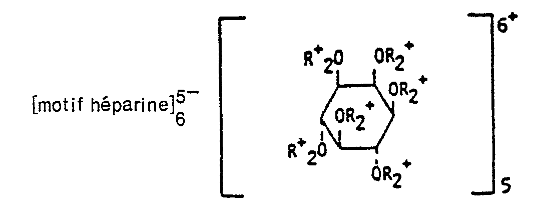

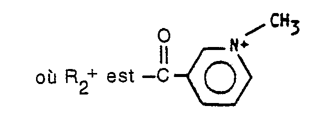

21. Composition pharmaceutique de matière comprenant un multiplet de formule (I) tel que défini par la revendication 1 et un support associé non toxique acceptable en pharmaceutique.

22. Composition pharmaceutique de matière, sous forme de doses unitaires, devant servir à tirer au clair une réponse à un anticoagulant chez l'animal à sang chaud, ladite composition comprenant, par dose unitaire, une quantité d'anticoagulant unitaire efficace d'un multiplet de formule (I) tel que défini par la revendication 1 et un support associé non toxique acceptable en pharmaceutique, ladite composition convenant pour administration par voie nasale, pour thérapie par inhalation par voie orale ou pour autre administration par voie orale.

23. Sel à formule de structure 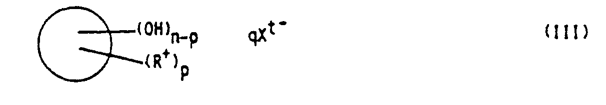 où O est le squelette d'un polyol, ledit squelette étant la partie dudit polyol demeurant après qu'on ait éliminé de celui-ci tous les substituants hydroxy; n est un nombre de 3 à 24 qui représente le nombre total de groupes hydroxy présents dans ledit polyol; p est un nombre ≥3 et ≤n; R⁺ est 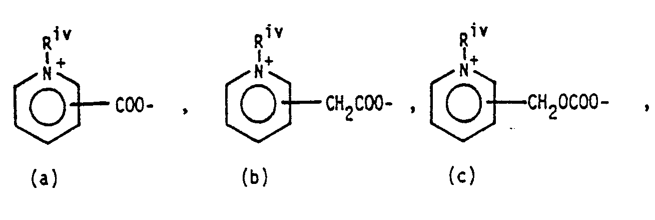 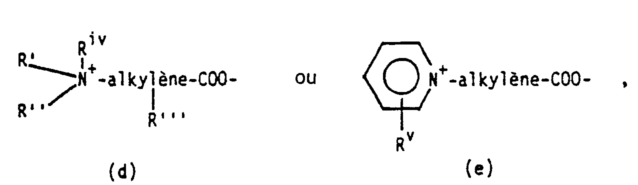 où les substituants ―COO―, ―CH₂COO―,―CH₂OCOO― et R^{v} du noyau peuvent se trouver placés chacun en 2-, 3-, ou 4- du noyau pyridinium; R'" est alkyle en C₁―C₃; R' et R", qui peuvent être identiques ou différents, sont chacun alkyle en C₁―C₇, ou R' et R" sont combinés avec l'atome d'azote voisin de sorte que 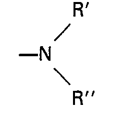 représente le résidu d'une amine secondaire monocyclique saturée; R"' est 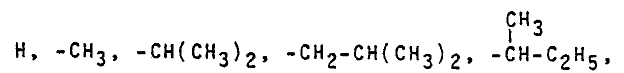 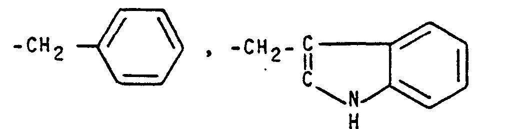 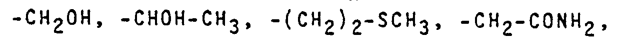 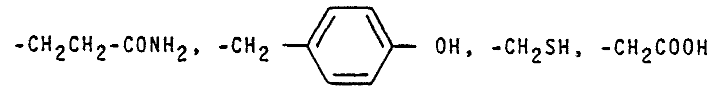 ou 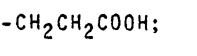 les groupes alkyléniques peuvent être rectilignes ou ramifiés et contiennent 1 à 3 atomes de carbone; R^{v} est H, ―CONH₂ ou ―COO(en C₁―C₅); x est l'anion d'un acide organique ou inorganique acceptable en pharmaceutique; t est la valence de l'anion acide; et q est le nombre qui, multiplié par t, est égal à p.

24. Sel selon la revendication 23, dans lequel le polyol est un monosaccharide ou un oligosaccharide.

25. Sel selon la revendication 24, dans lequel le monosaccharide est un pentose, hexose ou heptose, ou dans lequel l'oligosaccharide est un disaccharide, un trisaccharide ou une cyclodextrine.

26. Sel selon la revendication 23, dans lequel le polyol est un composé polyhydroxy aliphatique en C₃―C₁₅ ou un composé polyhydroxy alicyclique en C₅―C₁₈.

27. Sel selon la revendication 26, dans lequel le composé polyhydroxy aliphatique est un polyol alkylique en C₃-Cₐ, ou dans lequel le composé polyhydroxy alicyclique est un polyol cycloalkylique en C₅―C₁₀ ou un polyol aromatique complètement hydrogéné condensé.

28. Sel selon la revendication 27, dans lequel le polyol cycloalkylique en C₅―C₁₀ est un polyol cyclohexanique.

29. Sel selon la revendication 28, dans lequel le polyol cyclohexanique est l'inositol.

30. Sel selon la revendication 23, dans lequel R' et R" sont combinés avec l'atome d'azote voisin de sorte que 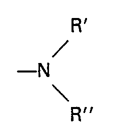 représente le résidu d'une amine secondaire monocyclique saturée comptant 5 à 7 atomes du noyau, contenant facultativement un autre atome hétérocyclique ―O,― ―S― ou ―N― et plus de l'atome d'azote indiqué et portant facultativement 1 à 3 substituants méthyle.

31. Sel selon la revendication 23, dans lequel:
(i) R⁺ est de structure (a), (b) ou (c); R^{iv} est méthyle; et le groupe -COO-, -CH₂COO- ou -CH₂0COO- est situé en 3; ou
(ii) R⁺ est de structure (d); R' est méthyle ou éthyle et R" est identique à R', ou R'R"N- représente morpholino, pipéridino, 1-pyrrolidinyl ou 1-pipérazinyl; R^{iv} est méthyle; et l'alkylène est 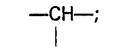 ou
(iii) R⁺ est de structure (e), l'alkylène est ―CH₂CH₂― et R^{v} est H ou ―CONH₂.

32. Sel selon la revendication 23, dans lequel p est égal à n, ou dans lequel t est de 1, ou dans lequel X-est I⁻, Br ou CI-.

33. Sel selon la revendication 23, dans lequel O est le squelette de l'inositol, n est 6 et p est 6.

34. Sel selon la revendication 33, ayant la formule de structure 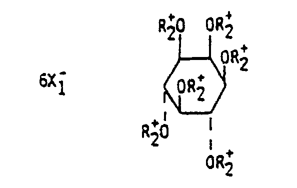 où X⁻₁ est I⁻, Br⁻ ou CI- et R₂⁺ est

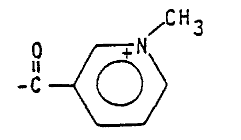

## Revendications (Revendications pour l'(les) Etat(s) contractant(s) suivant(s) : AT)

1. Procédé pour la préparation d'un multiplet à formule de structure: 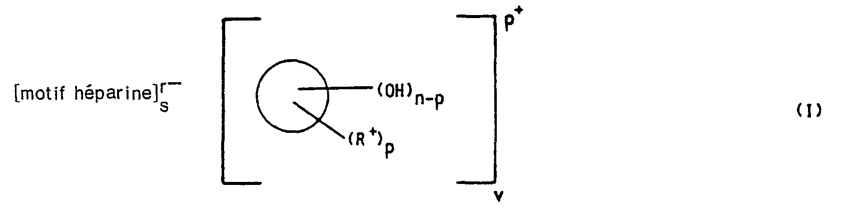 où O est le squelette d'un polyol, ledit squelette étant la partie dudit polyol demeurant après qu'on ait éliminé de celui-ci tous les substituants hydroxy; n est un nombre de 3 à 24 qui représente le nombre total de groupes hydroxy présents dans ledit polyol; p est un nombre ≥3 et ≤n; r est la valence disponible du motif héparine et est ≥3 et ≤7; s est le nombre qui multiplié par r est égal à pv; v est le nombre qui multiplié par p est égal à rs; R⁺ est 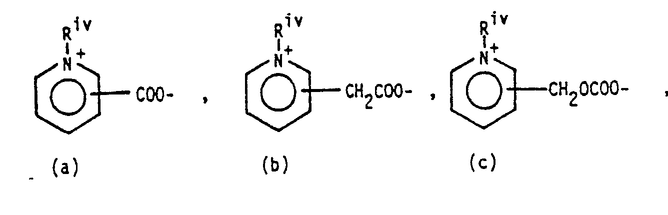 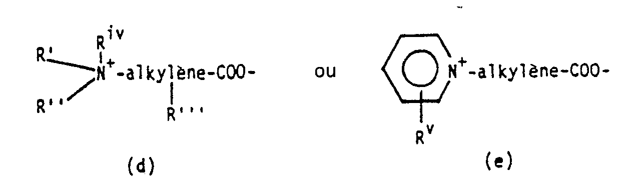 où les substituants―COO―, ―CH₂COO―, ―CH₂OCOO― et R^{v} du noyau peuvent se trouver chacun en 2, en 3 ou en 4 sur le noyau pyridinium; R^{iv} est alkyle en C₁―C₃; R' et R", qui peuvent être semblables ou différents, sont chacun alkyle en C₁―C₇, ou R' et R" sont combinés avec l'atome d'azote voisin de sorte que 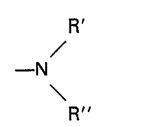 représente le résidu d'une amine secondaire monocyclique saturée; R"' est 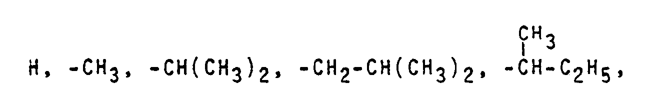 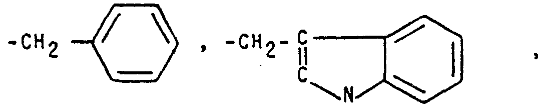 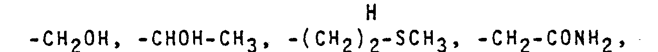 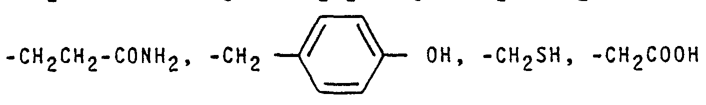 ou 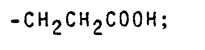 les groupes alkyléniques peuvent être rectilignes ou ramifiés et contiennent 1 à 3 atomes de carbone; et R est H, -CONH₂ ou -COO(alkyle en C₁―C₇); procédé selon lequel:
(a) on fait réagir le sel correspondant à formule de structure 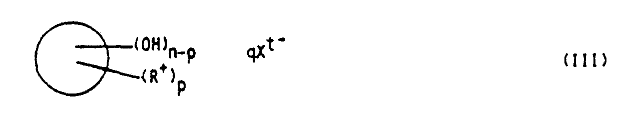 où O, n, p et R⁺ sont tels que définis par la formule (I) ci-dessus, X- est l'anion d'un acide organique ou inorganique acceptable en pharmaceutique, t est la valence de l'anion acide et q est le nombre qui, multiplié par t, est égal à p, avec de l'héparine sodique dans un milieu aqueux; ou
(b) on soumet le sel correspondant de formule (III) ci-dessus à un échange d'anions pour remplacer les anions X- du sel par des anions OH-, puis on fait réagir le produit intermédiaire résultant avec de l'acide héparinique.

2. Procédé selon la revendication 1, dans lequel le squelette de polyol est celui d'un monosaccharide ou d'un oligosaccharide.

3. Procédé selon la revendication 2, dans lequel le monosaccharide est un pentose, hexose ou heptose, ou dans lequel l'oligosaccharide est un disaccharide, un trisaccharide ou une cyclodextrine.

4. Procédé selon la revendication 1, dans lequel le squelette de polyol est celui de ribose, d'arabinose, de xylose, de lyxose, de ribulose, de xylulose, de glucose, de galactose, de mannose, de fructose, de sorbose, de tagatose, de mannoheptulose, de sédoheptulose, de saccharose, de lactose, de maltose, de raffinose, d'a-cyclodextrine, de β-cyclodextrine ou de y-cyclodextrine.

5. Procédé selon la revendication 1, dans lequel le squelette de polyol est celui d'un composé polyhydroxy aliphatique en C₃―C₁₅ ou d'un composé polyhydroxy alicyclique en C₅―C₁₈.

6. Procédé selon la revendication 5, dans lequel le composé polyhydroxy aliphatique est un polyol alkylique en C₃―C₈, ou dans lequel le composé polyhydroxy alicyclique est un polyol aromatique complètement hydrogéné condensé.

7. Procédé selon la revendication 6, dans lequel le polyol alkylique en C₃-Ce est le glycérol, l'érythritol ou le 1,2,6-trihydroxyhexane, ou dans lequel le polyol aromatique complètement hydrogéné condensé est un polyol décahydronaphtalénique.

8. Procédé selon la revendication 5, dans lequel le composé polyhydroxy alicyclique est un polyol cycloalkylique en C₅―C₁₀.

9. Procédé selon la revendication 8, dans lequel le polyol cycloalkylique en C₅―C₁₀ est un polyol cyclohexanique.

10. Procédé selon la revendication 9, dans lequel le polyol cyclohexanique est l'inositol.

11. Procédé selon la revendication 1, dans lequel R' et R" sont combinés avec l'atome d'azote voisin de sorte que 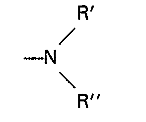 représente le résidu d'une amine secondaire monocyclique saturée comptant 5 à 7 atomes du noyau, contenant facultativement un autre atome hétérocyclique -0-, -S-, ou -N- en plus de l'atome d'azote indiqué, et portant facultativement 1 à 3 substituants méthyle.

12. Procédé selon la revendication 11 dans lequel 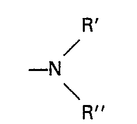 est morpholino, 1-pyrrolidinyl, perhydro-1,2,4-oxathiazine-4-yl, 1- ou 4-pipérazinyl, 4-méthyl-1-pipérazinyl, pipéridino, hexaméthylèneimino, 2-méthyl-1-pyrazolidinyl, 1- ou 2-pyrazolidinyl, 3-méthyl-1-imidazolidinyl ou 1- ou 3-imidazolidinyl.

13. Procédé selon la revendication 1, dans lequel:
(i) R⁺ est de structure (a), (b) ou (c); R^{iv} est méthyle; et le groupe -COO-, -CH₂COO- ou -CH₂0COO- est placé en 3; ou
(ii) R⁺ est de structure (d); R' est méthyle ou éthyle et R" est identique à R', ou R'R"N- représente morpholino, pipéridino, 1-pyrrolidinyl ou 1-pipérazinyl; R^{iv} est méthyle; et l'alkylène est 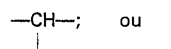
(iii) R⁺ est de structure (e), l'alkylène est ―CH₂CH₂― et R" est H ou -CONH₂.

14. Procédé selon la revendication 13(ii), dans lequel R'" est 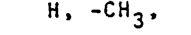 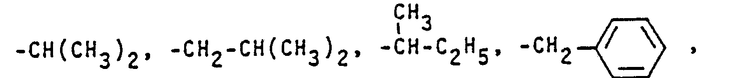 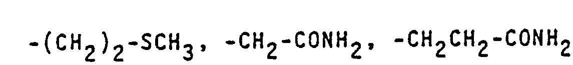 ou

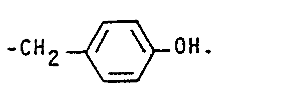

15. Procédé selon la revendication 1, dans lequel p est égal à n.

16. Procédé selon la revendication 15, dans lequel p est d'au moins 4.

17. Procédé selon la revendication 1, dans lequel r est de 5, 6 ou 7.

18. Procédé selon la revendication 1, dans lequel O est le squelette de l'inositol, n est 6 et p est 6.

19. Procédé selon la revendication 18, dans lequel v est de 5, r est de 5 et s est de 6.

20. Procédé selon la revendication 1, dans lequel le multiplet de formule (I) a la formule de structure:

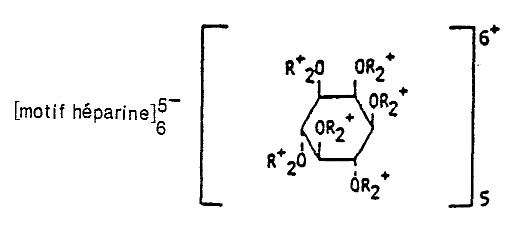

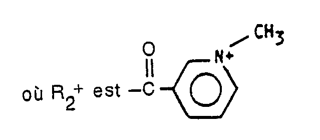

21. Procédé pour la préparation d'un sel à formule de structure 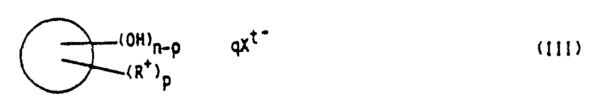 où O est le squelette d'un polyol, ledit squelette étant la partie dudit polyol demeurant après qu'on ait éliminé de celui-ci tous les substituants hydroxy; n est un nombre de 3 à 24 qui représente le nombre total de groupes hydroxy présents dans ledit polyol; p est un nombre ≥3 et ≤n; R⁺ est 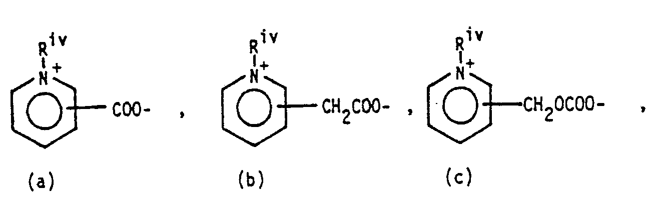 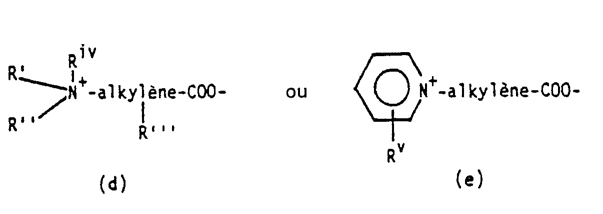 dans lequel les substituants -COO-, -CH₂COO-, -CH₂0COO- et R" du noyau peuvent se trouver chacun en 2-, 3-, ou 4- du noyau pyridinium; R'^{v} est alkyle en C₁―C₃; R' et R", qui peuvent être identiques ou différents, sont chacun alkyle en C₁―C₇, ou R' et R" sont combinés avec l'atome d'azote voisin de sorte 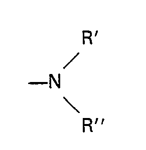 représente le résidu d'une amine secondaire monocyclique saturée; R"' est 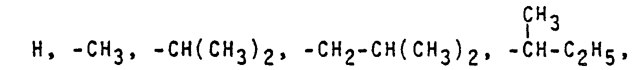 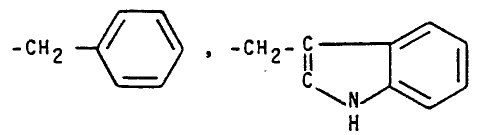 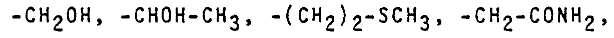 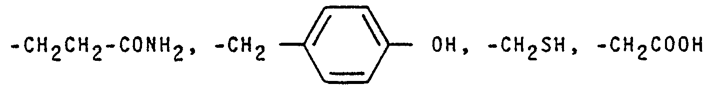 ou 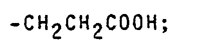 les groupes alkyléniques peuvent être rectilignes ou ramifiés et contiennent 1 à 3 atomes de carbone; R^{v} est H, ―CONH₂ ou ―COO(alkyle en C₁―C₇); X- est l'anion d'un acide organique ou inorganique acceptable en pharmaceutique; t est la valence de l'anion acide; et q est le nombre qui, multiplié par t, est égal à p; procédé selon lequel:
(a) on estérifie un polyol de formule 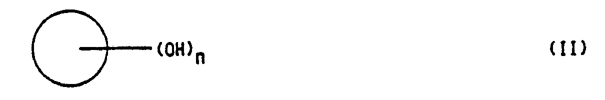 où O et n sont tels que définis ci-dessus, avec un sel d'acide quaternaire de formule 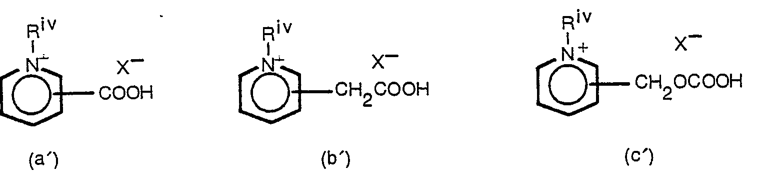 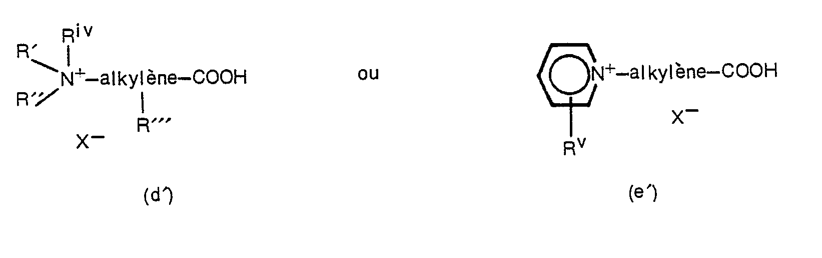 où les variables de structure sont telles que définies à propos des structures (a), (b), (c), (d) et (e), respectivement, ci-dessus et X- est tel que défini ci-dessus, ou avec l'anhydride mixte correspondant pour obtenir le sel correspondant de formule (III);
(b) on estérifie un polyol de formule (II) ci-dessus avec un acide tertiaire de formule 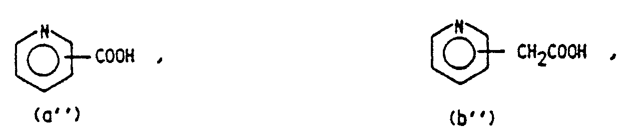 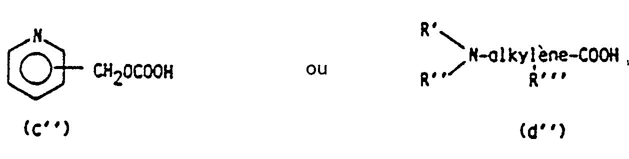 ou avec l'halogénure ou l'anhydride d'acide correspondant, où les variables de structure sont telles que définies à propos des structures (a), (b), (c) et (d), respectivement, ci-dessus, pour obtenir le composé correspondant de formule 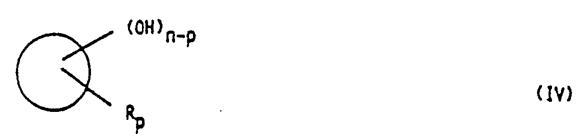 où O, n et p sont tels que définis ci-dessus et R est 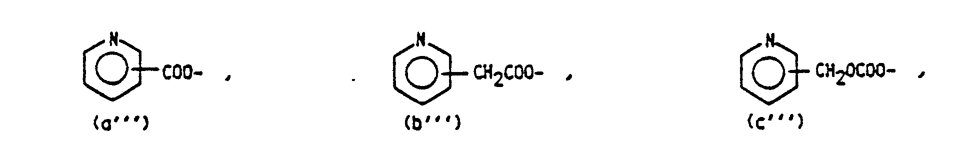 ou 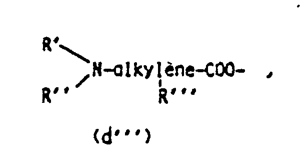 respectivement, où les variables de structure sont telles que définies à propos des structures (a), (b), (c) et (d) ci-dessus; après quoi on quaternise le composé résultant de formule (IV) avec un halogénure d'alkyle, R^{iv}-Hal, où R^{iv} est tel que défini ci-dessus, pour obtenir le sel correspondant de formule (III) où X- est un anion halogénure et R⁺ est de structure (a), (b), (c) ou (d), respectivement;
(c) on fait réagir un polyester de formule 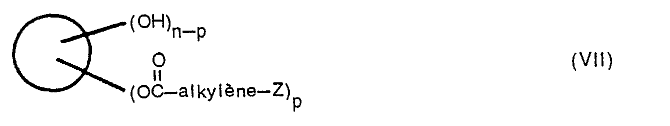 où O, n et p sont tels que définis ci-dessus, le groupe alkylénique peut être rectiligne ou ramifié et contient 1 à 3 atomes de carbone et Z est CI, Br ou I, avec l'excès de réactant de formule 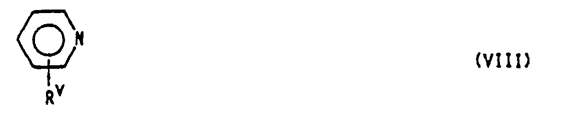 où R" est placé en 2-, 3- ou 4- sur le noyau pyridine et peut être H, -CONH₂ ou -COO(alkyle en C₁―C₇), pour obtenir le sel correspondant de formule (III) dans lequel X- est CI-, Br⁻ ou I⁻ et R⁺ a la structure (e);
(d) on fait réagir un ester activé de formule 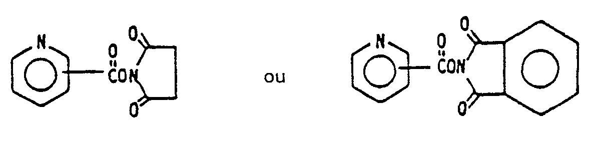 avec un polyol de formule (II) ci-dessus, après quoi on quaternise le composé résultant de formule (IV) ci-dessus où R est de structure (a"') avec un halogénure d'alkyle, R^{iv}-Hal, où R^{iv} est tel que défini ci-dessus, pour obtenir le sel correspondant de formule (III) où X- est un ion halogénure et R⁺ est de structure (a);
(e) on fait réagir un ester activé quaternisé de formule 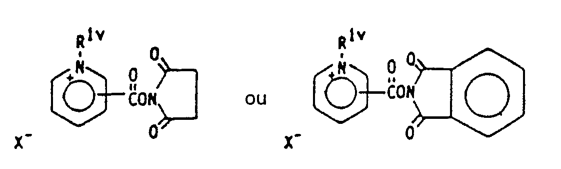 où R^{iv} et X- sont tels que définis ci-dessus, avec un polyol de formule (II) ci-dessus, pour obtenir le sel correspondant de formule (III) où R⁺ est de structure (a);
(f) on fait réagir un anhydride quaternisé de formule 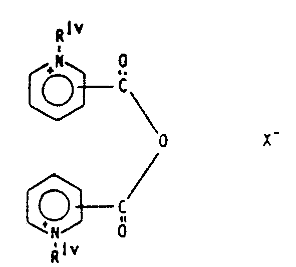 où X- et R^{iv} sont tels que définis ci-dessus, avec un polyol de formule (II) ci-dessus, pour obtenir le sel correspondant de formule (III) dans lequel R⁺ est de structure (a); ou
(g) on soumet un sel de formule (III) à un échange d'ions pour obtenir un sel de formule (III) contenant un anion X- différent.

22. Procédé selon la revendication 21, dans lequel le squelette de polyol est celui d'un monosaccharide ou d'un oligosaccharide.

23. Procédé selon la revendication 22, dans lequel le monosaccharide est un pentose, hexose ou heptose, ou dans lequel l'oligosaccharide est un disaccharide, un trisaccharide ou une cyclodextrine.

24. Procédé selon la revendication 21, dans lequel le squelette de polyol est celui d'un composé polyhydroxy aliphatique en C₃―C₁₅ ou d'un composé polyhydroxy alicyclique en C₅―C₁₈.

25. Procédé selon la revendication 24, dans lequel le composé polyhydroxy aliphatique est un polyol alkylique en C₃―C₈, ou dans lequel le composé polyhydroxy alicyclique est un polyol cycloalkylique en C₅―C₁₀ ou un polyol aromatique complètement hydrogéné condensé.

26. Procédé selon la revendication 25, dans lequel le polyol cycloalkylique en C₅―C₁₀ est le polyol cyclohexanique.

27. Procédé selon la revendication 26, dans lequel le polyol cyclohexanique est l'inositol.

28. Procédé selon la revendication 21, dans lequel R' et R" sont combinés avec l'atome d'azote voisin en sorte que 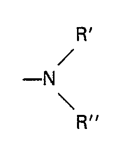 représente le résidu d'une amine secondaire monocyclique saturée comptant 5 à 7 atomes du noyau, contenant facultativement un autre atome hétérocyclique ―O―, ―S― ou ―N― en plus de l'atome d'azote indiqué, et portant facultativement 1 à 3 substituants méthyle.

29. Procédé selon la revendication 21, dans lequel:
(i) R⁺ est de structure (a), (b) ou (c); R^{iv} est méthyle; et le groupe -COO-, -CH₂COO- ou -CH₂0COO- est situé en position 3; ou
(ii) R⁺ est de structure (d); R' est méthyle ou éthyle et R" est identique à R', ou R'R"N- représente morpholino, pipéridino, 1-pyrrolidinyl ou 1-pipérazinyl; R^{iv} est méthyle; et l'alkylène est 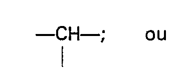
(iii) R⁺ est de structure (e), l'alkylène est ―CH₂CH₂― et R^{v} est H ou -CONH₂.

30. Procédé selon la revendication 21, dans lequel p est égal à n, ou dans lequel t est de 1 ou dans lequel X- est I⁻, Br⁻ ou Cl⁻.

31. Procédé selon la revendication 21, dans lequel O est le squelette de l'inositol, n est 6 et p est 6.

32. Procédé selon la revendication 21, dans lequel le sel de formule (III) a la forme de structure 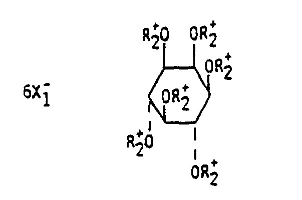 où X⁻₁ est I⁻, Br⁻ ou Cl⁻ est R₂⁺ est
